# EUROPÄISCHE PATENTSCHRIFT

(11) **EP 1 913 039 B1**
(45) Veröffentlichungstag und Bekanntmachung des Hinweises auf die Patenterteilung: **08.05.2013**
(21) Anmeldenummer: 06764244.7
(22) Anmeldetag: 21.07.2006
(51) Int. Cl.: C08F 220/04, C08F 220/34, C08F 226/00

(54) **SILICONGRUPPEN-HALTIGES COPOLYMER, DESSEN HERSTELLUNG UND VERWENDUNG**
COPOLYMER CONTAINING SILICONE GROUPS, ITS PREPARATION AND USE
COPOLYMERE CONTENANT DES GROUPES DE SILICONE, SA PRODUCTION ET SON UTILISATION

(30) Priorität: 26.07.2005 DE 102005034906
(43) Veröffentlichungstag der Anmeldung: 23.04.2008
(73) Patentinhaber: BASF SE, 67056 Ludwigshafen (DE)
(72) Erfinder: NGUYEN-KIM, Son, 69502 Hemsbach (DE); GARCIA CASTRO, Ivette, 67067 Ludwigshafen (DE); MATHAUER, Klemens, 69115 Heidelberg (DE); WENDEL, Volker, 64342 Seeheim-Jugenheim (DE); VÖLLMAR, Helmuth, 67061 Ludwigshafen (DE); KAISER, Thomas, 67454 Hassloch (DE)
(86) Internationale Anmeldenummer: PCT/EP2006/064507
(87) Internationale Veröffentlichungsnummer: WO 2007/012610

(56) Entgegenhaltungen:
- EP-A2- 0 524 557
- WO-A-00/39176
- WO-A-01/62809
- WO-A2-2004/058837
- US-A- 3 966 687

## Beschreibung

Die folgende Erfindung betrifft Silicongruppen-haltige Copolymere, die wenigstens ein Monomer mit einer ionogenen und/oder ionischen Gruppe und wenigstens ein vernetzendes Monomer einpolymerisiert enthalten, ein Verfahren zur Herstellung solcher Silicongruppen-haltiger Copolymere durch Fällungspolymerisation sowie die Verwendung dieser Copolymere.

An kosmetische, pharmazeutische und technische Mittel werden häufig spezielle Anforderungen bezüglich ihrer rheologischen Eigenschaften gestellt. Häufig können sie nur mit Hilfe von Zusatzstoffen, so genannten "Verdickern", in die gewünschte Anwendungsform gebracht werden. Beispiele für übliche niedermolekulare Verdickungsmittel sind z. B. die Alkali- und Aluminiumsalze von Fettsäuren, Fettalkohole oder Wachse. Die Verwendung der bekannten Verdickungsmittel ist jedoch, je nach Einsatzgebiet der zu verdickenden Zubereitung, häufig mit Nachteilen verbunden. So kann entweder die Verdickungswirkung der Verdickungsmittel nicht zufrieden stellend, ihr Einsatz unerwünscht oder ihre Einarbeitung in die zu verdickende Zubereitung, beispielsweise wegen ihrer Unverträglichkeit mit der zu verdickenden Verbindung erschwert oder ganz unmöglich sein. Schwierigkeiten bereitet oft die Bereitstellung von Produkten mit einem komplexen Eigenschaftsprofil unter Einsatz eines möglichst geringen Anteils oder möglichst wenig verschiedener wirkaktiver Substanzen. So besteht ein Bedarf an Polymeren für kosmetische und andere Mittel, die über gute konditionierende Eigenschaften verfügen, d. h. die sensorischen Eigenschaften der damit modifizierten Mittel positiv beeinflussen, und gleichzeitig eine Einstellung der rheologischen Eigenschaften der Mittel ermöglichen. Zudem werden an kosmetische und pharmazeutische Produkte vom Verbraucher zunehmend ästhetische Anforderungen gestellt. So wird bei derartigen Produkten derzeit eine Bevorzugung von klaren, opaken Formulieren in Form von Gelen beobachtet. Es besteht daher Bedarf an kosmetisch und pharmazeutisch verträglichen Polymeren, die geeignet sind, ein bestimmtes Eigenschaftsprofil bezüglich der sensorischen Eigenschaften und der Rheologie bereit zu stellen. Diese sollen sich insbesondere in Pulver überführen lassen und dennoch befähigt sein, sich in kurzer Zeit in eine zu verdickende Zusammensetzung einarbeiten zu lassen und dabei zuverlässig die gewünschten Rheologieeigenschaften bereit zu stellen.

Es ist bekannt, zur Modifizierung der rheologischen Eigenschaften Polymere einzusetzen. Diese haben den Vorteil, dass sie im Allgemeinen eine Einstellung der Viskosität in Abhängigkeit von ihrem Molekulargewicht ermöglichen. Ein häufig auftretender Nachteil beim Einsatz von Polymeren als Verdickungsmittel zur Herstellung von höherviskosen bzw. gelartigen Zubereitungen ist, dass mit zunehmendem Molekulargewicht des Polymers dessen Einarbeitung im Allgemeinen schwerer wird, und dass schließlich oftmals nur eine Quellung des Polymers anstatt der angestrebten Lösung zu beobachten ist.

Die WO 01/85821 beschreibt Polyurethane und deren Verwendung zur Modifizierung rheologischer Eigenschaften.

Die US 3,915,921 beschreibt Copolymere, die eine olefinisch ungesättigte Carbonsäure, ein C₁₀-C₃₀-Alkyl(meth)acrylat und gegebenenfalls ein vernetzendes Monomer mit wenigstens zwei ethylenisch ungesättigten Doppelbindungen einpolymerisiert enthalten. In neutralisierter Form dienen sie als Verdicker für diverse Anwendungen.

Die WO 97/21744 beschreibt vernetzte anionische Copolymere und deren Verwendung als Verdickungs- und Dispergiermittel in wässrigen Systemen.

Die EP-A-0 982 021 beschreibt die Verwendung von (teil)neutralisierten Copolymerisaten aus
A) 50 bis 99 Gew.-% monoethylenisch ungesättigten Carbonsäuren und
B) 1 bis 50 Gew.-% mindestens eines Comonomers, ausgewählt unter
   a) monoethylenisch ungesättigten Carbonsäureestern mit gesättigten C₈-C₃₀-Alkoholen,
   b) N-C₈-C₁₈-Alkyl- und N,N-Di-C₈-C₁₈-alkylcarbonsäureamiden,
   c) Vinylestern aliphatischer C₈-C₃₀-Carbonsäuren,
   d) C₈-C₁₈-Alkylvinylethern,
   und Mischungen davon
als Verdicker zur Herstellung von Haarwaschmitteln.

In der US 4,395,524 und der US 4,432,881 werden als Verdicker wirksame Copolymere auf Basis von Amidgruppen-haltigen Monomeren beschrieben.

Die DE-A-42 13 971 beschreibt Copolymere, die wenigstens ein olefinisch ungesättigtes Säuregruppen-haltiges Monomer, wenigstens eine olefinisch ungesättigte quartäre Ammoniumverbindung, gegebenenfalls wenigstens ein Polyether(meth)acrylat und gegebenenfalls wenigstens einen Vernetzer einpolymerisiert enthalten und deren Verwendung als Verdickungsmittel zur Verdickung wässriger Systeme, wobei es sich um kosmetische Zubereitungen handeln kann.

Die EP-A-893 117 und die EP-A-913 143 beschreiben vernetzte kationische Copolymere und deren Verwendung u. a. als haarfestigende Gelbildner in kosmetischen Zusammensetzungen.

Die EP-A-1 064 924 beschreibt die Verwendung von vernetzten kationischen Polymeren in hautkosmetischen und dermatologischen Zubereitungen, u. a. als Verdicker.

Die US 5,015,708 beschreibt ein Verfahren zur Herstellung eines Terpolymers aus (i) einem Vinyllactam, (ii) einem Säuregruppen-haltigen Monomer und (iii) einem hydrophoben Monomer, wobei es sich u. a. um eine ethylenisch ungesättigte Siliconverbindung handeln kann, durch Fällungspolymerisation sowie die Herstellung von Pulvern aus diesen Polymeren.

Die WO 00/39176 beschreibt ein hydrophiles, kationisches, ampholytisches Copolymer, das 0,05 bis 20 Mol-% eines anionischen Monomers mit wenigstens einer Carboxygruppe, 0 bis 45 Mol-% eines kationischen Monomers mit wenigstens einer Aminogruppe sowie gegebenenfalls ein hydrophobes Monomer und/oder einen Vernetzer einpolymerisiert enthält, wobei das Molverhältnis von kationischem zu anionischem Monomer etwa 2 : 1 bis 16 : 1 beträgt. Diese Silicongruppen-haltigen Copolymere können u. a. zur Modifizierung rheologischer Eigenschaften von Körperpflegemitteln eingesetzt werden.

Die WO 04/058837 beschreibt ein ampholytisches Copolymer, das durch radikalische Copolymerisation von
a) wenigstens einer ethylenisch ungesättigten Verbindung mit mindestens einer anionogenen und/oder anionischen Gruppe,
b) wenigstens einer ethylenisch ungesättigten Verbindung mit mindestens einer kationogenen und/oder kationischen Gruppe,
c) wenigstens einer ungesättigten amidgruppenhaltigen Verbindung
sowie gegebenenfalls weiterer Comonomere erhältlich sind. Die Polymerisation kann in Gegenwart einer Pfropfgrundlage erfolgen, wobei es sich u. a. um ein Polyalkylenoxidhaltiges Siliconderivat handeln kann. Beschrieben sind weiterhin Polyelektrolyt-Komplexe, die ein solches ampholytisches Copolymer enthalten sowie kosmetische oder pharmazeutische Mittel auf Basis dieser Silicongruppen-haltigen Copolymere und Polyelektrolyt-Komplexe.

Die US 2006/0084586 A1 beschreibt Rheologie-modifizierende Haarfestigerharze, die ein vernetztes Copolymer auf Basis von Vinylamid- und Carbonsäuremonomeren enthalten. Polymere, die durch radikalische Polymerisation in Gegenwart wenigstens einer eine Polyethergruppe und/oder eine radikalisch polymerisierbare Doppelbindung aufweisenden Siliconverbindung erhältlich sind, sind nicht beschrieben.

Der vorliegenden Erfindung liegt die Aufgabe zu Grunde, neue Polymere zur Verfügung zustellen, die sich zur Modifizierung der rheologischen Eigenschaften von kosmetischen, pharmazeutischen und weiteren Zusammensetzungen eignen. Insbesondere sollen sich diese Polymere in eine feste Form, vorzugsweise ein Pulver, überführen lassen, das sich leicht in die zu verdickenden Formulierungen einarbeiten lässt. Des Weiteren sollen die bereitgestellten Polymere weitere anwendungstechnische Eigenschaften der mit ihnen modifizierten Zusammensetzungen, insbesondere deren sensorische Eigenschaften, verbessern.

Überraschenderweise wurde nun gefunden, dass diese Aufgabe durch ein Copolymer gelöst wird, das wenigstens ein Monomer mit mindestens einer ionogenen und/oder ionischen Gruppe, wenigstens einen Vernetzer und zusätzlich wenigstens eine Siliconverbindung einpolymerisiert enthält.

Gegenstand der Erfindung ist daher ein Silicongruppen-haltiges Copolymer A) erhältlich durch radikalische Copolymerisation von
a) wenigstens einer Verbindung mit einer radikalisch polymerisierbaren, α,β-ethylenisch ungesättigten Doppelbindung und mindestens einer ionogenen und/oder ionischen Gruppe pro Molekül, wobei die Monomere a) ausschließlich ausgewählt sind unter Verbindungen a1) mit anionogenen und/oder anionischen Gruppen oder wobei die Komponente a) wenigstens eine Verbindung mit mindestens einer anionogenen und/oder anionischen Gruppe a1) und wenigstens eine Verbindung mit mindestens einer kationogenen und/oder kationischen Gruppe a2) umfasst und wobei das Copolymer A) einen molaren Überschuss an anionogenen/anionischen Gruppen gegenüber kationogenen/kationischen Gruppen von größer als 1 : 1 aufweist,
b) wenigstens einer radikalisch polymerisierbaren vernetzenden Verbindung, die wenigstens zwei α,β-ethylenisch ungesättigte Doppelbindungen pro Molekül enthält,

in Gegenwart wenigstens einer eine radikalisch polymerisierbare olefinisch ungesättigte Doppelbindung enthaltenden Siliconverbindung c), wobei es sich bei der wenigstens eine radikalisch polymerisierbare Doppelbindung aufweisenden Siliconverbindung um ein radikalisch polymerisierbares Siloxangruppen-haltiges Urethan(meth)acrylat handelt.

Die Herstellung der erfindungsgemäßen Silicongruppen-haltigen Copolymere A) kann nach herkömmlichen Polymerisationsverfahren, z. B. durch Lösungs- oder Substanzpolymerisation, erfolgen. Copolymere mit besonders vorteilhaften Eigenschaften, d. h. mit im Allgemeinen höheren Molekulargewichten und einer besseren Befähigung zur Bindung pulverförmiger Formulierungen, als sie nach herkömmlichen Polymerisationsverfahren erhalten werden, werden durch die Herstellung nach der Methode der Fällungspolymerisation erhalten. Eine bevorzugte Ausführungsform der Erfindung sind daher Silicongruppen-haltige Copolymere A), die durch radikalische Copolymerisation nach der Methode der Fällungspolymerisation in wenigstens einem organischen Lösungsmittel erhältlich sind. In einer speziellen Ausführung werden zur Herstellung der erfindungsgemäßen Copolymere wenigstens zwei Radikalinitiatoren eingesetzt, deren Zerfallstemperaturen und/oder deren Halbwertszeiten bei einer bestimmten Polymerisationstemperatur voneinander verschieden sind. Dabei können Copolymere mit besonders geringen Restmonomergehalten erzielt werden. Dies ist insbesondere der Fall, wenn der bei höherer Temperatur zerfallende Initiator vor Abschluss, vorzugsweise vor Beginn der Fällung des Polymers, zugegeben wird.

Bei der Fällungspolymerisation sind die eingesetzten Monomere im Reaktionsmedium (Monomer, Lösungsmittel) löslich, das entsprechende Polymer aber nicht. Das entstehende Polymer wird unter den gewählten Polymerisationsbedingungen unlöslich und fällt aus dem Reaktionsgemisch aus. Dabei lassen sich ampholytische Copolymere A) mit höheren Molekulargewichten erhalten als nach anderen Polymerisationsverfahren, z. B. durch Lösungspolymerisation, die sich besonders vorteilhaft als Rheologiemodifizierer (speziell Verdicker) eignen.

Im Rahmen der vorliegenden Erfindung umfasst der Ausdruck Alkyl geradkettige und verzweigte Alkylgruppen. Geeignete kurzkettige Alkylgruppen sind z. B. geradkettige oder verzweigte C₁-C₇-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc.

Geeignete längerkettige C₈-C₃₀-Alkyl- bzw. C₈-C₃₀-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z. B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en), Arachinyl(en), Behenyl(en), Lignocerinyl(en), Melissinyl(en), etc.

Cycloalkyl steht vorzugsweise für C₅-C₈-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Die erfindungsgemäßen Copolymere A) lassen sich unter Normalbedingungen (20 °C) vorteilhaft als Gele formulieren. "Gelförmige Konsistenz" zeigen Formulierungen, die eine höhere Viskosität als eine Flüssigkeit aufweisen und die selbsttragend sind, d. h. die eine ihnen verliehene Form ohne formstabilisierende Umhüllung behalten. Im Gegensatz zu festen Formulierungen lassen sich gelförmige Formulierungen jedoch leicht unter Anwendung von Scherkräften deformieren. Die Viskosität der gelförmigen Mittel liegt vorzugsweise in einem Bereich von größer als 600 bis etwa 60000 mPas, besonders bevorzugt von 6000 bis 30000 mPas. Vorzugsweise handelt es sich bei den Gelen um Haargele.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Unter wasserdispergierbaren Monomeren und Polymeren werden Monomere und Polymere verstanden, die unter Anwendung von Scherkräften, beispielsweise durch Rühren, in dispergierbare Partikel zerfallen. Hydrophile Monomere sind vorzugsweise wasserlöslich oder zumindest wasserdispergierbar. Die erfindungsgemäßen Copolymere A) sind im Allgemeinen wasserlöslich.

Die erfindungsgemäßen Copolymere A) eignen sich besonders vorteilhaft zur Modifizierung der rheologischen Eigenschaften von Zusammensetzungen, die wenigstens eine bei 20 °C und 1013 mbar flüssige Verbindung enthalten. "Modifizierung rheologischer Eigenschaften" wird im Rahmen der vorliegenden Erfindung weit verstanden. So eignen sich die erfindungsgemäßen Copolymere A) im Allgemeinen zur Verdickung der Konsistenz von flüssigen Verbindungen in einem weiten Bereich. Je nach Grundkonsistenz der flüssigen Verbindung(en) können in Abhängigkeit von der Einsatzmenge des Copolymers A) in der Regel Fließeigenschaften von dünnflüssig bis hin zu fest (nicht mehr fließend) erzielt werden. Unter "Modifizierung rheologischer Eigenschaften" wird daher u. a. die Erhöhung der Viskosität von Flüssigkeiten, die Verbesserung der Thixotropie-Eigenschaften von Gelen, die Verfestigung von Gelen und Wachsen etc. verstanden.

In einer speziellen Ausführungsform weisen die erfindungsgemäßen Silicongruppen-haltigen Copolymere A) sowohl anionogene und/oder anionische Gruppen als auch kationogene und/oder kationische Gruppen auf. Zur Herstellung solcher Silicongruppen-haltiger Copolymere A) können die entgegengesetzt geladenen/ladbaren Monomere a) gemeinsam, d. h. in Form eines Monomerenpaares ("Monomerensalzes") eingesetzt werden. In dieser Monomerzusammensetzung beträgt das molare Verhältnis von anionogenen und anionischen Gruppen zu kationogenen und kationischen Gruppen etwa 1 : 1 (d. h. einwertige Monomere werden im Wesentlichen äquimolar eingesetzt). Die Monomerenpaare können dabei vor ihrem Einsatz zur Polymerisation separat hergestellt werden. Bevorzugt ist jedoch die "in situ"-Herstellung der Monomerenpaare durch gemeinsamen Einsatz (z. B. gemeinsamen Zulauf) bei der Herstellung der Copolymerisate.

### Monomer a)

Die Komponente a) wird vorzugsweise in einer Menge von 1 bis 99 Gew.-%, besonders bevorzugt 5 bis 98 Gew.-%, insbesondere 10 bis 97 Gew.-%, bezogen auf das Gesamtgewicht der zur Polymerisation eingesetzten Verbindungen (d. h. Komponenten a), b), c) und, falls vorhanden, d) bis f)), eingesetzt.

Erfindungsgemäß enthalten die Copolymere A) als Komponente a) wenigstens eine Verbindung a1) mit mindestens einer anionogenen und/oder anionischen Gruppe pro Molekül einpolymerisiert. In einer speziellen Ausführung sind die Monomere a) ausschließlich ausgewählt unter Verbindungen a1) mit anionogenen und/oder anionischen Gruppen.

Vorzugsweise umfasst die Komponente a) a) wenigstens eine Verbindung a1), die ausgewählt ist unter monoethylenisch ungesättigten Carbonsäuren, Suffonsäuren, Phosphonsäuren und Mischungen davon.

Zu den Monomeren a1) zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren a1) zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Monomeren a1) zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Zu den Monomeren a1) zählen auch die Salze der zuvor genannten Säuren, insbesondere die Natrium-, Kalium- und Ammoniumsalze sowie die Salze mit Aminen. Die Monomere a1) können als solche oder als Mischungen untereinander eingesetzt werden. Die angegebenen Gewichtsanteile beziehen sich sämtlich auf die Säureform.

Vorzugsweise umfasst die Komponente a) wenigstens eine Verbindung a1), die ausgewählt ist unter Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure, 2-Acrylamido-2-methylpropansulfonsäure, Vinylphosphonsäure und Mischungen davon.

Besonders umfasst die Komponente a) wenigstens eine Verbindung a1), die ausgewählt ist unter Acrylsäure, Methacrylsäure und Mischungen davon.

In einer bevorzugten Ausführungsform enthalten die Copolymere A) wenigstens eine Verbindung a2) mit mindestens einer kationogenen und/oder kationischen Gruppe pro Molekül einpolymerisiert.

Vorzugsweise umfasst die Komponente a2) wenigstens eine Verbindung die ausgewählt ist unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen und Mischungen davon.

In einer besonders bevorzugten Ausführungsform umfasst die Komponente a) a) als vinylsubstituierte heteroaromatische Verbindung a2) wenigstens eine N-Vinylimidazol-Verbindung. In einer speziellen Ausführungsform ist die Komponente a) ausgewählt unter N-Vinylimidazol-Verbindungen und Mischungen, die wenigstens eine N-Vinylimidazol-Verbindung enthalten.

Bevorzugt handelt es sich bei den kationogenen und/oder kationischen Gruppen der Komponente a2) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung oder durch Quaternisierung mit Säuren oder Alkylierungsmitteln erzeugen. Dazu zählen z. B. Carbonsäuren, wie Milchsäure, oder Mineralsäuren, wie Phosphorsäure, Schwefelsäure und Salzsäure, oder als Alkylierungsmittel C₁-C₄-Alkylhalogenide oder -sulfate, wie Ethylchlorid, Ethylbromid, Methylchlorid, Methylbromid, Dimethylsulfat und Diethylsulfat. Eine Protonierung oder Quaternisierung kann im Allgemeinen sowohl vor als auch nach der Polymerisation erfolgen.

Geeignete N-Vinylimidazol-Verbindungen sind Verbindungen der Formel 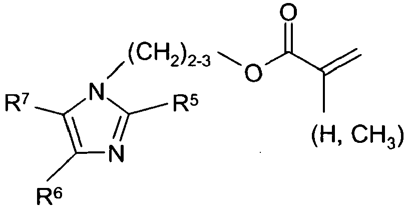 worin R⁵ bis R⁷ unabhängig voneinander für Wasserstoff, C₁-C₄-Alkyl oder Phenyl stehen. Bevorzugt stehen R⁵ bis R⁷ für Wasserstoff.

Des Weiteren bevorzugt enthält das Copolymer als Monomer a) wenigstens eine N-Vinylimidazol-Verbindung der allgemeinen Formel (II) 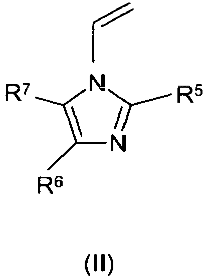 einpolymerisiert, worin R⁵ bis R⁷ unabhängig voneinander für Wasserstoff, C₁-C₄-Alkyl oder Phenyl stehen.

Beispiele für Verbindungen der allgemeinen Formel (II) sind folgender Tabelle 1 zu entnehmen:

**Tabelle 1**

| **R⁵** | **R⁶** | **R⁷** |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Bevorzugt als Monomer a2) ist 1-Vinylimidazol (N-Vinylimidazol) und sind Mischungen, die N-Vinylimidazol enthalten.

Geeignete Monomere a2) sind auch die durch Protonierung oder Quaternisierung der zuvor genannten N-Vinylimidazolverbindungen erhältlichen Verbindungen. Beispiele für solche geladenen Monomere a2) sind quatemisierte Vinylimidazole, insbesondere 3-Methyl-1-vinylimidazoliumchlorid und -methosulfat. Geeignete Säuren und Alkylierungsmittel sind im Folgenden aufgeführt.

Die Copolymere A) können an Stelle von oder zusätzlich zu den zuvor genannten N-Vinylimidazol-Verbindungen wenigstens ein anderes Monomer a2) mit mindestens einer kationogenen und/oder kationischen Gruppen einpolymerisiert enthalten. Vorzugsweise beträgt der Anteil an diesen Monomeren a2) 0 bis 50 Gew.-%, besonders bevorzugt 0 bis 30 Gew.-%, ganz besonders bevorzugt 0,1 bis 20 Gew.-%, bezogen auf das Gesamtgewicht der zur Polymerisation eingesetzten Verbindungen.

Wird wenigstens eine N-Vinylimidazol-Verbindung, speziell N-Vinylimidazol, als alleiniges Monomer a2) eingesetzt, so beträgt der Anteil vorzugsweise 3 bis 96 Gew.-%, bezogen auf das Gesamtgewicht der zur Polymerisation eingesetzten Verbindungen.

Geeignete Verbindungen a2) sind die Ester von α,β-ethylenisch ungesättigten Mono- und Dicarbonsäuren mit Aminoalkoholen. Bevorzugte Aminoalkohole sind C₂-C₁₂-Aminoalkohole, welche am Aminstickstoff C₁-C₈-mono- oder -dialkyliert sind. Als Säurekomponente dieser Ester eignen sich z. B. Acrylsäure, Metharylsäure, Fumarsäure, Maleinsäure, Itaconsäure, Crotonsäure, Maleinsäureanhydrid, Monobutylmaleat und Gemische davon. Bevorzugt werden als Säurekomponente Acrylsäure, Methacrylsäure und deren Gemische eingesetzt.

Bevorzugte Monomere a2) sind N-tert.-Butylaminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat. Besonders bevorzugt sind N-tert.-Butylaminoethyl(meth)acrylat und N,N-Dimethylaminoethyl(meth)acrylat.

Geeignete Monomere a2) sind weiterhin die Amide der zuvor genannten α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen.

Bevorzugt als Monomere a2) sind z. B. N-[tert.-Butylaminoethyl(meth)acrylamid, N-[2-dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylaminb)butyl]acrylamid, N-[4-(dimethylamino)butyl]methacrylamid, N-[2-(diethylamino)ethyl]acrylamid, N-[4-(dimethylamino)cyclohexyl]acrylamid und N-[4-(dimethylamino)cyclohexyl]methacrylamid. Besonders bevorzugt sind N-[3-(dimethylamino)propyl]acrylamid und N-[3-(dimethylamino)propyl]methacrylamid (DMAPMAM).

Eine spezielle Ausführungsform betrifft Copolymere A), die N-[3-(dimethylamino)propyl]acrylamid und N-[3-(dimethylamino)propyl]methacrylamid und keine Vinylimidazol-Verbindung enthalten. In einer ganz speziellen Ausführung besteht die Komponente a2) nur aus N-[3-(dimethylamino)propyl]acrylamid und/oder N-[3-(dimethylamino)propyl]methacrylamid. Dann beträgt der Anteil an N-[3-(dimethylamino)propyl]acrylamid und N-[3-(dimethylamino)propyl]methacrylamid (in Summe, soweit beide vorhanden) vorzugsweise 2 bis 95 Gew.-%, besonders bevorzugt 3 bis 60 Gew.-%, bezogen auf das Gesamtgewicht der zur Polymerisation eingesetzten Monomere.

Geeignete Monomere a2) sind weiterhin N,N-Diallylamine und N,N-Diallyl-N-alkylamine und deren Säureadditionssalze und Quaternisierungsprodukte. Alkyl steht dabei vorzugsweise für C₁-C₂₄-Alkyl. Bevorzugt sind N,N-Diallyl-N-methylamin und N,N-Diallyl-N,N-dimethylammonium-Verbindungen, wie z. B. die Chloride und Bromide. Besonders bevorzugt ist N,N-Diallyl-N-methylamin.

Geeignete Monomere a2) sind weiterhin von Vinylimidazolen verschiedene vinyl- und allylsubstituierte Stickstoffheterocyclen, wie 2- und 4-Vinylpyridin, 2- und 4-Allylpyridin, und die Salze davon.

### Vernetzer b)

Die Copolymere A) können gewünschtenfalls wenigstens einen Vernetzer, d. h. eine Verbindung mit zwei oder mehr als zwei ethylenisch ungesättigten, nichtkonjugierten Doppelbindungen einpolymerisiert enthalten.

Vorzugsweise werden Vernetzer in einer Menge von 0,01 bis 5 Gew.-%, besonders bevorzugt 0,1 bis 4 Gew.-%, bezogen auf das Gesamtgewicht der zur Polymerisation eingesetzten Monomere, verwendet.

Geeignete Vernetzer b) sind zum Beispiel Acrylester, Methacrylester, Allylether oder Vinylether von mindestens zweiwertigen Alkoholen. Die OH-Gruppen der zugrunde liegenden Alkohole können dabei ganz oder teilweise verethert oder verestert sein; die Vernetzer enthalten aber mindestens zwei ethylenisch ungesättigte Gruppen.

Beispiele für die zugrunde liegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrunde liegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden. Bevorzugt sind Ethylenglykoldi(meth)acrylat und Polyethylenglykoldi(meth)acrylate.

Weitere geeignete Vernetzer b) sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer b) sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Vernetzer b) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer b) sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer b) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer b) sind Divinyldioxan, Tetraallylsilan oder Tetravinylsilan.

Selbstverständlich können auch Mischungen der vorgenannten Verbindungen b) eingesetzt werden.

Ganz besonders bevorzugt als Vernetzer b) sind Ethylenglykoldi(meth)acrylat, Polyethylenglykoldi(meth)acrylate, Pentaerythrittriallylether, Methylenbisacrylamid, N,N'-Divinylethylenharnstoff, Triallylamin und Triallylmonoalkylammoniumsalze.

### Siliconverbindung c)

Die erfindungsgemäßen Copolymere A) werden durch Polymerisation gemeinsam mit wenigstens einer Siliconverbindung hergestellt. Die Einsatzmenge der Siliconverbindung c) beträgt vorzugsweise 0,05 bis 30 Gew.-%, besonders bevorzugt 0,1 bis 20 Gew.-%, insbesondere 0,5 bis 15 Gew.-%, speziell 1 bis 10 Gew.-%, bezogen auf das Gesamtgewicht der zur Polymerisation eingesetzten Verbindungen.

Geeignete Siliconverbindungen c) sind Verbindungen, die eine radikalisch polymerisierbare olefinisch ungesättigte Doppelbindung aufweisen. Geeignet sich selbstverständlich auch Verbindungen, die sowohl wenigstens eine radikalisch polymerisierbare Doppelbindung als auch eine Polyethergruppe aufweisen.

Die erfindungsgemäßen Silicongruppen-haltigen Copolymere A) umfassen ganz allgemein die Verfahrensprodukte der radikalischen Copolymerisation, worunter z. B. reine Pfropfpolymerisate, Mischungen von Pfropfpolymerisaten mit ungepfropften Verbindungen der Komponente c), Copolymerisate der zuvor genannten Monomere sowie beliebige Mischungen verstanden werden.

Erfindungsgemäß geeignete Siliconverbindungen c), die wenigstens eine radikalisch polymerisierbare Doppelbindung aufweisen, sind radikalisch polymerisierbare, Siloxangruppen-haltige Urethan(meth)acrylate. Geeignet sind beispielsweise die in der EP-A-0 274 699 beschriebenen (Meth)acrylat-funktionalisierten Organopolysiloxan-Urethan-Copolymere, die durch Umsetzung eines mit Aminogruppen funktionalisierten Polysiloxans mit Urethan(meth)acrylat-Oligomeren erhältlich sind. Auf die Offenbarung dieses Dokuments wird hier Bezug genommen.

Bevorzugt sind die in der WO 2004/055088 beschriebenen Verbindungen. Bevorzugt wird als Verbindung c) weiterhin wenigstens ein radikalisch polymerisierbares, Siloxangruppen-haltiges Urethan(meth)acrylat eingesetzt, wie es in der WO 00/12588 beschrieben wird. Dabei handelt es sich um Siloxangruppen-haltige Urethan(meth)-acrylate c), die
a) wenigstens eine Verbindung, die mindestens ein aktives Wasserstoffatom und mindestens eine radikalisch polymerisierbare, α,β-ethylenisch ungesättigte Doppelbindung pro Molekül enthält,
b) wenigstens ein Diisocyanat,
c) wenigstens eine Verbindung, die zwei aktive Wasserstoffatome pro Molekül enthält,
d) wenigstens eine Verbindung, die mindestens ein aktives Wasserstoffatom und mindestens eine Siloxangruppe pro Molekül enthält,
eingebaut enthalten, und die Salze davon.

Im Rahmen der vorliegenden Erfindung umfasst der Ausdruck "Urethan(meth)acrylate" ganz allgemein Verbindungen, die wenigstens eine olefinisch ungesättigte radikalisch polymerisierbare Doppelbindung aufweisen. Dazu zählen auch allylisch ungesättigte Verbindungen. Weiterhin umfasst der Ausdruck "Urethan(meth)acrylate" auch Verbindungen, die Harnstoffgruppen statt oder zusätzlich zu den Urethangruppen aufweisen. Harnstoffgruppen resultieren bei der Umsetzung einer Isocyanatgruppe mit einer primären oder sekundären Aminogruppe.

### Komponente c1)

Geeignete Verbindungen c1) sind z. B. die üblichen, dem Fachmann bekannten Vinyl-verbindungen, die zusätzlich wenigstens eine gegenüber Isocyanatgruppen reaktive Gruppe aufweisen, die vorzugsweise ausgewählt ist unter Hydroxylgruppen sowie primären und sekundären Aminogruppen. Dazu zählen z. B. die Ester α,β-ethylenisch ungesättigter Mono- und Dicarbonsäuren mit mindestens zweiwertigen Alkoholen. Als α,β-ethylenisch ungesättigte Mono- und/oder Dicarbonsäuren können z. B. Acrylsäure, Methacrylsäure, Fumarsäure, Maleinsäure, Crotonsäure, Itaconsäure etc. und Gemische davon eingesetzt werden. Geeignete Alkohole sind übliche Diole, Triole und Polyole, z. B. 1,2-Ethandiol, 1,3-Propandiol, 1,4-Butandiol, 1,5-Pentandiol, 1,6-Hexandiol, 1,10-Decandiol, Diethylenglykol, 2,2,4-Trimethylpentandiol-1,5, 2,2-Dimethylpropandiol-1,3, 1,4-Dimethylolcyclohexan, 1,6-Dimethylolcyclohexan, Glycerin, Trimethylolpropan, Erythrit, Pentaerythrit, Sorbit etc. Bei den Verbindungen a) handelt es sich dann z. B. um Hydroxymethyl(meth)acrylat, Hydroxyethylethacrylat, 2-Hydroxy-ethyl(meth)acrylat, 2-Hydroxypropyl(meth)acrylat, 3-Hydroxypropyl(meth)acrylat, 3-Hydroxybutyl(meth)acrylat, 4-Hydroxybutyl(meth)acrylat, 6-Hydroxyhexyl(meth)acrylat, 3-Hydroxy-2-ethylhexyl(meth)acrylat sowie um Di(meth)acrylsäureester des 1,1,1-Trimethylolpropans oder des Glycerins.

Geeignete Monomere c1) sind weiterhin die Ester und Amide der zuvor genannten α,β-ethylenisch ungesättigten Mono- und Dicarbonsäuren mit C₂-C₁₂-Aminoalkoholen, die eine primäre oder sekundäre Aminogruppe aufweisen. Dazu zählen Aminoalkylacrylate und Aminoalkylmethacrylate und deren N-Monoalkylderivate, die z. B. einen N-C₁-C₈-Monoalkylrest tragen, wie Aminomethyl(meth)acrylat, Aminoethyl(meth)acrylat, N-Methylaminomethyl(meth)acrylat, N-Ethylaminomethyl(meth)acrylat, N-Ethylaminoethyl(meth)acrylat, N-(n-propyl)aminomethyl(meth)acrylat, N-Isopropylaminomethyl(meth)acrylat und bevorzugt tert.-Butylaminoethylacrylat und tert.-Butylaminoethylmethacrylat. Dazu zählen weiterhin N-(Hydroxy-C₁-C₁₂-alkyl)(meth)acrylamide, wie N-Hydroxymethyl(meth)acrylamid, N-Hydroxyethyl(meth)acrylamid etc.

Geeignete Monomere c1) sind auch die Amide der zuvor genannten α,β-ethylenisch ungesättigten Mono- und Dicarbonsäuren mit Di- und Polyaminen, die mindestens zwei primäre oder zwei sekundäre oder eine primäre und eine sekundäre Aminogruppe(n) aufweisen. Dazu zählen z. B. die entsprechenden Amide der Acrylsäure und Methacrylsäure, wie Aminomethyl(meth)acrylamid, Aminoethyl(meth)acrylamid, Aminopropyl(meth)acrylamid, Amino-n-butyl(meth)acrylamid, Methylaminoethyl(meth)acrylamid, Ethylaminoethyl(meth)acrylamid, Methylaminopropyl(meth)acrylamid, Ethylaminopropyl(meth)acrylamid, Methylamino-n-butyl(meth)acrylamid etc.

Geeignete Monomere c1) sind auch die Reaktionsprodukte von Epoxidverbindungen, die mindestens eine Epoxidgruppe aufweisen, mit den zuvor genannten α,β-ethylenisch ungesättigten Mono- und/oder Dicarbonsäuren und deren Anhydriden. Geeignete Epoxidverbindungen sind z. B. Glycidylether, wie Bisphenol-A-diglycidylether, Resorcindiglycidylether, 1,3-Propandioldiglycidylether, 1,4-Butandioldiglycidylether, 1,5-Pentandioldiglycidylether, 1,6-Hexandioldiglycidylether etc.

### Komponente c2)

Bei der Komponente c2) handelt es sich um übliche aliphatische, cycloaliphatische und/oder aromatische Diisocyanate, wie Tetramethylendiisocyanat, Hexamethylendiisocyanat, Methylendiphenyldiisocyanat, 2,4- und 2,6-Toluylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon. Bevorzugt handelt es sich bei der Komponente c2) um Hexamethylendiisocyanat, Isophorondiisocyanat, o- und m-Xylylendiisocyanat, Dicyclohexylmethandiisocyanat und Mischungen davon. Gewünschtenfalls können bis zu 3 Mol-% der genannten Verbindungen durch Triisocyanate ersetzt sein.

### Komponente c3)

Geeignete Verbindungen der Komponente c3) sind z. B. Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol-% der genannten Verbindungen durch Triole oder Triamine ersetzt sein.

Geeignete Diole c3) sind z. B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Neopentylglykol und/oder Cyclohexandimethylol eingesetzt.

Geeignete Aminoalkohole c3) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc.

Geeignete Diamine c3) sind z. B. Ethylendiamin, Propylendiamin, 1,4-Diaminobutan, 1,5-Diaminopentan und 1,6-Diaminohexan.

Bevorzugte Verbindungen der Komponente c3) sind Polymerisate mit einem zahlenmittleren Molekulargewicht im Bereich von etwa 300 bis 5000, bevorzugt etwa 400 bis 4000, insbesondere 500 bis 3000. Dazu zählen z. B. Polyesterdiole, Polyetherole, α,ω-Diaminopolyether und Mischungen davon. Vorzugsweise werden Ethergruppenhaltige Polymerisate eingesetzt.

Bei den Polyetherolen c3) handelt es sich vorzugsweise um Polyalkylenglykole, z. B. Polyethylenglykole, Polypropylenglykole, Polytetrahydrofurane etc., Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten.

Geeignete α,ω-Diaminopolyether c3) sind z. B. durch Aminierung von Polyalkylenoxiden mit Ammoniak herstellbar.

Geeignete Polytetrahydrofurane c3) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

Brauchbare Polyesterdiole c3) weisen vorzugsweise ein zahlenmittleres Molekulargewicht im Bereich von etwa 400 bis 5000, bevorzugt 500 bis 3000, insbesondere 600 bis 2000, auf.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, sowie Poly(meth)acrylatdiole der Formel

HO-C(-R')(-COOR")-OH

worin R' für H oder CH₃ steht und R" für C₁-C₁₈-Alkyl (insbesondere C₁-C₁₂- oder C₁-C₈-Alkyl) steht, die eine Molmasse von bis zu etwa 3000 aufweisen. Derartige Diole sind auf übliche Weise herstellbar und im Handel erhältlich (Tegomer®-Typen MD, BD und OD der Fa. Goldschmidt).

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% und bevorzugt 50 bis 85 Mol-%, des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/- 1,6-Hexandiol, 5-NaSO₃-Isophthalsäure/Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/Diethylenglykol/Dimethylolcyclohexan.

Die Verbindungen der Komponente c3) können einzeln oder als Mischungen eingesetzt werden.

### Komponente c4)

Bevorzugt ist die Komponente c4) ausgewählt unter:
- Polysiloxanen der allgemeinen Formel VI.1 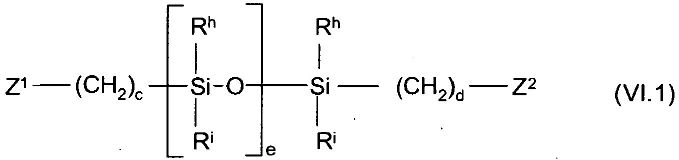 worin
   c und d unabhängig voneinander für 2 bis 8 stehen,
   e für 3 bis 100 steht,
   R^{h} und Rⁱ unabhängig voneinander für C₁-C₈-Alkyl, Benzyl oder Phenyl stehen,
   Z¹ und Z² unabhängig voneinander für OH, NHR^{k} oder einen Rest der Formel VII

   -O-(CH₂CH₂O)ᵥ(CH₂CH(CH₃)O)_{w}-H (VII)

   stehen, wobei
   in der Formel VII die Reihenfolge der Alkylenoxideinheiten beliebig ist und v und w unabhängig voneinander für eine ganze Zahl von 0 bis 200 stehen, wobei die Summe aus v und w > 0 ist,
   R^{k} für Wasserstoff, C₁-C₈-Alkyl oder C₅-C₈-Cycloalkyl steht;
- Polysiloxanen der allgemeinen Formel VI.2 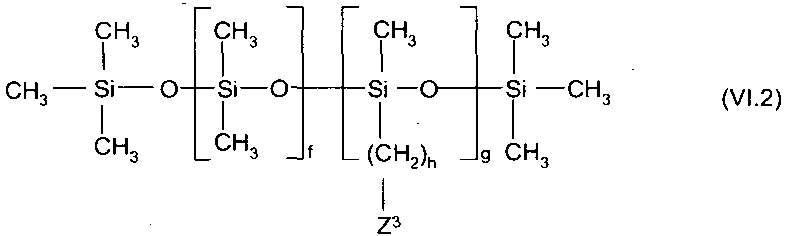 worin
   die Reihenfolge der Siloxaneinheiten beliebig ist,
   - f und g: unabhängig voneinander für 0 bis 100 stehen, wobei die Summe aus f und g mindestens 2 ist,
   - h: für eine ganze Zahl von 2 bis 8 steht,
   - Z³: für OH, NHR^{k} oder einen Rest der Formel VII steht, wobei R^{k} für Wasserstoff, C₁-C₈-Alkyl, C₅-C₈-Cycloalkyl oder einen Rest der Formel -(CH₂)ᵤ-NH₂ steht, wobei u für eine ganze Zahl von 1 bis 10, bevorzugt 2 bis 6, steht,
- Polysiloxanen mit sich wiederholenden Einheiten der allgemeinen Formel VI.3 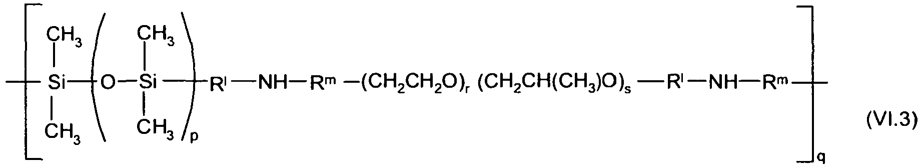 worin
   p für eine ganze Zahl von 0 bis 100 steht,
   q für eine ganze Zahl von 1 bis 8 steht,
   R^{l} und R^{m} unabhängig voneinander für C₁-C₈-Alkylen stehen,
   die Reihenfolge der Alkylenoxideinheiten beliebig ist und
   r und s unabhängig voneinander für eine ganze Zahl von 0 bis 200 stehen, wobei die Summe aus r und s > 0 ist,
- Polysiloxanen der allgemeinen Formel VI.4 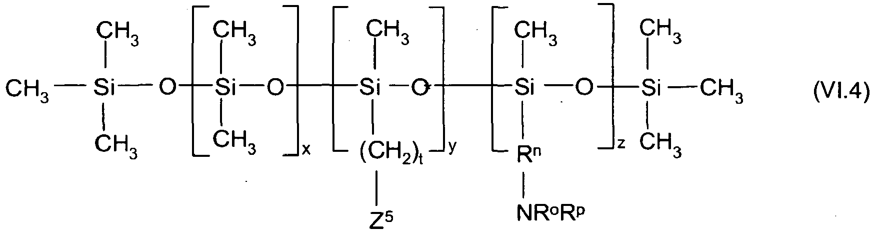 worin
   Rⁿ für einen C₁-C₈-Alkylenrest steht,
   R^{o} und R^{p} unabhängig voneinander für Wasserstoff, C₁-C₈-Alkyl oder C₅-C₈-Cycloalkyl stehen,
   die Reihenfolge der Siloxaneinheiten beliebig ist,
   x, y und z unabhängig voneinander für 0 bis 100 stehen, wobei die Summe aus x, y und z mindestens 3 ist,
   t für eine ganze Zahl von 2 bis 8 steht,
   Z⁵ für einen Rest der Formel VIII

   -(OCH₂CH₂)ᵢ(OCH₂CH(CH₃))ⱼ-R^{q} (VIII)

   steht, worin
   die Reihenfolge der Alkylenoxideinheiten beliebig ist und i und j unabhängig voneinander für eine ganze Zahl von 0 bis 200 stehen, wobei die Summe aus i und j > 0 ist,
   R^{q} für Wasserstoff oder einen C₁- C₈-Alkylrest steht.
und Mischungen davon.

Nach einer geeigneten Ausführungsform weisen die Polysiloxane c4) der allgemeinen Formel VI.1 keine Alkylenoxidreste der allgemeinen Formel VII auf. Diese Polysiloxane c4) weisen dann vorzugsweise ein zahlenmittleres Molekulargewicht im Bereich von etwa 300 bis 5000, bevorzugt 400 bis 3000 auf.

Geeignete Polysiloxane c4), die keine Alkylenoxidreste aufweisen, sind z. B. die Tegomer®-Marken der Fa. Goldschmidt.

Nach einer weiteren geeigneten Ausführungsform handelt es sich bei den Polysiloxanen c4) um Siliconpoly(alkylenoxid)-Copolymere der Formel VI.1, wobei wenigstens einer oder beide Reste Z¹ und/oder Z² für einen Rest der allgemeinen Formel VII stehen.

Vorzugsweise ist in der Formel VII die Summe aus v und w so gewählt, dass das Molekulargewicht der Polysiloxane c4) dann in einem Bereich von etwa 300 bis 30000 liegt.

Bevorzugt liegt die Gesamtzahl der Alkylenoxideinheiten der Polysiloxane c4), d. h. die Summe aus v und w in der Formel VII dann in einem Bereich von etwa 3 bis 200, bevorzugt 5 bis 180.

Nach einer weiteren geeigneten Ausführungsform handelt es sich bei den Polysiloxanen c4) um Siliconpoly(alkylenoxid)-Copolymere der Formel VI.2, die wenigstens einen Rest Z³ der allgemeinen Formel VII aufweisen.

Vorzugsweise ist dann in der Formel VII die Summe aus v und w wiederum so gewählt, dass das Molekulargewicht der Polysiloxane c4) dann in einem Bereich von etwa 300 bis 30000 liegt. Bevorzugt liegt die Gesamtzahl der Alkylenoxideinheiten der Polysiloxane c4), d. h. die Summe aus v und w in der Formel VII dann ebenfalls in einem Bereich von etwa 3 bis 200, bevorzugt 5 bis 180.

Geeignete Siliconpoly(alkylenoxid)-Copolymere c4), die unter dem internationalen Freinamen Dimethicon bekannt sind, sind die Tegopren®-Marken der Fa. Goldschmidt, Belsil® 6031 der Fa. Wacker und Silvet® L der Fa. Witco.

Nach einer bevorzugten Ausführungsform handelt es sich bei den Polysiloxanen c4) um Siliconpoly(alkylenoxid)-Copolymere der Formel VI.2, die wenigstens einen Rest Z³ aufweisen, worin Z³ für NHR^{k} steht und R³ für Wasserstoff oder einen Rest der Formel -(CH₂)ᵤ-NH₂ steht. Vorzugsweise steht u für eine ganze Zahl von 1 bis 10, bevorzugt 2 bis 6. Dazu zählen z. B. die MAN- und MAR-Marken der Fa. Hüls sowie die Finish-Marken der Fa. Wacker, z. B. Finish WT 1270.

Bevorzugt umfassen die Polysiloxane c4) wenigstens eine Verbindung der allgemeinen Formel VI.3. Bevorzugt stehen in der Formel VI.3 R^{l} und R^{m} unabhängig voneinander für einen C₂-C₄-Alkylenrest. Insbesondere stehen R^{l} und R^{m} unabhängig voneinander für einen C₂-C₃-Alkylenrest.

Vorzugsweise liegt das Molekulargewicht der Verbindung der Formel VI.3 in einem Bereich von etwa 300 bis 100000 liegt.

Vorzugsweise steht in der Formel VI.3 p für eine ganze Zahl von 1 bis 20, wie z. B. 2 bis 10.

Bevorzugt liegt die Gesamtzahl der Alkylenoxideinheiten der Verbindung der Formel VI.3, d. h. die Summe aus r und s, in einem Bereich von etwa 3 bis 200, bevorzugt 5 bis 180.

Bevorzugt sind die Endgruppen der Polysiloxane mit Wiederholungseinheiten der allgemeinen Formel VI.3 ausgewählt unter (CH₃)₃SiO, H, C₁-C₈-Alkyl und Mischungen davon.

Aminogruppen-haltige Verbindungen mit Wiederholungseinheiten der allgemeinen Formel VI.3 weisen bevorzugt eine Aminzahl in einem Bereich von etwa 2 bis 50, insbesondere 3 bis 20 auf.

Geeignete alkoxylierte Siloxanamine der Formel VI.3 sind z. B. in der WO-A-97/32917 beschrieben, auf die hier in vollem Umfang Bezug genommen wird. Kommerziell erhältliche Verbindungen sind z. B. die Silsoft®-Marken der Fa. Witco, z. B. Silsoft® A-843.

Bevorzugt steht in der Formel VI.4 der Rest Rⁿ für einen C₂-C₄-Alkylenrest.

Bevorzugt stehen in der Formel VI.4 R^{o} und R^{p} unabhängig voneinander für Wasserstoff oder C₁-C₄-Alkyl.

Vorzugsweise wird die Summe aus x, y und z so gewählt, dass das Molekulargewicht der Verbindung der Formel VI.4 in einem Bereich von etwa 300 bis 100000, bevorzugt 500 bis 50000, liegt.

Bevorzugt liegt die Gesamtzahl der Alkylenoxideinheiten des Restes der Formel VIII, d. h. die Summe aus i und j, in einem Bereich von etwa 3 bis 200, bevorzugt 5 bis 80.

Bevorzugt steht in der Formel VIII der Rest R^{q} für Wasserstoff oder C₁-C₄-Alkyl.

Eine geeignete Verbindung der Formel VI.4 ist z. B. Silsoft® A-858 der Fa. Witco.

Geeignete Polysiloxane c4) sind auch die in der EP-A-277 816 beschriebenen Polydimethylsiloxane.

Gegebenenfalls enthalten die erfindungsgemäßen Urethan(meth)acrylate zusätzlich wenigstens eine Komponente eingebaut, die ausgewählt ist unter

c5) Verbindungen, die zwei oder mehrere aktive Wasserstoffatome und mindestens eine ionogene und/oder ionische Gruppe pro Molekül enthalten,

c6) einwertigen Alkoholen, Aminen mit einer primären oder sekundären Aminogruppe, aliphatischen, cycloaliphatischen oder aromatischen Monoisocyanaten und Mischungen davon,

c7) α,β-ethylenisch ungesättigten Verbindungen, die zusätzlich wenigstens eine Isocyanatgruppe pro Molekül enthalten,
und Mischungen davon.

### Monomer d)

In einer bevorzugten Ausführungsform enthalten die erfindungsgemäßen Copolymere zusätzlich zu den zuvor genannten Monomeren a) bis c) wenigstens ein weiteres amidgruppenhaltiges Monomer d) der allgemeinen Formel I einpolymerisiert 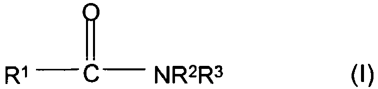 wobei
einer der Reste R¹ bis R³ für eine Gruppe der Formel CH₂=CR⁴- mit R⁴ = H oder C₁-C₄-Alkyl steht und die übrigen Reste R¹ bis R³ unabhängig voneinander für H, Alkyl, Cycloalkyl, Heterocycloalkyl, Aryl oder Hetaryl stehen,
wobei R¹ und R² gemeinsam mit der Amidgruppe, an die sie gebunden sind, auch für ein Lactam mit 5 bis 8 Ringatomen stehen können,
wobei R² und R³ gemeinsam mit dem Stickstoffatom, an das sie gebunden sind, auch für einen fünf- bis siebengliedrigen Heterocyclus stehen können,
mit der Maßgabe, dass die Summe der Kohlenstoffatome der Reste R¹, R² und R³ höchstens 8 beträgt.

Vorzugsweise weisen die Verbindungen der Komponente d) zusätzlich zu dem Carbonyl-Kohlenstoffatom der Amidgruppe höchstens 7 weitere Kohlenstoffatome auf

Bevorzugt sind die Verbindungen der Komponente d) ausgewählt unter primären Amiden α,β-ethylenisch ungesättigter Monocarbonsäuren, N-Vinylamiden gesättigter Monocarbonsäuren, N-Vinyllactamen, N-Alkyl- und N,N-Dialkylamiden α,β-ethylenisch ungesättigter Monocarbonsäuren und Mischungen davon.

Bevorzugte Monomere d) sind N-Vinyllactame und deren Derivate, die z. B. einen oder mehrere C₁-C₆-Alkylsubstituenten, wie Methyl, Ethyl, n-Propyl, Isopropyl, n-Butyl, sec.-Butyl, tert.-Butyl etc. aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc.

Besonders bevorzugt werden N-Vinylpyrrolidon und N-Vinylcaprolactam eingesetzt.

Geeignete Monomere d) sind weiterhin Acrylsäureamid und Methacrylsäureamid.

Geeignete N-Alkyl- und N,N-Dialkylamide α,β-ethylenisch ungesättigter Monocarbonsäuren, die zusätzlich zu dem Carbonyl-Kohlenstoffatom der Amidgruppe höchstens 7 weitere Kohlenstoffatome aufweisen, sind beispielsweise N-Methyl(meth)acrylamid, N-Ethyl(meth)acrylamid, N-Propyl(meth)acrylamid, N-(n-Butyl)(meth)acrylamid, N-tert-Butyl(meth)acrylamid, n-Pentyl(meth)acrylamid, n-Hexyl(meth)acrylamid, n-Heptyl(meth)acrylamid, N,N-Dimethyl(meth)acrylamid, N,N-Diethyl(meth)acrylamid, Piperidinyl(meth)acrylamid, Morpholinyl(meth)acrylamid und Mischungen davon.

Als Monomere d) geeignete offenkettige N-Vinylamidverbindungen sind beilspielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid, N-Vinyl-butyramid und Mischungen davon. Bevorzugt wird N-Vinylformamid eingesetzt.

Als Monomere d) eignen sich auch Verbindungen der Formel

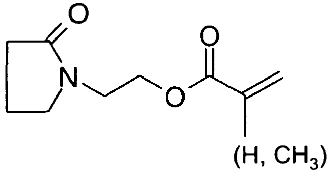

Besonders bevorzugt werden N-Vinylpyrrolidon, N-Vinylcaprolactam, N-Vinylformamid und die Verbindungen der obigen Formel eingesetzt.

Die erfindungsgemäßen Copolymere A) enthalten vorzugsweise 5 bis 95 Gew.-%, besonders bevorzugt 10 bis 90 Gew.-%, bezogen auf das Gesamtgewicht der zur Polymerisation eingesetzten Verbindungen, wenigstens eines Monomers d) einpolymerisiert.

### Monomer e)

Die erfindungsgemäßen Copolymere A) können zusätzlich wenigstens ein hydrophobes Monomer e) einpolymerisiert enthalten. Vorzugsweise enthalten die Copolymere A) dann 0,1 bis 30 Gew.-%, besonders bevorzugt 0,2 bis 20 Gew.-%, insbesondere 0,5 bis 15 Gew.-%, bezogen auf das Gesamtgewicht der zur Polymerisation eingesetzten Verbindungen, wenigstens eines hydrophoben Monomers e) einpolymerisiert.

Geeignete Verbindungen e) sind ausgewählt ist unter Verbindungen der allgemeinen Formeln III a), III b), III c), III d) und III e) 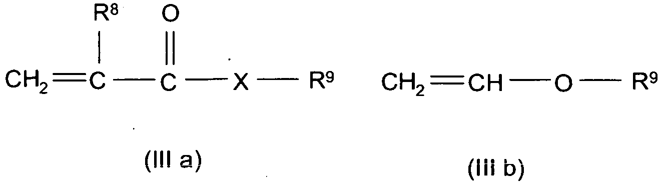 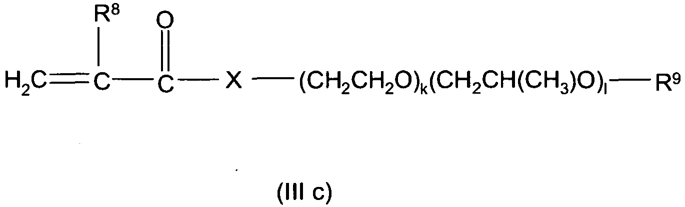 H₂C=CH-CH₂-O-(CH₂CH₂O)ₖ(CH₂CH(CH₃)O)ₗ-R⁹ (III d)

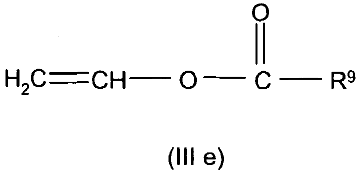

worin
die Reihenfolge der Alkylenoxideinheiten beliebig ist,
- k und l: unabhängig voneinander für eine ganze Zahl von 0 bis 1000 stehen, wobei die Summe aus k und l mindestens 5 beträgt,
- R⁸: für Wasserstoff oder C₁-C₄-Alkyl, vorzugsweise Methyl, steht,
- R⁹: für C₈-C₃₀-Alkyl oder C₈-C₃₀-Alkenyl steht, und
- X: für O oder eine Gruppe der Formel NR¹⁰ steht, worin R¹⁰ für H, Alkyl, Alkenyl, Cycloalkyl, Heterocycloalkyl, Aryl oder Hetaryl steht.

Geeignete Monomere der Formel III a) worin X für O steht sind z. B. n-Octyl(meth)acrylat,1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon.

Geeignete Monomere der Formel III a) worin X für NR¹⁰ steht sind z. B. n-Octyl(meth)acrylamid,1,1,3,3-Tetramethylbutyl(meth)acrylamid, Ethylhexyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arrachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid, N-Methyl-N-(n-octyl)(meth)acrylamid, N,N-Di-(n-octyl)(meth)acrylamid und Mischungen davon.

Geeignete Monomere der Formel III b) sind C₈-C₂₂-Alkylvinylether, z. B. n-Octylvinylether, 1,1,3,3-Tetramethylbutylvinylether, Ethylhexylvinylether, n-Nonylvinylether, n-Decylvinylether, n-Undecylvinylether, Tridecylvinylether, Myristylvinylether, Pentadecylvinylether, Palmitylvinylether, Heptadecylvinylether, Octadecylvinylether, Nonadecylvinylether, Arrachinylvinylether, Behenylvinylether, Lignocerenylvinylether, Cerotinylvinylether, Melissinylvinylether, Palmitoleinylvinylether, Oleylvinylether, Linolylvinylether, Linolenylvinylether, Stearylvinylether, Laurylvinylether und Mischungen davon.

In den Formeln III c) und III d) steht k vorzugsweise für eine ganze Zahl von 1 bis 500, insbesondere 3 bis 250. Bevorzugt steht I für eine ganze Zahl von 0 bis 100.

Bevorzugt steht R⁸ in der Formel III c) für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R⁹ in den Formeln III c) und III d) für n-Octyl, 1,1,3,3-Tetramethylbutyl, Ethylhexyl, n-Nonyl, n-Decyl, n-Undecyl, Tridecyl, Myristyl, Pentadecyl, Palmityl, Heptadecyl, Octadecyl, Nonadecyl, Arrachinyl, Behenyl, Lignocerenyl, Cerotinyl, Melissinyl, Palmitoleinyl, Oleyl, Linolyl, Linolenyl, Stearyl, Lauryl.

Vorzugsweise steht X in der Formel III c) für O oder NH.

Geeignete Polyetheracrylate III c) sind z. B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Starteralkohol R⁹-OH hergestellt werden. Die Alkylenoxide können einzeln, alternierend nacheinander oder als Mischung eingesetzt werden. Die Polyetheracrylate III c) können allein oder in Mischungen zur Herstellung der erfindungsgemäß eingesetzten Polymere verwendet werden.

Geeignete Allylalkoholalkoxilate III d) sind z. B. die Veretherungsprodukte von Allylchlorid mit entsprechenden Polyetherolen. Geeignete Polyetherole können leicht durch Umsetzung von Ethylenoxid, 1,2-Propylenoxid und/oder Epichlorhydrin mit einem Starteralkohol R⁹-OH hergestellt werden. Die Alkylenoxide können einzeln, alternierend nacheinander oder als Mischung eingesetzt werden. Die Allylalkoholalkoxilate III d) können allein oder in Mischungen zur Herstellung der erfindungsgemäß eingesetzten Polymere verwendet werden.

Geeignete Monomere III e) sind C₈-C₃₀-, vorzugsweise C₈-C₂₂-Carbonsäurevinylester. Dazu zählen z. B. n-Octylvinylester, 1,1,3,3-Tetramethylbutylvinylester, Ethylhexylvinylester, n-Nonylvinylester, n-Decylvinylester, n-Undecylvinylester, Tridecylvinylester, Myristylvinylester, Pentadecylvinylester, Palmitylvinylester, Heptadecylvinylester, Octadecylvinylester, Nonadecylvinylester, Arrachinylvinylester, Behenylvinylester, Lignocerenylvinylester, Cerotinylvinylester, Melissinylvinylester, Palmitoleinylvinylester, Oleylvinylester, Linolylvinylester, Linolenylvinylester, Stearylvinylester, Laurylvinylester und Mischungen davon.

### Monomer f)

Die erfindungsgemäßen Copolymere A) können zusätzlich wenigstens ein Monomer f) einpolymerisiert enthalten, das ausgewählt ist unter von Komponente e) verschiedenen Estern α,β-ethylenisch ungesättigter Mono- und Dicarbonsäuren mit C₁-C₇-Alkanolen, von III c) verschiedenen Polyetheracrylaten, C₁-C₇-Alkylvinylethern, von III d) verschiedenen Allylalkoholalkoxilaten und Estern des Vinylalkohols mit C₁-C₇-Monocarbonsäuren.

Vorzugsweise beträgt der Anteil an Monomeren f) bis zu 40 Gew.-%, bezogen auf das Gesamtgewicht der zur Polymerisation eingesetzten Verbindungen. Eine geeignete Einsatzmenge für zusätzliche Monomere f) liegt in einem Bereich von 0,1 bis 30 Gew.-%, insbesondere 1 bis 25 Gew.-%, bezogen auf das Gesamtgewicht der zur Polymerisation eingesetzten Verbindungen.

Vorzugsweise ist die Verbindung f) ausgewählt ist unter Verbindungen der allgemeinen Formeln III a*), III b*), III c*), III d*) und III e*) 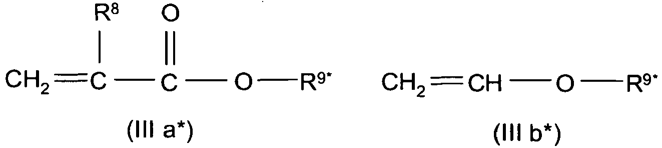 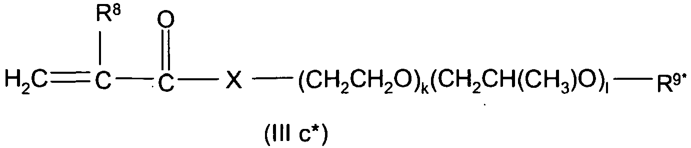 H₂C=CH -CH₂-O-(CH₂CH₂O)ₖ(CH₂CH(CH₃)O)ₗ-R^{9*} (III d*)

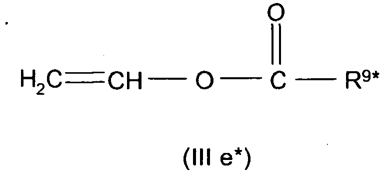

worin
die Reihenfolge der Alkylenoxideinheiten beliebig ist,
- k und l: unabhängig voneinander für eine ganze Zahl von 0 bis 1000 stehen, wobei die Summe aus k und l mindestens 5 beträgt,
- R⁸: für Wasserstoff oder C₁-C₄-Alkyl, vorzugsweise Methyl, steht,
- R^{9*}: für Wasserstoff, C₁-C₈-Alkyl oder C₃-C₈-Alkenyl steht, und
- X: für O oder eine Gruppe der Formel NR¹⁰ steht, worin R¹⁰ für H, Alkyl, Alkenyl, Cycloalkyl, Heterocycloalkyl, Aryl oder Hetaryl steht.

Geeignete Ester α,β-ethylenisch ungesättigter Mono- und Dicarbonsäuren mit C₁-C₇-Alkanolen sind z. B. Methyl(meth)acrylat, Methylethacrylat, Ethyl(meth)acrylat, Ethylethacrylat, n-Butyl(meth)acrylat, tert.-Butyl(meth)acrylat, tert.-Butylethacrylat, n-Pentyl(meth)acrylat, n-Hexyl(meth)acrylat, n-Heptyl(meth)acrylat, etc. Bevorzugte Monomere f) sind die Ester α,β-ethylenisch ungesättigter Mono- und Dicarbonsäuren mit C₁-C₃-Alkanolen, insbesondere Methylmethacrylat.

In den Formeln III c*) und III d*) steht k vorzugsweise für eine ganze Zahl von 1 bis 500, insbesondere 3 bis 250. Bevorzugt steht l für eine ganze Zahl von 0 bis 100.

Bevorzugt steht R⁸ in der Formel III c*) für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R^{9*} in den Formeln III c*) und III d*) für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht X in der Formel III c*) für O oder NH.

Geeignete Polyetheracrylate III c*) sind z. B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit Wasser oder einem Starteralkohol R^{9*}-OH hergestellt werden. Die Alkylenoxide können einzeln, alternierend nacheinander oder als Mischung eingesetzt werden. Die Polyetheracrylate III c*) können allein oder in Mischungen zur Herstellung der erfindungsgemäß eingesetzten Polymere verwendet werden.

Geeignete Allylalkoholalkoxilate III d*) sind z. B. die Veretherungsprodukte von Allylchlorid mit entsprechenden Polyetherolen. Geeignete Polyetherole können leicht durch Umsetzung von Ethylenoxid, 1,2-Propylenoxid und/oder Epichlorhydrin mit Wasser oder einem Starteralkohol R^{9*}-OH hergestellt werden. Die Alkylenoxide können einzeln, alternierend nacheinander oder als Mischung eingesetzt werden. Die Allylalkoholalkoxilate III d*) können allein oder in Mischungen zur Herstellung der erfindungsgemäß eingesetzten Polymere verwendet werden.

Geeignete zusätzliche Monomere III e*)) sind weiterhin Vinylacetat, Vinylpropionat, Vinylbutyrat und Mischungen davon.

### Monomer g)

Die erfindungsgemäßen Copolymere A) können zusätzlich wenigstens ein von den Komponenten a) bis f) verschiedenes, damit copolymerisierbares Monomer g) einpolymerisiert enthalten.

Vorzugsweise beträgt der Anteil an Monomeren g) bis zu 40 Gew.-%, bezogen auf das Gesamtgewicht der zur Polymerisation eingesetzten Verbindungen. Eine geeignete Einsatzmenge für zusätzliche Monomere g) liegt in einem Bereich von 0,1 bis 25 Gew.-%, insbesondere 0,5 bis 20 Gew.-%, bezogen auf das Gesamtgewicht der zur Polymerisation eingesetzten Verbindungen.

Vorzugsweise ist die Komponente g) ausgewählt unter Estern α,β-ethylenisch ungesättigter Mono- und Dicarbonsäuren mit C₂-C₃₀-Diolen, Amiden α,β-ethylenisch ungesättigter Mono- und Dicarbonsäuren mit C₂-C₃₀-Aminoalkoholen, die eine primäre oder sekundäre Aminogruppe aufweisen, Estern von Vinylalkohol und Allylalkohol mit C₁-C₇-Monocarbonsäuren, von III c) und III c*) verschiedenen Polyetheracrylaten, Vinylaromaten, Vinylhalogeniden, Vinylidenhalogeniden, C₁-C₈-Monoolefinen, nicht aromatischen Kohlenwasserstoffen mit mindestens zwei konjugierten Doppelbindungen und Mischungen davon.

Geeignete zusätzliche Monomere g) sind weiterhin 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat und 3-Hydroxy-2-ethylhexylmethacrylat.

Geeignete zusätzliche Monomere g) sind weiterhin 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.

Geeignete Polyetheracrylate g) sind auch Urethan(meth)acrylate mit Alkylenoxidgruppen. Derartige Verbindungen sind in der DE 198 38 851 (Komponente e2)) beschrieben, worauf hier in vollem Umfang Bezug genommen wird.

Geeignete zusätzliche Monomere g) sind weiterhin Ethylen, Propylen, Isobutylen, Butadien, Styrol, α-Methylstyrol, Acrylnitril, Methacrylnitril, Vinylchlorid, Vinylidenchlorid, Vinylfluorid, Vinylidenfluorid und Mischungen davon.

Die zuvor genannten zusätzlichen Monomere g) können jeweils einzeln oder in Form von beliebigen Mischungen eingesetzt werden.

### Besonders bevorzugt ist ein Copolymer A), das

- wenigstens 2 Gew.-% mindestens einer Verbindung a1) mit einer radikalisch polymerisierbaren, α,β-ethylenisch ungesättigten Doppelbindung und mindestens eine anionogenen und/oder anionischen Gruppe pro Molekül, vorzugsweise Acrylsäure und/oder Methacrylsäure,
- 0,05 bis 5 Gew.-% wenigstens eines Vernetzers b), vorzugsweise Ethylenglykoldi(meth)acrylat und/oder Pentaerythrittriallylether,
- 0,05 bis 30 Gew.-% wenigstens einer Siliconverbindung c),
einpolymerisiert enthält.

Besonders bevorzugt ist weiterhin ein Copolymer A), das
- wenigstens 2 Gew.-% mindestens einer Verbindung a1) mit einer radikalisch polymerisierbaren, α,β-ethylenisch ungesättigten Doppelbindung und mindestens einer anionogenen und/oder anionischen Gruppe pro Molekül, vorzugsweise Acrylsäure und/oder Methacrylsäure,
- 0,05 bis 5 Gew.-% wenigstens eines Vernetzer b), vorzugsweise Ethylenglykoldi(meth)acrylat und/oder Pentaerythrittriallylether,
- 0,05 bis 30 Gew.-% wenigstens einer Siliconverbindung c),
- 20 bis 95 Gew.-% Vinylpyrrolidon d),
einpolymerisiert enthält. Anstelle von oder zusätzlich zu Vinylpyrrolidon kann auch Vinylcaprolactam eingesetzt werden.

Besonders bevorzugt ist weiterhin ein Copolymer A), das
- wenigstens 2 Gew.-% mindestens einer Verbindung a1) mit einer radikalisch polymerisierbaren, α,β-ethylenisch ungesättigten Doppelbindung und mindestens eine anionogenen und/oder anionischen Gruppe pro Molekül, vorzugsweise Acrylsäure und/oder Methacrylsäure,
- 0,05 bis 5 Gew.-% wenigstens eines Vernetzers b), vorzugsweise Ethylenglykoldi(meth)acrylat und/oder Pentaerythrittriallylether,
- 0,05 bis 30 Gew.-% wenigstens einer Siliconverbindung c),
- 0,1 bis 20 Gew.-% wenigstens einer Verbindung e), die vorzugsweise ausgewählt ist unter C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon,
einpolymerisiert enthält.

Besonders bevorzugt ist weiterhin ein Copolymer A), das
- wenigstens 2 Gew.-% mindestens einer Verbindung a1) mit einer radikalisch polymerisierbaren, α,β-ethylenisch ungesättigten Doppelbindung und mindestens einer anionogenen und/oder anionischen Gruppe pro Molekül, vorzugsweise Acrylsäure und/oder Methacrylsäure,
- 0,05 bis 5 Gew.-% wenigstens eines Vernetzers b), vorzugsweise Ethylenglykoldi(meth)acrylat und/oder Pentaerythrittriallylether,
- 0,05 bis 30 Gew.-% wenigstens einer Siliconverbindung c),
- 5 bis 40 Gew.-% wenigstens eines Monomers f), das vorzugsweise ausgewählt ist unter C₁-C₆-(Meth)acrylaten, insbesondere Methyl(meth)acrylat, Ethyl(meth)acrylat, n-Butyl(meth)acrylat und Mischungen davon,
einpolymerisiert enthält.

Die zuvor beschriebenen Ausführungsformen des Copolymers A) können zusätzlich noch wenigstens eine Verbindung a2), vorzugsweise wenigstens eine N-Vinylimidazol-Verbindung, einpolymerisiert enthalten. Die zuvor beschriebenen Ausführungsformen des Copolymers A) können anstelle von der zusätzlich zu wenigstens einer N-Vinylimidazol-Verbindung wenigstens eine davon verschiedene Verbindung a2) einpolymerisiert enthalten. Diese ist vorzugsweise ausgewählt unter N-[3-(dimethylamino)propyl]acrylamid, N-[3-(dimethylamino)propyl]methacrylamid und Mischungen davon.

Besonders bevorzugt ist weiterhin ein Copolymer A), das
- wenigstens 2 Gew.-% mindestens einer Verbindung a1) mit einer radikalisch polymerisierbaren, α,β-ethylenisch ungesättigten Doppelbindung und mindestens eine anionogenen und/oder anionischen Gruppe pro Molekül, vorzugsweise Acrylsäure und/oder Methacrylsäure,
- 0,05 bis 5 Gew.-% wenigstens eines Vernetzers b), vorzugsweise Ethylenglykoldi(meth)acrylat und/oder Pentaerythrittriallylether,
- 0,05 bis 30 Gew.-% wenigstens einer Siliconverbindung c),
- 20 bis 95 Gew.-% Vinylpyrrolidon d),
- 0,1 bis 20 Gew.-% wenigstens einer Verbindung e), die vorzugsweise ausgewählt ist unter C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon,
- 5 bis 40 Gew.-% wenigstens eines Monomers f), das vorzugsweise ausgewählt ist unter C₁-C₆-(Meth)acrylaten, insbesondere Methyl(meth)acrylat, Ethyl(meth)acrylat, n-Butyl(meth)acrylat und Mischungen davon,
einpolymerisiert enthält. Anstelle von oder zusätzlich zu Vinylpyrrolidon kann auch Vinylcaprolactam eingesetzt werden.

Besonders bevorzugte Silicongruppen-haltige Copolymere A) sind weiterhin erhältlich durch radikalische Copolymerisation von
- wenigstens 2 Gew.-%, bezogen auf das Gesamtgewicht der zur Polymerisation eingesetzten Monomere, wenigstens eines Monomerenpaars aus wenigstens einer N-Vinylimidazol-Verbindung a2) und Acrylsäure und/oder Methacrylsäure a1),
- wenigstens 1 Gew.-%, bezogen auf das Gesamtgewicht der zur Polymerisation eingesetzten Monomere, wenigstens eines zusätzlichen Monomers a1) mit einer anionogenen oder anionischen Gruppe oder wenigstens eines zusätzlichen Monomers a2) mit einer kationogenen oder kationischen Gruppe,
- 0,05 bis 5 Gew.-%, bezogen auf das Gesamtgewicht der zur Polymerisation eingesetzten Monomere, wenigstens einer radikalisch polymerisierbaren vernetzenden Verbindung b),
- 0,05 bis 30 Gew.-%, besonders bevorzugt 0,1 bis 20 Gew.-% wenigstens einer Siliconverbindung c),
- 0 bis 95 Gew.-% wenigstens eines amidgruppenhaltigen Monomers d),
- 0 bis 40 Gew.-%, bezogen auf das Gesamtgewicht der zur Polymerisation eingesetzten Monomere, wenigstens eines hydrophoben Monomers e),
- 0 bis 40 Gew.-%, bezogen auf das Gesamtgewicht der zur Polymerisation eingesetzten Monomere, wenigstens eines Monomers f).

Eine spezielle Ausführungsform der erfindungsgemäßen Copolymere A) sind anionisch ampholytische Copolymere. Diese enthalten als Komponente a1) vorzugsweise Acrylsäure, Methacrylsäure oder eine Mischung davon. Bevorzugt als Komponente a2) sind N-Vinylimidazol, N-[3-(dimethylamino)propyl]acrylamid, N-[3-(dimethylamino)propyl]methacrylamid und Mischungen davon.

Bevorzugte anionisch ampholytische Copolymere A) sind erhältlich durch radikalische Copolymerisation von
- Methacrylsäure und/oder Acrylsäure a1),
- wenigstens einer Verbindung a2), ausgewählt unter N-Vinylimidazol, N-[3-(dimethylamino)propyl]acrylamid, N-[3-(dimethylamino)propyl]methacrylamid und Mischungen davon,
- wenigstens einem Vernetzer b), vorzugsweise Ethylenglykoldi(meth)acrylat und/oder Pentaerythrittriallylether,
- wenigstens einer Siliconverbindung c),
wobei das molare Verhältnis von anionogenen/anionischen Gruppen zu kationogenen/kationischen Gruppen wenigstens 1 : 1 beträgt.

Bevorzugte anionisch ampholytische Copolymere A) sind weiterhin erhältlich durch radikalische Copolymerisation von
- Methacrylsäure und/oder Acrylsäure a1),
- wenigstens einer Verbindung a2), ausgewählt unter N-Vinylimidazol, N-[3-(dimethylamino)propyl]acrylamid, N-[3-(dimethylamino)propyl]methacrylamid und Mischungen davon,
- wenigstens einem Vernetzer b), vorzugsweise Ethylenglykoldi(meth)acrylat und/oder Pentaerythrittriallylether,
- wenigstens einer Siliconverbindung c),
- 3 bis 35 Gew.-%, bezogen auf das Gesamtgewicht der zur Polymerisation eingesetzten Monomere, wenigstens eines Monomers f), das vorzugsweise ausgewählt ist unter C₁-C₆-(Meth)acrylaten,
wobei das molare Verhältnis von anionogenen/anionischen Gruppen zu kationogenen/kationischen Gruppen wenigstens 1,2 : 1 beträgt.

Bevorzugte anionisch ampholytische Copolymere A) sind weiterhin erhältlich durch radikalische Copolymerisation von
- Methacrylsäure und/oder Acrylsäure a1),
- wenigstens einer Verbindung a2), ausgewählt unter N-Vinylimidazol, N-[3-(dimethylamino)propyl]acrylamid, N-[3-(dimethylamino)propyl]methacrylamid und Mischungen davon,
- wenigstens einem Vernetzer b), vorzugsweise Ethylenglykoldi(meth)acrylat und/oder Pentaerythrittriallylether,
- wenigstens einer Siliconverbindung c),
- 0,1 bis 10 Gew.-%, bezogen auf das Gesamtgewicht der zur Polymerisation eingesetzten Monomere, wenigstens einer Verbindung e), die vorzugsweise ausgewählt ist unter C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon,
wobei das molare Verhältnis von anionogenen/anionischen Gruppen zu kationogenen/kationischen Gruppen wenigstens 1,4 : 1 beträgt.

Bei den drei letztgenannten anionisch ampholytischen Copolymeren kann bis zu 60 Gew.-% der Komponente a1), bezogen auf das Gesamtgewicht der Monomere a1), durch wenigstens ein Monomer d), vorzugsweise Vinylpyrrolidon und/oder Vinylcaprolactam, ersetzt werden.

Bevorzugt sind weiterhin anionisch ampholytische Copolymere A), zu deren Herstellung zumindest ein Teil der Monomere a1) und a2) in Form eines Monomerenpaares eingesetzt werden.

Besonders bevorzugte anionische Silicongruppen-haltige Copolymere A) sind erhältlich durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol a2) und Acrylsäure und/oder Methacrylsäure a1),
- 5 bis 70 Gew.-% Methacrylsäure und/oder Acrylsäure a1),
- 0,1 bis 2 Gew.-% wenigstens eines Vernetzers b), vorzugsweise Ethylenglykoldi(meth)acrylat und/oder Pentaerythrittriallylether,
- 0,05 bis 30 Gew.-%, besonders bevorzugt 0,1 bis 20 Gew.-% wenigstens einer Siliconverbindung c),
- 20 bis 95 Gew.-% Vinylpyrrolidon d),
- 0,1 bis 20 Gew.-% wenigstens einer Verbindung e), die vorzugsweise ausgewählt ist unter C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon,
- 5 bis 40 Gew.-% wenigstens eines Monomers f), das vorzugsweise ausgewählt ist unter C₁-C₆-(Meth)acrylaten, insbesondere Methyl(meth)acrylat, Ethyl(meth)acrylat, n-Butyl(meth)acrylat und Mischungen davon.

Anstelle von oder zusätzlich zu Vinylpyrrolidon kann auch Vinylcaprolactam eingesetzt werden. Anstelle von oder zusätzlich zu Vinylimidazol kann auch N-[3-(dimethylamino)propyl]acrylamid und/oder
N-[3-(dimethylamino)propyl]methacrylamid eingesetzt werden.

Besonders bevorzugte anionische Silicongruppen-haltige Copolymere A) sind weiterhin erhältlich durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol a2) und Acrylsäure und/oder Methacrylsäure a1),
- 5 bis 70 Gew.-% Methacrylsäure und/oder Acrylsäure a1),
- 0,1 bis 2 Gew.-% wenigstens eines Vernetzers b), vorzugsweise Ethylenglykoldi(meth)acrylat und/oder Pentaerythrittriallylether,
- 0,05 bis 30 Gew.-%, besonders bevorzugt 0,1 bis 20 Gew.-% wenigstens einer Siliconverbindung c),
- 20 bis 85 Gew.-% Vinylpyrrolidon d),
- 5 bis 40 Gew.-% wenigstens eines weiteren Monomers f), das vorzugsweise ausgewählt ist unter C₁-C₆-(Meth)acrylaten, insbesondere Methyl(meth)acrylat, Ethyl(meth)acrylat, n-Butyl(meth)acrylat und Mischungen davon.

Anstelle von oder zusätzlich zu Vinylpyrrolidon kann auch Vinylcaprolactam eingesetzt werden. Anstelle von oder zusätzlich zu Vinylimidazol kann auch N-[3-(dimethylamino)propyl]acrylamid und/oder
N-[3-(dimethylamino)propyl]methacrylamid eingesetzt werden.

Besonders bevorzugte anionische Silicongruppen-haltige Copolymere A) sind weiterhin erhältlich durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol a2) und Acrylsäure und/oder Methacrylsäure a1),
- 5 bis 70 Gew.-% Methacrylsäure und/oder Acrylsäure a1),
- 0,1 bis 2 Gew.-% wenigstens eines Vernetzers b), vorzugsweise Ethylenglykoldi(meth)acrylat und/oder Pentaerythrittriallylether,
- 0,05 bis 30 Gew.-%, besonders bevorzugt 0,1 bis 20 Gew.-% wenigstens einer Siliconverbindung c),
- 20 bis 85 Gew.-% Vinylpyrrolidon d),
- 1 bis 20 Gew.-% wenigstens eines weiteren Monomers, das ausgewählt ist unter C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten und Mischungen davon, insbesondere unter Stearylmethacrylat, mit C₁₈-C₂₂-Alkylgruppen terminierten Polyethylenglycol(meth)acrylaten, C₈-C₂₂-Carbonsäurevinylestern und Mischungen davon.

Anstelle von oder zusätzlich zu Vinylpyrrolidon kann auch Vinylcaprolactam eingesetzt werden. Anstelle von oder zusätzlich zu Vinylimidazol kann auch N-[3-(dimethylamino)propyllacrylamid und/oder N-[3-(dimethylamino)propyl]methacrylamid eingesetzt werden.

In einer speziellen Ausführung werden alle zuvor genannten anionischen Silicongruppen-haltigen Copolymere A), die wenigstens 5 Gew.-% mindestens einer Vinylimidazol-Verbindung einpolymerisiert enthalten, einer teilweisen oder vollständigen Quaternisierung unterzogen. Geeignete Quaternisierungsmittel sind die im Folgenden genannten.

Bevorzugte sind wasserfreie Quaternisierungsmittel. Ein besonders bevorzugtes Quaternisierungsmittel ist CH₃-Cl.

Die Herstellung der Copolymere A₁) erfolgt nach üblichen, dem Fachmann bekannten Verfahren, z. B. durch Lösungs-, Fällungs-, Suspensions- oder mulsionspolymerisation. Geeignet ist auch die W/W-Polymerisation in Wasser mit einem geeigneten Verdrängungsmittel, z. B. einem Salz, wie NaCl.

Bevorzugte Lösemittel zur Lösungspolymerisation sind wässrige Lösungsmittel, wie Wasser und Gemische aus Wasser mit wassermischbaren Lösungsmitteln, beispielsweise Alkoholen, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Polyethylenglykolen mit zahlenmittleren Molekulargewichten bis etwa 3000, Glycerin und Dioxan. Besonders bevorzugt ist die Polymerisation in Wasser oder einem Wasser/Alkohol-Gemisch, beispielsweise in einem Wasser/Ethanol-Gemisch. Die Polymerisationstemperaturen liegen bei der Lösungspolymerisation vorzugsweise in einem Bereich von etwa 30 bis 120 °C, besonders bevorzugt 40 bis 100 °C.

Die Herstellung der Silicongruppen-haltigen Copolymere A) erfolgt besonders bevorzugt durch Fällungspolymerisation.

Die Fällungspolymerisation erfolgt vorzugsweise in einem weitgehend wasserfreien, aprotischen Lösungsmittel oder Lösungsmittelgemisch, vorzugsweise in Ethylacetat und/oder n-Butylacetat. Unter einem weitgehend wasserfreien, aprotischen Lösungsmittel oder Lösungsmittelgemisch wird ein Lösungsmittel oder Lösungsmittelgemisch mit einem Wassergehalt von höchstens 5 Gew.-% verstanden.

Bevorzugt erfolgt die Fällungspolymerisation bei einer Temperatur im Bereich von 70 bis 140 °C, bevorzugt 75 bis 100 °C, insbesondere von 80 bis 95 °C. Die resultierenden Polymerteilchen fallen aus der Reaktionslösung aus und können durch übliche Verfahren, wie Filtration mittels Unterdruck, isoliert werden. Zur Fällungspolymerisation können oberflächenaktive, polymere Verbindungen, vorzugsweise auf Polysiloxanbasis, eingesetzt werden. Bei der Fällungspolymerisation werden in der Regel Polymere mit höheren Molekulargewichten als bei der Lösungspolymerisation erhalten.

Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Herstellung der Polymerisate können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-midinopropan)dihydrochlorid oder 2-2'-Azo-bis-(2-methyl-butyronitril). Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat, H₂O₂/Cu^{l}.

In einer speziellen Ausführung werden zur Herstellung der erfindungsgemäßen Copolymere wenigstens zwei Radikalinitiatoren eingesetzt, die eine im Wesentlichen unabhängige Initiierung in wenigstens zwei Phasen ermöglichen. Dabei können Copolymere mit besonders geringen Restmonomergehalten erzielt werden.

Bevorzugt werden zur Copolymerisation wenigstens zwei Initiatoren eingesetzt, deren Zerfallstemperaturen um wenigstens 10 °C voneinander verschieden sind. Im Rahmen der Erfindung ist die Zerfallstemperatur ist definiert als die Temperatur, bei der 50 % der Moleküle innerhalb von 2,5 Stunden in freie Radikale zerfallen. Vorzugsweise erfolgt die Copolymerisation bei dieser Vorgehensweise bis zum Abschluss der Fällung des Copolymers bei einer Temperatur größer oder gleich der niedrigeren Zerfallstemperatur und kleiner der höheren Zerfallstemperatur, und nach der Fällung erfolgt eine weitere Umsetzung bei einer Temperatur größer oder gleich der höheren Zerfallstemperatur.

Bevorzugt umfasst das erfindungsgemäße Verfahren eine erste Polymerisationsphase bei einer ersten Polymerisationstemperatur und eine zweite Polymerisationsphase bei einer zweiten Polymerisationstemperatur oberhalb der ersten Polymerisationstemperatur, wobei zur Polymerisation wenigstens zwei Initiatoren eingesetzt werden, deren Halbwertszeiten bei der ersten Polymerisationstemperatur sich so unterscheiden, dass wenigstens einer dieser Initiatoren während der ersten Polymerisationsphase in Radikale zerfällt und wenigstens einer dieser Initiatoren während der ersten Polymerisationsphase im Wesentlichen nicht in Radikale zerfällt und während der zweiten Polymerisationsphase in Radikale zerfällt. Vorzugsweise beginnt bei dieser Vorgehensweise die zweite Polymerisationsphase im Wesentlichen nach Fällung des Copolymers. Unter "im Wesentlichen" nach Fällung des Copolymers wird verstanden, dass das Copolymer vorzugsweise zu mindestens 80 Gew.-%, vorzugsweise mindestens 90 Gew.-%, insbesondere wenigstens 95 Gew.-%, bezogen auf das Gesamtgewicht des Copolymers, in gefällter Form vorliegt.

Die Halbwertszeit eines Initiators kann nach üblichen, dem Fachmann bekannten Verfahren bestimmt werden, wie z. B. in der Druckschrift "Initiators for high polymers", Akzo Nobel, Nr. 10737, beschrieben. Vorzugsweise liegt die Halbwertszeit des ersten Polymerisationsinitiators bei der ersten Polymerisationstemperatur und des zweiten Polymerisationsinitiators bei der zweiten Polymerisationstemperatur in einem Bereich von etwa 1 Minute bis 3 Stunden, besonders bevorzugt 5 Minuten bis 2,5 Stunden. Gewünschtenfalls können auch kürzere Halbwertszeiten z. B. von 1 Sekunde bis 1 Minute oder längere Halbwertszeiten als 3 Stunden zum Einsatz kommen, solange sichergestellt ist, dass der/die bei der höheren Temperatur zerfallende(n) Initiator(en) im Wesentlichen während der zweiten Polymerisationsphase in Radikale zerfällt.

Zusätzlich zu der ersten und zweiten Polymerisationsphase können weitere Polymerisationsphasen bei davon verschiedenen Polymerisationstemperaturen angewandt werden. So ist es z. B. möglich, eine erste Polymerisationsphase bei einer ersten Polymerisationstemperatur durchzuführen, die so gewählt ist, dass eine kontrollierte Polymerisation (d. h. z. B. unter Vermeidung eines unerwünschten Temperaturanstiegs durch die Reaktionswärme, einer zu hohen Reaktionsgeschwindigkeit, etc.) erfolgt. Anschließend kann sich z. B. eine Nachpolymerisation bei einer Temperatur anschließen, die oberhalb der ersten und unterhalb der zweiten Polymerisationstemperatur liegt und die so gewählt ist, dass der/die bei der höheren Temperatur zerfallende(n) Initiator(en) im Wesentlichen nicht in Radikale zerfallen. Nach Abschluss dieser Nachpolymerisation, zu der gewünschtenfalls nochmals der bei der niedrigeren Temperatur zerfallende Initiator und/oder ein anderer unter den Bedingungen der Nachpolymerisation zerfallender Initiator zugegeben werden kann, kann sich dann die zweite Polymerisationsphase anschließen.

Vorzugsweise enthält das eingesetzte Initiatorsystem wenigstens zwei Initiatoren, deren Zerfallstemperaturen sich um wenigstens 15 °C voneinander unterscheiden.

Der bei der niedrigeren Temperatur zerfallende Initiator weist vorzugsweise eine Zerfallstemperatur von 50 bis 100 °C auf.

Der bei der höheren Temperatur zerfallende Initiator weist vorzugsweise eine Zerfallstemperatur von 80 bis 150 °C auf.

Vorzugsweise wird der bei der höheren Temperatur zerfallende Initiator zu Beginn der Copolymerisation vorgelegt oder vor oder während der Fällung des Copolymers zugegeben.

Vorzugsweise wird der bei der höheren Temperatur zerfallende Initiator zu Beginn der Copolymerisation vorgelegt oder vor der Fällung des Copolymers zugegeben.

Bei einer bevorzugten Initiatorkombination handelt es sich bei dem bei der niedrigeren Temperatur zerfallenden Initiator um Trigonox® EHP (Bis(2-ethylhexyl)peroxydicarbonat, CAS-Nr. 16111-62-9) und ist der bei der höheren Temperatur zerfallende Initiator ausgewählt unter tert.-Butylperoxypivalat (z. B. Luperox 11 M75 der Fa. Atochem), tert.-Butylperoctoat, Lauroylperoxid (LPO, CAS-Nr. 105-74-8) oder 2,5-Dimethyl-2,5-bis(t-butylperoxy)hexan (Trigonox® 101).

Eine weitere bevorzugte Initiatorkombination enthält Trigonox® EHP und tert.-Butylperoctoat.

Eine weitere bevorzugte Initiatorkombination enthält Lauroylperoxid und tert.-Butylperoctoat oder 2,5-Dimethyl-2,5-bis(t-butylperoxy)hexan (Trigonox® 101).

Eine weitere bevorzugte Initiatorkombination enthält tert.-Butylperoxypivalat (Luperox 11 M75 und tert.-Butylperoctoat oder 2,5-Dimethyl-2,5-bis(t-butylperoxy)hexan (Trigonox® 101).

Eine weitere bevorzugte Initiatorkombination enthält tert.-Butylperoctoat und 2,5-Dimethyl-2,5-bis(t-butylperoxy)hexan (Trigonox® 101).

Zur Erzielung möglichst reiner Polymere können die Polymere z. B. einem Waschschritt mit einem geeigneten Lösungsmittel, z. B. dem auch zur Polymerisation eingesetzten Lösungsmittel unterzogen werden.

Die anionogenen Gruppen (Säuregruppen) der Copolymere A) können mit einer Base teilweise oder vollständig neutralisiert werden. Als Base für die Neutralisation der Polymere können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin. Bevorzugt sind Aminoalkohole, z. B. Trialkanolamine, wie Triethanolamin, Alkyldialkanolamine, wie Methyl- oder Ethyldiethanolamin und Dialkylalkanolamine, wie Dimethylethanolamin sowie 2-Amino-2-methyl-1-propanol. Besonders für den Einsatz in Haarbehandlungsmitteln haben sich zur Neutralisation der Säuregruppen enthaltenden Polymere NaOH, KOH, 2-Amino-2-methyl-1-propanol, 2-Amino-2-ethylpropan-1,3-diol, Diethylaminopropylamin und Triisopropanolamin bewährt. Die Neutralisation der Säuregruppen kann auch mit Hilfe von Mischungen mehrerer Basen vorgenommen werden, z. B. Mischungen aus Natronlauge und Triisopropanolamin. Die Neutralisation kann je nach Anwendungszweck partiell oder vollständig erfolgen.

Geladene kationische Gruppen lassen sich aus den vorliegenden kationogenen stickstoffhaltigen Gruppen entweder durch Protonierung, z. B. mit ein- oder mehrwertigen Carbonsäuren, wie Milchsäure oder Weinsäure, oder mit Mineralsäuren, wie Phosphorsäure, Schwefelsäure und Salzsäure, oder durch Quaternisierung, z. B. mit Alkylierungsmitteln, wie C₁-C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Sollen die erfindungsgemäßen Copolymere A) sowohl quaternisiert als auch neutralisiert werden, so erfolgt vorzugsweise zuerst die Quaternisierung und anschließend die Neutralisierung.

Die erfindungsgemäßen Silicongruppen-haltigen Copolymere A) eignen sich in vorteilhafter Weise zur Modifizierung der rheologischen Eigenschaften wässriger Wirk- oder Effektstoffzusammensetzungen. Dabei kann es sich ganz allgemein beispielsweise um kosmetische Zusammensetzungen, pharmazeutische Zusammensetzungen, Hygieneprodukte, Anstrichmittel, Zusammensetzungen für die Papierindustrie sowie die Textilindustrie handeln. In einer bevorzugten Ausführungsform enthalten die Zusammensetzungen wenigstens einen wasserlöslichen oder zumindest wasserdispergierbaren Wirk- oder Effektstoff. Selbstverständlich eignen sich die erfindungsgemäßen Copolymere A) auch zur Modifizierung der rheologischen Eigenschaften von Zusammensetzungen, die wenigstens einen wasserunlöslichen (hydrophoben) Wirk- oder Effektstoff enthalten.

"Modifizierung rheologischer Eigenschaften" wird im Rahmen der vorliegenden Erfindung weit verstanden. Die erfindungsgemäß verwendeten Copolymere A) eignen sich im Allgemeinen zur Verdickung der Konsistenz von wässrigen Zusammensetzungen in einem weiten Bereich. Je nach Grundkonsistenz der flüssigen Zusammensetzungen können in Abhängigkeit von der Einsatzmenge des Copolymers A) in der Regel Fließeigenschaften von dünnflüssig bis hin zu fest (im Sinne von "nicht mehr fließend") erzielt werden. Unter "Modifizierung rheologischer Eigenschaften" wird daher u. a. die Erhöhung der Viskosität von Flüssigkeiten, die Verbesserung der Thixotropie-Eigenschaften von Gelen, die Verfestigung von Gelen und Wachsen etc. verstanden. Die erfindungsgemäßen Mittel eignen sich vorzugsweise zur Formulierung von wässrigen kosmetischen und pharmazeutischen Produkten. Vorzugsweise sind dabei die Zusammensetzungen der Copolymere A) im Allgemeinen klar. Vorteilhafterweise können somit Formulierungen, insbesondere kosmetische Formulierungen, ohne Beeinträchtigung durch die Eigenfarbe der Zusammensetzungen angefärbt werden. Des Weiteren können die Zusammensetzungen in Form von opaken bis klaren Gelen formuliert werden.

Die erfindungsgemäßen Silicongruppen-haltigen Copolymere A) eignen sich speziell als Rheologiemodifizierungsmittel mit über den pH-Wert steuerbaren Eigenschaften. So eignen sich z. B. die zuvor genannten anionischen Silicongruppen-haltigen Copolymere A) als pH-schaltbare Verdicker für einen pH-Bereich größer oder gleich 6. Die zuvor genannten kationischen Silicongruppen-haltigen Copolymere A) eignen sich als ph-schaltbare Verdicker für einen pH-Bereich kleiner oder gleich 6,5. Quaternisierte Silicongruppen-haltigen Copolymere A), die im Wesentlichen keine protonierbaren Gruppen aufweisen, eignen sich unabhängig vom pH-Wert als Rheologiemodifizierungsmittel in einem pH-Bereich von etwa 2 bis 10.

Die erfindungsgemäßen Silicongruppen-haltigen Copolymere A) eignen sich speziell auch als Rheologiemodifizierungsmittel für salzhaltige Zusammensetzungen.

Vorteilhafterweise wirken die erfindungsgemäßen Silicongruppen-haltigen Copolymere A) auch als filmbildende und/oder konditionierend wirkende Rheologiemodifizierungsmittel. Sie eignen sich somit speziell für kosmetische und dermatologische Mittel, speziell in Haarfestigem als "festigende Verdicker" und in Haarpflegemitteln als "konditionierende Verdicker".

Die Silicongruppen-haltigen Copolymere A) eignen sich sowohl zur Herstellung homogenphasiger wässriger Zusammensetzungen als auch zur Formulierung von heterogenphasigen Zusammensetzungen, die zusätzlich wenigstens eine wasserunlösliche (hydrophobe) flüssige oder feste Verbindung umfassen. "Homogenphasige Zusammensetzungen" weisen unabhängig von der Anzahl ihrer Bestandteile nur eine einzige Phase auf. "Heterogenphasige Zusammensetzungen" sind disperse Systeme von zwei oder mehreren miteinander nicht mischbaren Komponenten. Dazu zählen fest/flüssig-, flüssig/flüssig- und fest/flüssig/flüssig-Zusammensetzungen, wie Dispersionen und Emulsionen, z. B. O/W- und W/O-Formulierungen, die wenigstens eine der im Folgenden näher beschriebenen Öl- bzw. Fettkomponenten und Wasser als nicht mischbare Phasen aufweisen. Prinzipiell können die Copolymere A) sowohl in der Wasserphase als auch in der Ölphase eingesetzt werden. Im Allgemeinen enthalten heterogenphasige flüssig/flüssig-Zusammensetzungen die Copolymere A) im Wesentlichen in der Wasserphase.

Die erfindungsgemäßen Copolymere A) eignen sich weiterhin als Solubilisator für im

Wesentlichen wasserunlösliche Verbindungen. Ein weiterer Gegenstand der Erfindung ist daher die Verwendung eines Silicongruppen-haltigen Copolymers A), wie zuvor definiert, als Solubilisator zur Herstellung wässriger Formulierungen von Wirk- und Effektstoffen, die in Wasser bei 25 °C und 1013 mbar eine Löslichkeit unterhalb von 10 g/l aufweisen.

Des Weiteren wurde gefunden, dass sich die erfindungsgemäßen Copolymere A) in vorteilhafter Weise als Schutzkolloid eignen. Ein weiterer Gegenstand der Erfindung ist daher die Verwendung eines Silicongruppen-haltigen Copolymers A), wie zuvor definiert, als Schutzkolloid bei der radikalischen wässrigen Emulsionspolymerisation.

Auf Grund der zuvor genannten Eigenschaften eignen sich die erfindungsgemäßen Copolymere A) ganz allgemein zur Herstellung von Wirk- oder Effektstoffzusammensetzungen, enthaltend
A) wenigstens ein Silicongruppen-haltiges Copolymer, wie zuvor definiert,
B) wenigstens einen Wirk- oder Effektstoff und
C) gegebenenfalls wenigstens einen weiteren, von A) und B) verschiedenen Wirk- oder Hilfsstoff.

Wirkstoffe für Kosmetika, Arzneimittel, Hygienemittel, Textilbehandlungsmittel etc., d. h. Substanzen, die im Allgemeinen bereits in geringer Konzentration eine Wirkung entfalten, z. B. eine kosmetische Wirkung auf Haut und/oder Haaren, eine pharmakologische Wirkung in einem Organismus, eine reinigende und/oder desinfizierende Wirkung, eine Modifizierung eines textilen Stoffes, z. B. eine knitterfreie Ausrüstung, sowie Effektstoffe, die Lebewesen oder unbelebten Substraten eine bestimmte Eigenschaft verleihen, beispielsweise Farbpigmente für Schminken oder Dispersionsfarben, werden häufig in Form wässriger Wirk- oder Effektstoffzusammensetzungen formuliert und angewendet.

Die Wirk- und Effektstoffzusammensetzungen enthalten die Polymerkomponente A) vorzugsweise in einem Anteil von etwa 0,001 bis 50 Gew.-%, besonders bevorzugt 0,01 bis 30 Gew.-%, insbesondere 0,1 bis 20 Gew.-%, bezogen auf das Gesamtgewicht des Mittels.

Die Komponenten B) und C) werden nach dem gewünschten Einsatzbereich der Zusammensetzung ausgewählt. Neben einsatzbereichtypischen Komponenten (z. B. bestimmten pharmazeutischen Wirkstoffen) sind sie ausgewählt unter Trägern, Exzipienten, Emulgatoren, Tensiden, Konservierungsmitteln, Duftstoffen, von Komponente A) verschiedenen Verdickern, Polymeren, Gelbildnern, Farbstoffen, Pigmenten, Lichtschutzmitteln, Konsistenzgebern, Antioxidantien, Entschäumem, Antistatika, Harzen, Lösemitteln, Lösungsvermittlern, Neutralisierungsmitteln, Stabilisatoren, Sterilantien, Treibmitteln, Trocknungsmitteln, Trübungsmitteln, etc.

Vorzugsweise weisen die Zusammensetzungen eine Trägerkomponente C) auf, die ausgewählt ist unter Wasser, hydrophilen Komponenten, hydrophoben Komponenten und Mischungen davon.

Geeignete hydrophile Träger C) sind z. B. ein-, zwei- oder mehrwertige Alkohole mit vorzugsweise 1 bis 8 Kohlenstoffatomen, wie Ethanol, n-Propanol, Isopropanol, Propylenglycol, Glycerin, Sorbit, etc.

### Geeignete hydrophobe Träger C) sind vorzugsweise ausgewählt unter

i) Ölen, Fetten, Wachsen,
ii) von iii) verschiedenen Estern von C₆-C₃₀-Monocarbonsäuren mit ein-, zwei- oder dreiwertigen Alkoholen,
iii) gesättigten acyclischen und cyclischen Kohlenwasserstoffen,
iv) Fettsäuren,
v) Fettalkoholen,
vi) Treibgasen,
und Mischungen davon.

Geeignete Siliconöle C) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten C) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten C) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Die erfindungsgemäßen Zusammensetzungen können als Wirkstoff, z. B. als kosmetischen und/oder pharmazeutischen Wirkstoff B) (wie auch gegebenenfalls als Hilfsstoff C)) wenigstens ein Polymer enthalten, das sich von den erfindungsgemäßen Silicongruppen-haltigen Copolymeren A) unterscheidet. Dazu zählen ganz allgemein anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset®-Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. C₄-C₃₀-Alkylester der (Meth)acrylsäure, C₄-C₃₀-Alkylvinylester, C₄-C₃₀-Alkylvinylether und Hyaluronsäure. Beispiele für anionische Polymere sind weiterhin Vinylacetat/- Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Weiterhin geeignet sind Vinylpyrrolidon/Ethylmethacrylat/Methacrylsäure-Copolymere wie sie von der Fa. Stepan unter den Bezeichnungen Stepanhold-Extra und -R1 vertrieben werden und die Carboset®-Marken der Fa. BF Goodrich..

Geeignete kationische Polymere sind z. B. kationische Polymere mit der Bezeichnung Polyquaternium nach INCl, z. B. Copolymere aus Vinylpyrrolidon/N-Vinylimidazoliumsalzen (Luviquat® FC, Luviquat® HM, Luviquat® MS, Luviset Clear®, Luviquat Supreme®, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Ganz besonders geeignete Polymere sind neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF), oder Polyvinylpyrrolidon und deren Copolymere, insbesondere mit Vinylestern, wie Vinylacetat, z. B. Luviskol® VA 37 (BASF); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Wie bereits ausgeführt, sind die Copolymere A) in vorteilhafter Weise geeignet, wasserunlösliche (bzw. nur gering wasserlösliche) Wirk- und Effektstoffe B) in wässriger Phase zu stabilisieren und ermöglichen daher die Herstellung wässriger Formulierungen derartiger Wirk- und Effektstoffe. Sie eignen sich auch zur Herstellung fester Formulierungen dieser Wirk- und Effektstoffe, die sich in eine wässrige Formulierung, z. B. als Handels-, Darreichungs- oder Wirkform, überführen lassen. Dies kann auch erst nach Applikation der festen Zusammensetzung erfolgen (z. B. im Verdauungstrakt eines Organismus, etc.).

Die mit den erfindungsgemäß eingesetzten Polymeren A) erzielte "Löslichkeitsverbesserung" wird im Rahmen der vorliegenden Erfindung daher breit verstanden. Dazu zählt zum einen die Stabilisierung heterogener Systeme, bei denen der Wirkstoff als emulgierte bzw. dispergierte Phase (disperse Phase) in einem wässrigen Medium als kontinuierliche Phase vorliegt. Dazu zählt weiterhin die Stabilisierung von Übergangsstufen zu homogenen Lösungen, wie kolloidale Lösungen, etc., bis hin zu molekulardispersen Lösungen. Dazu zählt auch eine Löslichkeitsverbesserung im Sinne einer Solubilisierung, bei der die schlecht wasserlöslichen oder wasserunlöslichen Stoffe in klare, höchstens opaleszierende wässrige Lösungen überführt werden. Dazu zählt schließlich auch die Befähigung zur Bildung so genannter "fester Lösungen".

Eine geringe (schlechte) Löslichkeit bedeutet im Rahmen dieser Erfindung eine Löslichkeit des Wirk- bzw. Effektstoffs in Wasser von unterhalb 10 g/l, insbesondere unterhalb 1 g/l und speziell unterhalb 0,1 g/l bei 25 °C und 1013 mbar.

Die unter Verwendung der Copolymere A) hergestellten wässrigen Wirkstoffzusammensetzungen von in Wasser unlöslichen Wirk- bzw. Effektstoffen umfassen neben einem wässrigen Medium als kontinuierlicher Phase wenigstens einen in der kontinuierlichen Phase solubilisierten oder dispergierten Wirkstoff und/oder Effektstoff B), der in Wasser bei 25 °C/1013 mbar eine Löslichkeit unterhalb 10 g/l, insbesondere unterhalb 1 g/l und speziell unterhalb 0,1 g/l aufweisen, sowie wenigstens ein Silicongruppen-haltiges Copolymer A).

Der Wirkstoff liegt in der kontinuierlichen wässrigen Phase in äußerst feinteiliger Form vor. Dies kann beispielsweise darauf zurückzuführen sein, dass der Wirkstoff in der wässrigen Phase mit den Polymeren A) Aggregate bildet. Diese Aggregate weisen in der Regel mittlere Teilchengrößen unterhalb 1 µm, häufig unterhalb 500 nm, insbesondere unterhalb 400 nm, speziell unterhalb 300 nm auf. Je nach Art des Polymers und des Wirkstoffs bzw. Effektstoffs sowie abhängig von den Konzentrationsverhältnissen können die Aggregate auch so klein werden, dass sie nicht mehr in Form nachweisbarer, diskreter Partikel sondern in gelöster Form vorliegen. (Teilchengröße < 10 nm).

Die hier angegebenen Teilchengrößen sind gewichtsmittlere Teilchengrößen, wie sie durch dynamische Lichtstreuung ermittelt werden können. Verfahren hierzu sind dem Fachmann geläufig, beispielsweise aus H. Wiese in D. Distler, Wässrige Polymerdispersionen, Wiley-VCH 1999, Kapitel 4.2.1, S. 40ff und dort zitierte Literatur sowie H. Auweter, D. Horn, J. Colloid Interf. Sci. 105 (1985) 399, D. Lilge, D. Horn, Colloid Polym. Sci. 269 (1991) 704 oder H. Wiese, D. Horn, J. Chem. Phys. 94 (1991) 6429.

Vorteilhaft können die Copolymere A) als Solubilisatoren für in Wasser schwerlösliche oder unlösliche UV-Absorber verwendet werden.

Der Begriff UV-Absorber umfasst im Rahmen der vorliegenden Erfindung UV-A-, UV-Bund/oder Breitbandfilter.

Vorteilhafte Breitbandfilter, UV-A- oder UV-B-Filtersubstanzen sind beispielsweise Vertreter der folgenden Verbindungsklassen:

Bis-Resorcinyltriazinderivate mit der folgenden Struktur: 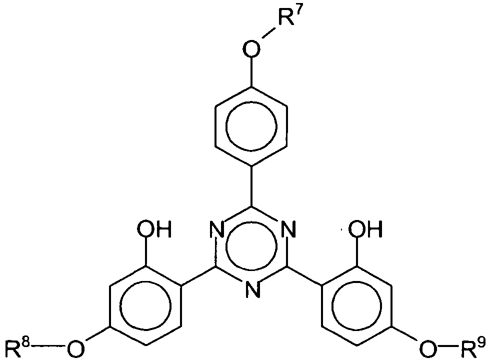 wobei R⁷, R⁸ und R⁹ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb^{®} S bei der CIBA-Chemikalien GmbH erhältlich ist.

Auch andere UV-Filtersubstanzen, welche das Strukturmotiv 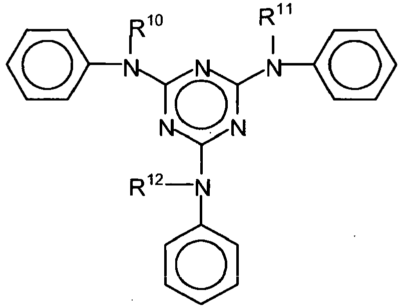 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 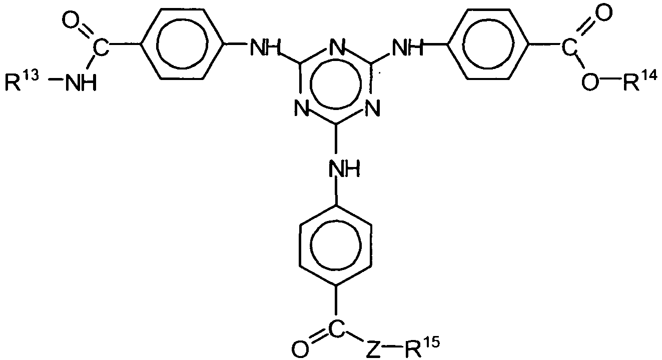 wiedergegeben wird, wobei
- R¹³: einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, darstellt,
- Z: ein Sauerstoffatom oder eine NH-Gruppe darstellt,
- R¹⁴: einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, oder ein Wasserstoffatom, ein Alkalimetallatom, eine Ammoniumgruppe oder eine Gruppe der Formel 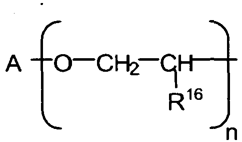
bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄-Alkylgruppen,
- R¹⁶: ein Wasserstoffatom oder eine Methylgruppe darstellt,
- n: eine Zahl von 1 bis 10 darstellt,
- R¹⁵: einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, darstellt, wenn X die NH-Gruppe darstellt, und einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, oder ein Wasserstoffatom, ein Alkalimetallatom, eine Ammoniumgruppe oder eine Gruppe der Formel 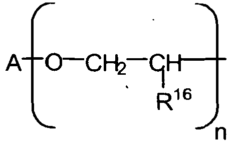
bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄-Alkylgruppen,
- R¹⁶: ein Wasserstoffatom oder eine Methylgruppe darstellt,
- n: eine Zahl von 1 bis 10 darstellt, wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 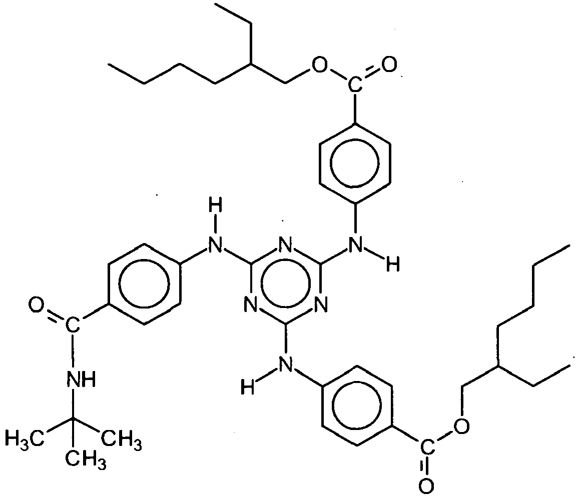 wiedergegeben wird, welches im Folgenden auch als Dioctylbutylamidotriazon (INCI: Diethylhexylbutamidotriazone) bezeichnet wird und unter der Handelsbezeichnung UVASORB^{®} HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL^{®} T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 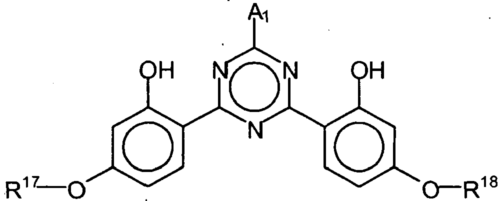 wiedergegeben wird, wobei R¹⁷ und R¹⁸ u. a. C₃-C₁₈-Alkyl oder C₂-C₁₈-Alkenyl und A₁ einen aromatischen Rest repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxypropyloxy)-2-hydroxy]-phenyl}-6-(4-methoxyphenyl)-1,3,5-triazin, das 2,4-Bis-{[4-(2-ethylhexyloxy)-2-hydroxy]-phenyl}-6-[4-(2-methoxyethylcarboxyl)-phenylamino]-1,3,5-triazin, das 2,4-Bis-{[4-(3-(2-propyloxy)-2-hydroxypropyloxy)-2-hydroxy]-phenyl}-6-[4-(2-ethylcarboxyl)-phenylamino]-1,3,5-triazin, das 2,4-Bis-{[4-(2-ethyl-hexyloxy)-2-hydroxy]-phenyl}-6-(1-methylpyrrol-2-yl)-1,3,5-triazin, das 2,4-Bis-{[4-tris(trimethylsiloxysilylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-heptamethylsiloxy-2"-methylpropyloxy)-2-hydroxy]-phenyl}-6-(4-methoxyphenyl)-1,3,5-triazin.

Vorteilhafte öllösliche UV-B- und/oder Breitband-Filtersubstanzen sind z. B.:
3-Benzylidencampher-Derivate, vorzugsweise 3-(4-Methylbenzyliden)campher, 3-Benzylidencampher;
4-Aminobenzoesäure-Derivate, vorzugsweise
4-(Dimethylamino)-benzoesäure(2-ethylhexyl)ester,
4-(Dimethylamino)benzoesäureamylester, Derivate des Benzophenons, vorzugsweise 2-Hydroxy-4-methoxybenzophenon (unter der Handelsbezeichnung Uvinul^{®} M40 von der Fa. BASF erhältlich) 2-Hydroxy-4-methoxy-4'-methylbenzophenon,
2,2'-Dihydroxy-4-methoxybenzophenon, 2,2',4,4'-Tetrahydroxybenzophenon (unter der Handelsbezeichnung Uvinul^{®} D 50 von der Fa. BASF erhältlich).

Besonders vorteilhafte, bei Raumtemperatur flüssige UV-Filtersubstanzen im Sinne der vorliegenden Erfindung sind Homomethylsalicylat,
2-Ethylhexyl-2-cyano-3,3-diphenylacrylat, 2-Ethylhexyl-2-hydroxybenzoat und Ester der Zimtsäure, vorzugsweise 4-Methoxyzimtsäure(2-ethylhexyl)ester und 4-Methoxyzimtsäureisopentylester.

Homomethylsalicylat (INCI: Homosalate) zeichnet sich durch die folgende Struktur aus: 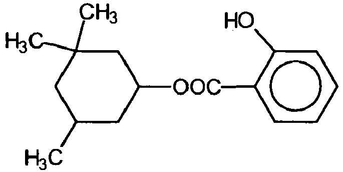 2-Ethylhexyl-2-cyano-3,3-diphenylacrylat (INCI: Octocrylene) ist von BASF unter der Bezeichnung Uvinul^{®} N 539T erhältlich und zeichnet sich durch folgende Struktur aus: 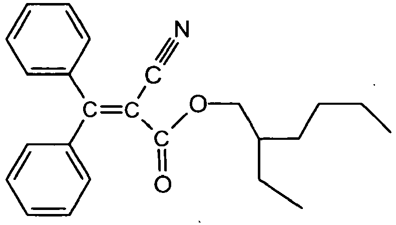 2-Ethylhexyl-2-hydroxybenzoat (2-Ethylhexylsalicylat, Octylsalicylat, INCI: Ethylhexyl Salicylate) ist beispielsweise bei Haarmann & Reimer unter der Handelsbezeichnung Neo Heliopan^{®} OS erhältlich und zeichnet sich durch die folgende Struktur aus: 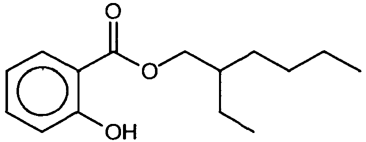 4-Methoxyzimtsäure(2-ethylhexyl)ester (2-Ethylhexyl-4-methoxycinnamat, INCI: Ethylhexyl Methoxycinnamate) ist beispielsweise bei Fa. BASF unter der Handelsbezeichnung Uvinul^{®} MC 80 erhältlich und zeichnet sich durch die folgende Struktur aus: 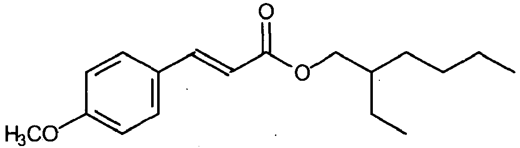 4-Methoxyzimtsäureisopentylester (Isopentyl-4-methoxycinnamat, INCI: Isoamyl p-Methoxycinnamate) ist beispielsweise bei Haarmann & Reimer unter der Handelsbezeichnung Neo Heliopan^{®} E 1000 erhältlich und zeichnet sich durch die folgende Struktur aus:

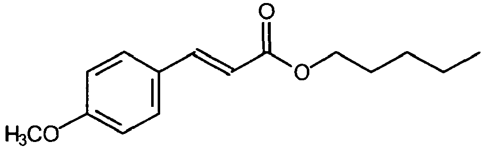

Ein vorteilhaftes Dibenzoylmethanderivat im Sinne der vorliegenden Erfindung ist insbesondere das 4-(tert.-Butyl)-4'-methoxydibenzoylmethan (CAS-Nr. 70356-09-1), welches von BASF unter der Marke Uvinul^{®} BMBM und von Merck unter der Handelsbezeichnung Eusolex^{®} 9020 verkauft wird. Es zeichnet sich durch folgende Struktur aus:

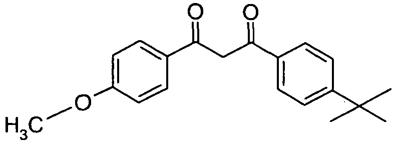

Ein weiteres vorteilhaftes Dibenzoylmethanderivat ist das 4-Isopropyl-Dibenzoylmethan (CAS-Nr. 63250-25-9), welches von Merck unter dem Namen Eusolex^{®} 8020 verkauft wird. Das Eusolex 8020 zeichnet sich durch folgende Struktur aus:

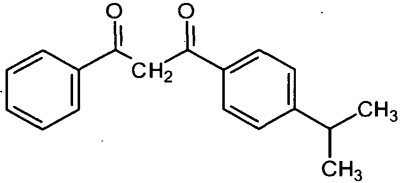

Benzotriazole zeichnen sich durch die folgende Strukturformel aus: 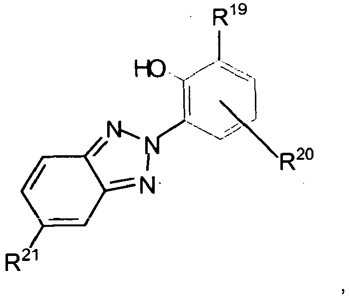 worin
R¹⁹ und R²⁰ unabhängig voneinander lineare oder verzweigte, gesättigte oder ungesättigte, substituierte (z. B. mit einem Phenylrest substituierte) oder unsubstituierte Alkylreste mit 1 bis 18 Kohlenstoffatomen stehen.

Vorteilhaftes Benzotriazol im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]-propyl]-phenol (CAS-Nr.: 155633-54-8) mit der INCI-Bezeichnung Drometrizole Trisiloxane, welches von Fa. Chimex unter der Marke Mexoryl^{®} XL verkauft wird und durch die folgende chemische Strukturformel 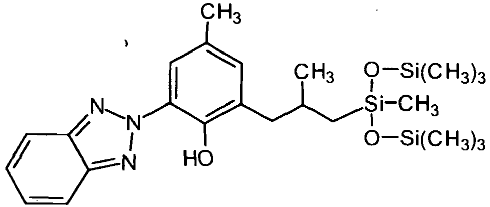 gekennzeichnet ist.

Weitere vorteilhafte Benzotriazole im Sinne der vorliegenden Erfindung sind [2,4'-Dihydroxy-3-(2H-benzotriazol-2-yl)-5-(1,1,3,3-tetramethylbutyl)-2'-n-octoxy-5'-benzoyl]diphenylmethan, 2,2'-Methylen-bis-[6-(2H-benzotriazol-2-yl)-4-(methyl)phenol], 2,2'-Methylen-bis-[6-(2H-benzotriazol-2-yl)-4-(1,1,3,3-tetramethylbutyl)phenol], 2-(2'-Hydroxy-5'-octylphenyl)-benzotriazol, 2-(2'-Hydroxy-3',5'-di-t-amylphenyl)benzotriazol und 2-(2'-Hydroxy-5'-methylphenyl)benzotriazol.

Ein weiterer, im Sinne der vorliegenden Erfindung vorteilhafter UV-Filter ist die in EP-A-0 916 335 beschriebene Diphenylbutadienverbindung der folgenden Formel.

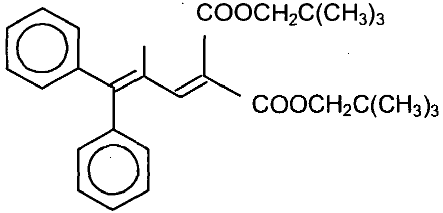

Ein weiterer im Sinne der vorliegenden Erfindung vorteilhafter UV-A-Filter ist der in EP-A-0 895 776 beschriebene 2-(4-Ethoxy-anilinomethylen)-propandicarbonsäurediethylester der folgenden Formel.

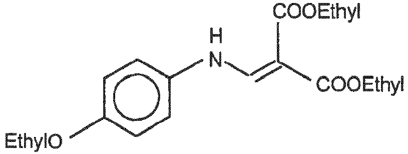

Vorteilhaft im Sinne der vorliegenden Erfindung ist ebenfalls ein aminosubstituiertes Hydroxybenzophenon der folgenden Formel: 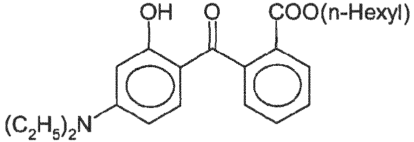 welches von der BASF Aktiengesellschaft als UV-A Filter unter der Warenbezeichnung UVINUL^{®} A Plus vertrieben wird.

Die erfindungsgemäß zu verwendenden Copolymerisate A) eignen sich ebenso für die Verwendung zur Modifizierung der rheologischen Eigenschaften sowie als Solubilisator in pharmazeutischen Zubereitungen jeder Art.

Ein weiterer Gegenstand der Erfindung ist daher ein pharmazeutisches Mittel, enthaltend
A) wenigstens ein Silicongruppen-haltiges Copolymer, wie zuvor definiert,
B) wenigstens einen pharmazeutisch akzeptablen Wirkstoff und
C) gegebenenfalls wenigstens einen weiteren, von B) verschiedenen pharmazeutisch akzeptablen Wirk- oder Hilfsstoff.

In einer speziellen Ausführung enthalten die pharmazeutischen Mittel wenigstens einen pharmazeutisch akzeptablen Wirkstoff B), der in Wasser bei 25 °C und 1013 mbar eine Löslichkeit unterhalb 10 g/l aufweist. Dazu dienen die Copolymere A) als Solubilisator für den/die schwerlöslichen Wirkstoff(e). Die Formullerungsgrundlage der erfindungsgemäßen pharmazeutischen Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hitfsstoffe, insbesondere die in einschlägigen Arzneibüchern (z. B. DAB, Ph. Eur., BP, NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder ÖI-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung von erfindungsgemäßen pharmazeutischen Mitteln können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Insbesondere handelt es sich dabei um wässrige Lösungen bzw. Solubilisate zur oralen oder zur parenteralen Applikation. Des Weiteren eignen sich die erfindungsgemäß zu verwendenden Copolymere auch zum Einsatz in oralen Darreichungsformen wie Tabletten, Kapseln, Pulvern, Lösungen. Hier können sie den schwerlöslichen Arzneistoff mit einer erhöhten Bioverfügbarkeit zur Verfügung stellen. Bei der parenteralen Applikation können neben Solubilisaten auch Emulsionen, beispielsweise Fettemulsionen eingesetzt werden. Auch für diesen Zweck eignen sich die erfindungsgemäßen Copolymere A) um einen schwerlöslichen Arzneistoff zu verarbeiten.

Pharmazeutische Formulierungen der oben genannten Art können durch Verarbeiten der erfindungsgemäß zu verwendenden Copolymere A) mit pharmazeutischen Wirkstoffen nach herkömmlichen Methoden und unter Einsatz bekannter und neuer Wirkstoffe erhalten werden.

Die erfindungsgemäße Anwendung kann zusätzlich pharmazeutische Hilfsstoffe und/oder Verdünnungsmittel enthalten. Als Hilfsstoffe werden Cosolventien, Stabilisatoren, Konservierungsmittel besonders aufgeführt.

Die verwendeten pharmazeutischen Wirkstoffe sind in Wasser lösliche oder unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Besonders bevorzugt sind von den oben genannten pharmazeutischen Mitteln solche, bei denen es sich um parenteral applizierbare Formulierungen handelt.

Der Gehalt an Copolymer A) in den pharmazeutischen Mitteln liegt, abhängig vom Wirkstoff, im Bereich von 0,01 bis 50 Gew.-%, bevorzugt 0,1 bis 40 Gew.-%, besonders bevorzugt 1 bis 30 Gew.-%, bezogen auf das Gesamtgewicht des Mittels.

Zur Herstellung der erfindungsgemäßen pharmazeutischen Mittel eignen sich prinzipiell alle pharmazeutischen Wirkstoffe und Pro-Drugs. Hierzu zählen Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel. Beispiele für geeignete pharmazeutische Wirkstoffe sind die insbesondere die in den Absätzen 0105 bis 0131 der US 2003/0157170 genannten Wirkstoffe.

Ein weiterer Aspekt der vorliegenden Erfindung betrifft die Verwendung der Silicongruppen-haltigen Copolymere A) als Solubilisatoren in molekulardispersen Systemen. Feststoffdispersionen, also homogene feinstdisperse Phasen von zwei oder mehreren Feststoffen sowie ihr Sonderfall der so genannten "festen Lösungen" (molekulardisperse Systeme), sowie ihr Einsatz in der pharmazeutischen Technologie sind allgemein bekannt (vgl. Chiou und Riegelmann, J. Pharm. Sci., 1971, 60, 1281 - 1300). Daneben betrifft die vorliegende Erfindung auch feste Lösungen, die mindestens ein erfindungsgemäß zu verwendendes Copolymer A) enthalten.

Die Herstellung von festen Lösungen kann mit Hilfe von Schmelzeverfahren oder nach dem Lösungsverfahren erfolgen.

Als polymerer Hilfsstoff, d. h. Solubilisator für die Herstellung solcher Feststoffdispersionen bzw. fester Lösungen eignen sich die erfindungsgemäßen Copolymere.

Nach dem Schmelzeverfahren können beispielsweise ein Wirkstoff B) und das Copolymer A) im gewünschten Verhältnis, z. B. zu gleichen Teilen, abgewogen und gemischt werden. Zur Mischung eignet sich beispielsweise ein Freifallmischer. Die Mischung kann anschließend, z. B. in einem Zweischneckenextruder, extrudiert werden. Der Durchmesser des so erhaltenen, abgekühlten Produktstrangs, bestehend aus einer festen Lösung des gewählten Wirkstoffes in dem gewählten erfindungsgemäß zu verwendenden Copolymeren, ist abhängig vom Durchmesser der Perforation der Lochscheiben des Extruders. Durch das Abschneiden der gekühlten Produktstränge mit Hilfe eines rotierenden Messers können zylindrische Partikel gewonnen werden, deren Höhe abhängig ist vom Abstand zwischen Lochscheibe und Messer. Der mittlere Durchmesser der zylindrischen Partikel beträgt in der Regel etwa 1000 bis etwa 3000 µm, die Höhe in der Regel etwa 2000 bis etwa 5000 µm. Größere Extrudate können in einem nachgeschalteten Schritt zerkleinert werden.

Alternativ kann man eine feste Lösung auch im Lösungsverfahren herstellen. Hierzu löst man üblicherweise den Wirkstoff B) und das Copolymer A) in einem geeigneten Lösungsmittel. Anschließend wird die Lösung üblicherweise in eine geeignete Form gegossen, und das Lösungsmittel, beispielsweise durch Trocknung, entfernt. Die Trocknungsbedingungen wählt man vorteilhaft je nach den Eigenschaften von Wirkstoff (z. B Thermolabilität) und Lösungsmittel (z. B. Siedepunkt).

Unter Beachtung des Materialverhaltens kann der entstandene Formling bzw. das Extrudat beispielsweise mit einer geeigneten Mühle (z. B. Stiftmühle) zerkleinert werden. Die feste Lösung zerkleinert man vorteilhafterweise bis zu einer mittleren Teilchengröße von weniger als etwa 2000 µm, bevorzugt weniger als etwa 1000 µm und besonders bevorzugt weniger als etwa 500 µm.

Mit geeigneten Hilfsstoffen kann nun das entstandene Schüttgut zu einer Tablettiermischung oder zu einem Kapselfüllgut verarbeitet werden. Die Tablettierung führt man vorteilhaft so durch, dass man Tabletten mit Härte von größer etwa 35 N, bevorzugt größer etwa 60 N, besonders bevorzugt von etwa 80 bis etwa 100 N erhält.

Die so erhältlichen Formulierungen können wie herkömmliche Formulierungen erforderlichenfalls mit geeigneten Überzugsmaterialien zur Erzielung von Magensaftresistenz, Retardierung, Geschmacksmaskierung usw. überzogen werden.

Neben der Anwendung in der Pharmazie eignen sich die erfindungsgemäß zu verwendenden Copolymere A) auch im Lebensmittelbereich zur Modifizierung der rheologischen Eigenschaften und/oder als Solubilisatoren für schwer wasserlösliche oder wasserunlösliche Nähr-, Hilfs- oder Zusatzstoffe, wie z. B. fettlösliche Vitamine oder Carotinoide. Als Beispiele seien klare, mit Carotinoiden gefärbte Getränke genannt. Gegenstand der Erfindung sind daher auch lebensmitteltechnische Zubereitungen, die mindestens eines der erfindungsgemäß zu verwendenden Copolymere A) enthalten. Zu den Lebensmittelzubereitungen sind im Rahmen der vorliegenden Erfindung auch Nahrungsergänzungsmittel wie z. B. Lebensmittelfarbstoffe enthaltende Zubereitungen und diätetische Lebensmittel zu verstehen. Darüber hinaus eigenen sich die genannten Copolymere A) auch zur Modifizierung der rheologischen Eigenschaften und/oder als Solubilisatoren für Futtermittelzusätze für die Tierernährung.

Außerdem eignen sich die Silicongruppen-haltigen Copolymere A) zur Herstellung wässriger Zubereitungen von Nahrungsergänzungsmitteln wie wasserunlöslichen Vitaminen und Provitaminen wie Vitamin A, Vitamin A-Acetat, Vitamin D, Vitamin E, Tocopherol-Derivate wie Tocopherolacetat und Vitamin K.

Beispiele für Effektstoffe, die als erfindungsgemäße wässrige Wirkstoffzusammensetzung formuliert werden können, sind Farbstoffe: z. B. die in DE-A 102 45 209 beschriebenen Farbstoffe sowie die gemäß Colour-Index als Disperse-Farbstoffe und als Solvent-Farbstoffe bezeichneten Verbindungen, die auch als Dispersionsfarbstoffe bezeichnet werden. Eine Zusammenstellung geeigneter Dispersionsfarbstoffe findet sich beispielsweise in Ullmanns Enzyklopädie der technischen Chemie, 4. Auflage, Bd. 10, S. 155-165 (siehe auch Bd. 7, S. 585ff - Anthrachinonfarbstoffe; Bd. 8, S. 244ff - Azofarbstoffe; Bd. 9, S. 313ff - Chinophthalonfarbstoffe). Auf diese Literaturstelle und die darin genannten Verbindungen wird hiermit ausdrücklich Bezug genommen. Erfindungsgemäß geeignete Dispersionsfarbstoffe und Solvent-Farbstoffe umfassen verschiedenste Farbstoffklassen mit unterschiedlichen Chromophoren, beispielsweise Anthrachinonfarbstoffe, Monoazo- und Disazofarbstoffe, Chinophthalone, Methin- und Azamethinfarbstoffe, Naphthalimidfarbstoffe, Naphthochinonfarbstoffe und Nitrofarbstoffe. Beispiele für erfindungsgemäß geeignete Dispersionsfarbstoffe sind die Dispersionsfarbstoffe der folgenden Colour-Index Liste: C. I. Disperse Yellow 1 - 228, C. I. Disperse Orange 1 - 148, C. I. Disperse Red 1 - 349, C. I. Disperse Violet 1 - 97, C. I. Disperse Blue 1 - 349, C. I. Disperse Green 1 - 9, C. I. Disperse Brown 1 - 21, C. I. Disperse Black 1 - 36. Beispiele für erfindungsgemäß geeignete Solvent-Farbstoffe sind die Verbindungen der folgenden Colour-Index Liste: C. I. Solvent Yellow 2 - 191, C. I. Solvent Orange 1 - 113, C. I. Solvent Red 1 - 248, C. I. Solvent Violet 2 - 61, C. I. Solvent Blue 2 - 143, C. I. Solvent Green 1 - 35, C. I. Solvent Brown 1 - 63, C. I. Solvent Black 3 - 50. Erfindungsgemäß geeignete Farbstoffe sind weiterhin Derivate des Naphthalins, des Anthracens, des Perylens, des Terylens, des Quarterylens, sowie Diketopyrrolopyrrolfarbstoffe, Perinonfarbstoffe, Cumarinfarbstoffe, Isoindolin- und Isoindolinonfarbstoffe, Porphyrinfarbstoffe, Phthalocyanin- und Naphthalocyaninfarbstoffe.

Neben den vorgenannten Bestandteilen können die erfindungsgemäßen Wirk- und Effektstoffzusammensetzungen auch konventionelle oberflächenaktive Substanzen und sonstige Additive enthalten. Zu den oberflächenaktiven Substanzen zählen Tenside, Dispergierhilfsmittel und Netzmittel. Zu den sonstigen Additiven zählen insbesondere Verdickungsmittel, Entschäumer, Konservierungsmittel, Frostschutzmittel, Stabilisierungsmittel, etc.

Prinzipiell brauchbar sind anionische, kationische, nichtionische und amphotere Tenside, wobei Polymertenside sowie Tenside mit Heteroatomen in der hydrophoben Gruppe eingeschlossen sind.

Zu den anionischen Tensiden gehören beispielsweise Carboxylate, insbesondere Alkali-, Erdalkali- und Ammoniumsalze von Fettsäuren, z. B. Kaliumstearat, die üblicherweise auch als Seifen bezeichnet werden; Acylglutamate; Sarkosinate, z. B. Natriumlauroylsarkosinat; Taurate; Methylcellulosen; Alkylphosphate, insbesondere Mono- und Diphosphorsäurealkylester; Sulfate, insbesondere Alkylsulfate und Alkylethersulfate; Sulfonate, weitere Alkyl- und Alkylarylsulfonate, insbesondere Alkali-, Erdalkali- und Ammoniumsalze von Arylsulfonsäuren sowie alkylsubstituierten Arylsulfonsäuren, Alkylbenzolsulfonsäuren, wie beispielsweise Lignin- und Phenolsulfonsäure, Naphthalin- und Dibutylnaphthalinsulfonsäuren, oder Dodecylbenzolsulfonate, Alkylnaphthalinsulfonate, Alkylmethylestersulfonate, Kondensationsprodukte von sulfoniertem Naphthalin und Derivaten davon mit Formaldehyd, Kondensationsprodukte von Naphthalinsulfonsäuren, Phenol- und/oder Phenolsulfonsäuren mit Formaldehyd oder mit Formaldehyd und Harnstoff, Mono- oder Dialkylbernsteinsäureestersulfonate; sowie Eiweißhydrolysate und Lignin-Sulfitablaugen. Die zuvor genannten Sulfonsäuren werden vorteilhafterweise in Form ihrer neutralen oder gegebenenfalls basischen Salze verwendet.

Zu den kationischen Tensiden gehören beispielsweise quaternierte Ammoniumverbindungen, insbesondere Alkyltrimethylammonium- und Dialkyldimethylammonium-Halogenide und -alkylsulfate sowie Pyridin- und Imidazolin-Derivate, insbesondere Alkylpyridinium-Halogenide.

Zu den nichtionischen Tensiden gehören beispielsweise:
- Fettalkoholpolyoxyethylenester, beispielsweise Laurylalkoholpolyoxyethylenetheracetat,
- Alkyl-Polyoxyethylen- und -polyoxypropylenether, z. B. von iso-Tridecylalkohol und Fettalkohol-Polyoxyethylenether,
- Alkylarylalkohol-Polyoxyethylenether, z. B. Octylphenol-Polyoxyethylenether,
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Fettalkoholalkoxylate und Oxoalkoholalkoxylate, insbesondere vom Typ RO-(R₁₈O)ᵣ(R₁₉O)ₛR₂₀ mit R₁₈ und R₁₉ unabhängig voneinander = C₂H₄, C₃H₆, C₄H₈ und R₂₀ = H, oder C₁-C₁₂-Alkyl, R = C₃-C₃₀-Alkyl oder C₆-C₃₀-Alkenyl, r und s unabhängig voneinander 0 bis 50, wobei nicht beide für 0 stehen können, wie iso-Tridecylalkohol und Oleylalkoholpolyoxyethylenether,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Zuckertenside, Sorbitester, wie beispielsweise Sorbitanfettsäureester (Sorbitanmonooleat, Sorbitantristearat), Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Zu den amphoteren Tensiden gehören beispielsweise Sulfobetaine, Carboxybetaine und Alkyldimethylaminoxide, z. B. Tetradecyldimethylaminoxid.

Weitere Tenside, die hier beispielhaft genannt werden sollen, sind Perfluortenside, Silicontenside, Phospholipide, wie beispielsweise Lecithin oder chemisch modifizierte Lecithine, Aminosäuretenside, z. B. N-Lauroylglutamat.

Sofern nicht spezifiziert, handelt es sich bei den Alkylketten der oben aufgeführten Tenside um lineare oder verzweigte Reste mit üblicherweise 8 bis 20 Kohlenstoffatomen.

In einer Ausführungsform enthalten die erfindungsgemäßen wässrigen Wirkstoffzusammensetzungen nicht mehr als 10 Gew.-%, vorzugsweise nicht mehr als 5 Gew.-% und insbesondere nicht mehr als 3 Gew.-%, z. B. 0,01 bis 5 Gew.-% oder 0,1 bis 3 Gew.-% an konventionellen oberflächenaktiven Substanzen, jeweils bezogen auf die Gesamtmenge an Wirkstoff und Polymerzusammensetzung. Die konventionellen oberflächenaktiven Substanzen machen dann vorzugsweise nicht mehr als 5 Gew.-% und insbesondere nicht mehr als 3 Gew.-%, z. B. 0,01 bis 5 Gew.-% oder 0,1 bis 3 Gew.-%, bezogen auf das Gesamtgewicht der Zusammensetzung, aus.

Je nach Anwendung kann es jedoch von Vorteil sein, wenn die erfindungsgemäßen Wirkstoffzusammensetzungen mit oberflächenaktiven Substanzen formuliert werden. Dann liegt der Anteil an konventioneller oberflächenaktiver Substanz häufig im Bereich von 0,5 bis 30 Gew.-%, insbesondere im Bereich von 1 bis 20 Gew.-%, bezogen auf die Gesamtmenge an Wirkstoff und Polymerzusammensetzung, bzw. im Bereich von 0,2 bis 20 Gew.-% und insbesondere im Bereich von 0,5 bis 15 Gew.-%, bezogen auf das Gesamtgewicht der formulierten Zusammensetzung.

Auch wenn ein Vorteil der erfindungsgemäßen Zusammensetzungen ihr geringer Gehalt an flüchtigen organischen Substanzen ist, kann es für einige Anwendungen erwünscht sein, die erfindungsgemäßen Zusammensetzungen mit organischen Lösungsmitteln, Ölen und Fetten, vorzugsweise solchen Lösungsmitteln oder Ölen und Fetten, die umweltverträglich oder biokompatibel sind, einzusetzen, z. B. die vorgenannten mit Wasser mischbaren Lösungsmittel oder Lösungsmittel, Ölen oder Fetten die mit Wasser nicht oder nur sehr begrenzt mischbar sind, z. B.:
- Paraffinöle, aromatische Kohlenwasserstoffe und aromatische Kohlenwasserstoffgemische, z. B. Xylole, Solvesso 100, 150 oder 200, und dergleichen,
- Phenole und Alkylphenole, z. B. Phenol, Hydrochinon, Nonylphenol, etc.
- Ketone mit mehr als 4 C-Atomen wie Cyclohexanon, Isophoron, Isopheron, Acetophenon, Acetonaphthon,
- Alkohole mit mehr als 4 C-Atomen wie acetylierter Lanolinalkohol, Cetylalkohol, 1-Decanol, 1-Heptanol, 1-Hexanol, Isooctadecanol, Isopropylalkohol, Oleylalkohol, Benzylalkohol,
- Carbonsäureester, z. B. Adipinsäuredialkylester wie Adipinsäurebis(2-ethylhexyl)ester, Phthalsäuredialkylester wie Phthalsäurebis(2-ethylhexyl)ester, Essigsäurealkylester (auch verzweigte Alkylgruppen) wie Ethylacetat und Acetoessigsäureethylester, Stearate wie Butylstearat, Glycerinmonostearat, Citrate wie Acetyltributylcitrat, weiterhin Cetyloctanoat, Methyloleat, Methyl-p-hydroxybenzoat, Methyltetradecanoat, Propyl-p-hydroxybenzoat, Methylbenzoat, Milchsäureester wir Isopropyllactat, Butyllactat und 2-Ethylhexyllactat,
- Pflanzenöle wie Palmöl, Rapsöl, Rizinusöl und Derivate davon wie z. B. oxydiert, Kokosnussöl, Lebertran, Maiskeimöl, Sojabohnenöl, Leinsamenöl, Olivenöl, Erdnussöl, Färberdistelöl, Sesamsamenöl, Grapefruitöl, Basilikumöl, Aprikosenöl, Ingweröl, Geranienöl, Orangenöl, Rosmarinöl, Macadamiaöl, Zwiebelöl, Mandarinenöl, Kiefernöl, Sonnenblumenöl,
- hydrogenierte Pflanzenöle wie hydrogeniertes Palmöl, hydrogeniertes Rapsöl, hydrogeniertes Sojabohnenöl,
- tierische Öle wie Schweinefettöl, Fischöle,
- Dialkylamide mittel- bis langkettiger Fettsäuren, z. B. Hallcomide, sowie
- Pflanzenölester wie Rapsölmethylester.

Die Copolymere A) können gemeinsam mit konventionellen Verdickern eingesetzt werden.

Geeignete Verdicker sind Verbindungen, die der Formulierung ein pseudoplastisches Fließverhalten verleihen, d. h. hohe Viskosität im Ruhezustand und niedrige Viskosität im bewegten Zustand. Hier sind beispielsweise Polysaccharide bzw. organische Schichtmineralien wie Xanthan Gum^{®} (Kelzan^{®} der Fa. Kelco), Rhodopol^{®} 23 (Rhone Poulenc) oder Veegum^{®} (Firma R. T. Vanderbilt) oder Attaclay^{®} (Firma Engelhardt) zu nennen, wobei Xanthan-Gum^{®} bevorzugt verwendet wird.

Als für erfindungsgemäße Dispersionen geeignete Antischaummittel kommen beispielsweise Siliconemulsionen (wie z. B. Silikon^{®} SRE, Firma Wacker oder Rhodorsil^{®} der Firma Rhodia), langkettige Alkohole, Fettsäuren, fluororganische Verbindungen und deren Gemische in Betracht.

Bakterizide können zur Stabilisierung den erfindungsgemäßen Zusammensetzungen gegen Befall mit Mikroorganismen zugesetzt werden. Geeignete Bakterizide sind beispielsweise Proxel^{®} der Fa. ICI oder Acticide^{®} RS der Fa. Thor Chemie und Kathon^{®} MK der Firma Rohm & Haas.

Geeignete Frostschutzmittel sind organische Polyole, z. B. Ethylenglycol, Propylenglycol oder Glycerin. Diese werden üblicherweise in Mengen von nicht mehr als 10 Gew.-%, bezogen auf das Gesamtgewicht der Wirkstoffzusammensetzung eingesetzt, um den gewünschten Gehalt an flüchtigen Verbindungen nicht zu überschreiten. In einer Ausführungsform der Erfindung beträgt der Anteil hiervon verschiedener flüchtiger organischer Verbindungen vorzugsweise nicht mehr als 1 Gew.-%, insbesondere nicht mehr als 1000 ppm.

Gegebenenfalls können die erfindungsgemäßen Wirkstoffzusammensetzungen 1 bis 5 Gew.-% Puffer, bezogen auf die Gesamtmenge der hergestellten Formulierung, zur pH-Wert Regulation enthalten, wobei sich die Menge und Art des eingesetzten Puffers nach den chemischen Eigenschaften des Wirkstoffes bzw. der Wirkstoffe richtet. Beispiele für Puffer sind Alkalisalze schwacher anorganischer oder organischer Säuren wie z. B. Phosphorsäure, Borsäure, Essigsäure, Propionsäure, Citronensäure, Fumarsäure, Weinsäure, Oxalsäure und Bernsteinsäure.

In einer besonders bevorzugten Ausführungsform werden die erfindungsgemäßen Copolymere als Komponente in einem kosmetischen Mittel eingesetzt. Sie können dabei, wie zuvor beschrieben, zur Modifizierung der rheologischen Eigenschaften eines kosmetischen Mittels auf Basis eines wässrigen Mediums dienen. Sie können weiterhin als Solubilisatoren für kosmetische Mittel dienen, die wenigstens einen kosmetisch akzeptablen Wirk- oder Effektstoff enthalten, der in Wasser bei 25 °C und 1013 mbar eine Löslichkeit unter 10 g/l aufweist. Unabhängig davon verfügen die erfindungsgemäßen Copolymere A) auch über gute Filmbildungseigenschaften und können als solche auch als kosmetischer Wirkstoff eingesetzt werden.

Ein weiterer Gegenstand der Erfindung ist ein kosmetisches oder pharmazeutisches Mittel, enthaltend
α) wenigstens ein Silicongruppen-haltiges Copolymer A), wie zuvor definiert, und
β) wenigstens einen kosmetisch oder pharmazeutisch akzeptablen Träger.

Vorzugsweise ist die Komponente β) ausgewählt unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Geeignete hydrophile und hydrophobe Komponenten β) sind die zuvor genannten.

Speziell geeignete kosmetisch verträgliche Öl- bzw. Fettkomponenten β) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Bevorzugte hydrophile Träger β) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglykol, Glycerin, Sorbit, etc.

Bei den erfindungsgemäßen kosmetischen Mitteln kann es sich um hautkosmetische, haarkosmetische, dermatologische, hygienische oder pharmazeutische Mittel handeln. Aufgrund ihrer filmbildenden und verdickenden Eigenschaften eignen sich die zuvor beschriebenen Copolymere A) insbesondere als Zusatzstoffe für Haar- und Hautkosmetika. Sie eignen sich speziell zur Formulierung von Gelen.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch aktiven Mittel können zusätzlich kosmetisch und/oder dermatologisch aktive Wirk- und Effektstoffe sowie Hilfsstoffe enthalten. Geeignet sind prinzipiell die zuvor genannten Wirk- und Effektstoffe B) sowie Hilfsstoffe C). In einer speziellen Ausführung enthalten die erfindungsgemäßen kosmetischen Mittel wenigstens einen wasserunlöslichen oder nur gering wasserlöslichen Wirk- oder Effektstoff.

Vorzugsweise enthalten die erfindungsgemäßen kosmetischen Mittel wenigstens ein wie vorstehend definiertes Copolymer A), wenigstens einen wie vorstehend definierten Träger β) und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, zusätzlichen Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumem, Antistatika, Emollienzien und Weichmachern.

Zusätzlich zu den Copolymeren A) geeignete konventionelle Verdickungsmittel in derartigen Formulierungen sind vernetzte Polyacrylsäuren und deren Derivate, Polysaccharide und deren Derivate, wie Xanthan-gum, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon.

Geeignete kosmetisch und/oder dermatologisch aktive Wirkstoffe sind z. B. Haut- und Haarpigmentierungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, desodorierende Wirkstoffe, sebostatische Wirkstoffe, Pflanzenextrakte, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B- und/oder UV-A-Bereich absorbieren. Geeignete UV-Filter sind die zuvor genannten. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables Polymer enthalten, das sich von den erfindungsgemäßen Copolymeren A) unterscheidet. Dazu zählen ganz allgemein anionische, kationische, amphotere und neutrale Polymere. Auf die zuvor genannten Polymere wird hier voll Bezug genommen.

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Außerdem können die Copolymere A) verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Copolymer A) zeigen vorteilhafte Wirkungen. Die Polymere können unter anderem zur Feuchthaltung und Konditionierung der Haut und zur Verbesserung des Hautgefühls beitragen. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein Copolymere A) in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Besonders Lichtschutzmittel auf Basis der Copolymere A) besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den Copolymeren A) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen Polymere auch mit herkömmlichen Polymeren, wie zuvor beschrieben, abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-ÖI-(W/O)- oder Öl-in-Wasser-(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einem Copolymere A) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann ein Copolymer A) eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-ÖI; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Neben den Copolymeren A) können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein Copolymer A) sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Weiterhin können die Duschgel-/Shampoo-Formulierungen Verdicker, wie z. B. Kochsalz, PEG-55, Propylenglykol-Oleat, PEG-120-Methylglucosedioleat und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens ein Copolymer A) in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen oder "Hot-Oil-Treatments" vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform
a) 0,05 bis 20 Gew.-% wenigstens eines Copolymers A),
b) 20 bis 99,95 Gew.-% Wasser und/oder Alkohol,
c) 0 bis 50 Gew.-% wenigstens eines Treibgases,
d) 0 bis 5 Gew.-% wenigstens eines Emulgators,
e) 0 bis 3 Gew.-% wenigstens eines Verdickers, sowie
f) bis zu 25 Gew.-% weitere Bestandteile.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymerisate eignen sich insbesondere als Festigungsmittel in Haarstyling-Zubereitungen, insbesondere Haarschäume.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4; Cetethe, z. B. Ceteth-1, Polyethylenglykolcetylether; Cetearethe, z. B. Ceteareth-25, Polyglykolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat, Cetyltrimoniumchlorid, Cetyltrimoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder PropylenoxidEinheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:
a) 0,1 bis 5 Gew.-% wenigstens eines Copolymers A),
b) 0 bis 5 Gew.-% wenigstens eines von A) verschiedenen kosmetisch akzeptablen wasserlöslichen oder wasserdispergierbaren Haarfestigerpolymers,
c) 80 bis 99,85 Gew.-% Wasser und/oder Alkohol,
d) 0 bis 1 Gew.-% eines von A) verschiedenen Gelbildners,
e) 0 bis 20 Gew.-% weitere Bestandteile.

Als zusätzliche Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquaternium-32 (und) Paraffinum Liquidum (INCI), Natriumacrylat-Copolymere (und) Paraffinum Liquidum (und) PPG-1 Trideceth-6, Acrylamidopropyltrimoniumchlorid/Acrylamid-Copolymere, Steareth-10 Allylether Acrylat-Copolymere, Polyquaternium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44. Als zusätzliche Gelbildner geeignete vernetzte Homopolymere der Acrylsäure sind beispielsweise kommerziell unter dem Namen Carbopol® von der Firma BF GOODRICH erhältlich. Bevorzugt sind auch hydrophob modifizierte vernetzte Polyacrylat Polymere, wie Carbopol® Ultrez 21 von der Firma Noveon. Weitere Beispiele für als Gelbildner geeignete anionische Polymere sind Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus (Meth)acrylsäure und Polyetheracrylaten, wobei die Polyetherkette mit einem C₈-C₃₀-Alkylrest terminiert ist. Dazu zählen z. B. Acrylat/Beheneth-25-methacrylat-Copolymere, die unter der Bezeichnung Aculyn® von der Firma Rohm und Haas erhältlich sind.

Die erfindungsgemäßen Copolymere A) können in kosmetischen Zubereitungen als Konditioniermittel eingesetzt werden.

Geeignete anionische Tenside zur Formulierung mit den Copolymeren A) sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den Copolymeren A) eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat@ Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Ein weiterer Gegenstand der Erfindung ist die Verwendung eines Copolymers A), wie zuvor definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

Die Erfindung wird anhand der folgenden nicht einschränkenden Beispiele näher erläutert.

### Herstellung von Silicon-Urethan-Polyallyl

| | | |
|---|---|---|
| Vorlage: | 138,45 g | (0,28 mol) Pluriol® A 01 0R (Fa. BASF Aktiengesellschaft) [Allylalkoholethoxilat, M_{w} = etwa 500] |
| | 0,50 g | DABCO |
| Zulauf 1: | 61,55 g | (0,28 mol) Isophorondiisocyanat |
| Zulauf 2: | 541,70 g | (0,15 mol) Pluriol® ST 4005 (Fa. BASF Aktiengesellschaft) [ethoxiliertes propoxiliertes Polydimethylsiloxan, M_{w} = etwa 8000] |
| Zulauf 3: | 184 g | Ethanol |

In einen Vierhalskolben, der mit Rührer, Tropftrichter, Thermometer, Rückflusskühler und einer Vorrichtung für das Arbeiten unter Stickstoff ausgestattet war, wurden Pluriol® A 010R und DABCO unter einer Stickstoffatmosphäre unter Erwärmen auf eine Temperatur von ca. 60 °C und unter Rühren vorgelegt. Anschließend wurden unter Rühren innerhalb von 15 Minuten Zulauf 1 zugetropft, wobei die Reaktionstemperatur anstieg. Bei einer Temperatur von etwa 78 °C wurde die Reaktionsmischung etwa 80 Minuten gerührt. Danach wurde innerhalb 10 Minuten Zulauf 2 zudosiert. Das Reaktionsgemisch wurde noch 3 h bei etwa 75 °C gerührt (Rest NCO-Gehalt etwa 0,5 %). Danach versetzte man mit dem Zulauf 3 und rührte weitere 30 Minuten bei 75 °C. Nach dem Abkühlen auf Raumtemperatur erhielt man nach Zugabe von Ethanol eine 75 gew.-%ige Silicon-Urethan-Polyallyl-Lösung.

### Allgemeine Herstellungsvorschriften

### Beispiel 5 (= Variante A1): Polymerisation von siliconhaltigem Urethan-Polyallyl/VP/MAS/VI/EGDMA

| | | |
|---|---|---|
| Vorlage: | 412 g | Butylacetat |
| Zulauf 1: | 3,6 g | 75%iges Silicon-Urethan-Polyallyl in Ethanol |
| | 63,6 g | Vinylpyrrolidon |
| | 67,5 g | Methacrylsäure |
| | 15,0 g | Vinylimidazol |
| | 1,2 g | Ethylenglykoldimethacrylat |
| Zulauf 2: | 38,2 g | Butylacetat |
| | 0,15 g | tert.-Butylperoctoat |
| Zulauf 3: | 95,6 g | Butylacetat |
| | 0,39 g | tert.-Butylperoctoat |
| Zulauf 4: | 70 g | Methylchlorid |

In einer Apparatur, die mit Rührer, Rückflusskühler, Innenthermometer und vier Zulaufvorrichtungen ausgestattet war, gab man bei 85 bis 88 °C Zulauf 1 und Zulauf 2 innerhalb von zwei Stunden zu. Man rührte das erhaltene Reaktionsgemisch noch weitere 2 Stunden bei ca. 88 °C. Danach dosierte man Zulauf 3 in 30 Minuten zu. Das Reaktionsgemisch wurde 3 Stunden bei 90 °C nachpolymerisiert. Das als weiße Pulver ausgefallene Produkt wurde mit Methylchlorid (Zulauf 4) in ca. 1 h bei 90 °C quaternisiert. Man saugte danach das erhaltene Pulver über eine Filternutsche ab, wusch zweimal mit Aceton und trocknete bei 40 °C unter vermindertem Druck.

In analoger Weise wurden die in Tabelle I angegebenen Polymere 11, 15, 26 und 33 hergestellt.

### Beispiel 6 (= Variante A2): Polymerisation von VP/ MAS/ VI / EGDMA in Gegenwart eines ethoxylierten Dimethylsiloxans (nicht erfindungsgemäß)

| | | |
|---|---|---|
| Vorlage: | 412 g | Butylacetat |
| | 1,5 g | Belsil® DMC 6031 |
| | | |
| Zulauf 1: | 64,2 g | Vinylpyrrolidon |
| | 67,5 g | Methacrylsäure |
| | 15,0 g | Vinylimidazol |
| | 1,8 g | Ethylenglykoldimethacrylat |
| | | |
| Zulauf 2: | 38,2 g | Butylacetat |
| | 0,15 g | tert.-Butylperoctoat |
| | | |
| Zulauf 3 | 95,6 g | Butylacetat |
| | 0,39 g | tert.-Butylperoctoat |
| | | |
| Zulauf 4: | 23,4 g | Triethanolamin (etwa 20 % bezogen auf Methacrylsäure) |

In einer Rührapparatur, die mit Rückflusskühler, Innenthermometer und vier Zulaufvorrichtungen ausgestattet war, wurden 1,5 g Belsil® DMC 6031 (Fa. Wacker) in 412 g Butylacetat vorgelegt. Bei 85 bis 88 °C dosierte man Zulauf 1 und Zulauf 2 innerhalb von zwei Stunden zu. Das erhaltene Reaktionsgemisch wurde bei ca. 88 °C 2 h gerührt. Danach dosierte man Zulauf 3 innerhalb von 30 Minuten zu. Das Reaktionsgemisch wurde weitere 3 Stunden bei 90 °C nachpolymerisiert. Nach dem Abkühlen auf etwa 40 °C wurde das als weißes Pulver ausgefallene Produkt mit Triethanolamin (Zulauf 4) in 1 h bei 40 °C teilneutralisiert oder mit Methylchlorid (Zulauf 4) in ca. 1 h bei 90 °C quaternisiert. Das Pulver wurde über eine Filternutsche abgesaugt, zweimal mit Aceton gewaschen und bei 40 °C unter vermindertem Druck getrocknet.

In analoger Weise wurden die in Tabelle I angegebenen Polymere 1-4, 7-10, 12-14, 16-25, 27-32 und 34-50 hergestellt.

Die in der folgenden Tabelle 1 angegebenen Polymere können auch besonders vorteilhaft nach den folgenden Herstellungsvarianten hergestellt werden. Dabei werden in der Regel Polymere mit geringen Restmonomergehalten erzielt.

| Polymer | Variante | Polymer | Variante | Polymer | Variante | Polymere | Variante |
|---|---|---|---|---|---|---|---|
| 1 | D * | 14 | C * | 27 | B * | 40 | B * |
| 2 | A2 * | 15 | A1 | 28 | B * | 41 | B * |
| 3 | B * | 16 | B * | 29 | B * | 42 | B * |
| 4 | B * | 17 | B * | 30 | B * | 43 | B * |
| 5 | A1 | 18 | B * | 31 | B * | 44 | B * |
| 6 | A2 * | 19 | B * | 32 | B * | 45 | B * |
| 7 | B * | 20 | C * | 33 | B * | 46 | B * |
| 8 | B * | 21 | B * | 34 | B * | 47 | B * |
| 9 | B * | 22 | B * | 35 | B * | 48 | B * |
| 10 | B * | 23 | B * | 36 | B * | 49 | B * |
| 11 | A1 | 24 | B * | 37 | B * | 50 | B * |
| 12 | B * | 25 | B * | 38 | B * | | |
| 13 | C * | 26 | B * | 39 | B * | | |

| | | | | | | | |
|---|---|---|---|---|---|---|---|
| * nicht erfindungsgemäß | | | | | | | |

**Tabelle I:**

| Bsp. | (EO)-Silicon^{#} | SI-UPA^{#} | VP^{#} | VCap^{#} | MAS^{#} | AS^{#} | VI^{#} | DMAP- MAM^{#} | SMA^{#} | MMA^{#} | C₁₈-PEG-MA^{#} | BMA | EGDMA^{#} | PETA-E^{#} | PEG 9000^{#} | Lu ten sol AT25^{#} | Quat. mit CH₃-Cl [%] | Neut. mit TEA NG [%] |
|---|---|---|---|---|---|---|---|---|---|---|---|---|---|---|---|---|---|---|
| 1 * | 1,0 | -- | -- | -- | -- | 94 | -- | -- | 4 | -- | -- | | -- | 1,0 | -- | -- | -- | 20 |
| 2 * | 1,0 | -- | -- | -- | 20 | 58,5 | -- | -- | -- | 15 | 5 | | -- | 0,5 | -- | -- | -- | 20 |
| 3 * | 1,0 | -- | 45 | -- | -- | 25 | -- | -- | 10 | -- | -- | | -- | 0,5 | 18,5 | -- | -- | 20 |
| 4 * | 1,0 | -- | 43 | -- | 45 | -- | -- | -- | -- | 7,5 | 2,5 | | 1,0 | -- | -- | -- | -- | 20 |
| 5 | - | 1,8 | 42,4 | -- | 45 | -- | 10 | -- | -- | -- | -- | | 0,8 | -- | -- | -- | -- | -- |
| 6 * | 1,0 | -- | 42,8 | -- | 45 | -- | 10 | -- | -- | -- | -- | | 1,2 | -- | -- | -- | -- | 20 |
| 7 * | 3,0 | -- | 42,8 | -- | 40 | -- | 8 | -- | -- | -- | -- | | 1,2 | -- | -- | 5 | -- | 20 |
| 8 * | 5,0 | -- | 42,8 | -- | 40 | -- | 6 | -- | -- | -- | -- | | 1,2 | -- | -- | 5 | -- | 20 |
| 9 * | 1,0 | -- | 44 | -- | 45 | -- | 9 | -- | -- | -- | -- | | 1,0 | -- | -- | -- | 75 | -- |
| 10 * | 1,8 | -- | 44 | -- | 42 | -- | 9 | -- | 2 | -- | -- | | 1,2 | -- | -- | -- | -- | 20 |
| 11 | -- | 2,0 | 44 | -- | 42 | -- | 9 | -- | 2 | -- | -- | | 1,0 | -- | -- | -- | -- | 20 |
| 12 * | 2,0 | -- | 43 | -- | 30 | 15 | - | 10 | -- | -- | -- | | 1,2 | -- | -- | -- | 75 | -- |
| 13 * | 2,0 | -- | 43 | -- | 30 | 15 | 10 | -- | -- | -- | -- | | 1,2 | -- | -- | -- | 75 | -- |
| 14 * | 2,0 | -- | 55 | -- | 20 | 10 | 5 | -- | 8 | -- | -- | | 1,2 | -- | -- | -- | -- | 20 |
| 15 | -- | 2,2 | 60 | -- | 25 | -- | 5 | -- | -- | 6 | 2 | | 0,8 | -- | -- | -- | -- | 20 |
| 16 * | 0,5 | -- | 33,6 | -- | 44,8 | -- | 9,95 | -- | -- | -- | -- | 9,95 | 1,2 | -- | -- | -- | ≥ 70 | -- |
| 17 * | 1,0 | -- | 78,6 | -- | 7 | -- | 10 | -- | 3 | -- | -- | | -- | 0,4 | -- | -- | 75 | -- |
| 18 * | 3,5 | -- | 73 | -- | 5 | -- | 8 | -- | -- | 7,5 | 2,5 | | -- | 0,5 | -- | -- | 75 | -- |
| 19 * | 5 | -- | 83 | -- | 3 | -- | 8 | -- | 0,5 | -- | -- | | -- | u,5 | -- | -- | 75 | -- |
| 20* | 2 | -- | 60 | -- | 10 | -- | -- | 22,5 | -- | 3.75 | 1,25 | -- | -- | 0,5 | -- | -- | 75 | -- |
| 21* | 1 | -- | 70 | -- | 4 | -- | 22 | -- | 2,5 | -- | -- | -- | -- | 0,5 | -- | -- | 75 | -- |
| 22* | 2,0 | -- | 42,9 | -- | 44,1 | -- | 9,8 | -- | -- | -- | -- | -- | 1,2 | -- | -- | -- | ≥ 70 | -- |
| 23* | 1,0 | -- | 33,5 | 9,9 | 44,5 | -- | 9,9 | -- | -- | -- | -- | -- | 1,2 | -- | -- | -- | ≥ 70 | -- |
| 24* | 1,0 | -- | 43,2 | - | 44,5 | -- | 9,9 | -- | -- | -- | -- | -- | 7,4 | -- | -- | -- | ≥ 70 | -- |
| 25* | 0,5 | -- | 43,4 | - | 44,8 | -- | 9,9 | -- | -- | -- | -- | -- | 1,4 | -- | -- | -- | ≥ 70 | -- |
| 26 | -- | 1,7 | 70 | - | 4 | -- | 22 | -- | 2 | -- | -- | | -- | 0,3 | -- | -- | 75 | -- |
| 27* | 1 | -- | 70 | - | 3,5 | -- | 15 | -- | -- | 7,5 | 2,5 | | -- | 0,5 | -- | -- | 75 | -- |
| 28* | 1,5 | -- | 50 | 23 | 5 | -- | 20 | -- | -- | -- | -- | | -- | 0,5 | -- | -- | 75 | -- |
| 29* | 4,5 | -- | 25 | 25 | 5 | -- | 40 | -- | -- | -- | -- | | -- | 0,5 | -- | -- | 75 | -- |
| 30 * | 5 | -- | -- | -- | 10 | -- | 75 | -- | -- | 7,5 | 2,5 | | -- | 0,5 | -- | -- | 75 | -- |
| 31* | 1,5 | -- | -- | -- | 5 | -- | 78 | -- | -- | -- | -- | | -- | 0,5 | -- | 15 | 90 | -- |
| 32* | 0,5 | -- | 38,6 | -- | 44,8 | -- | 5,0 | -- | -- | 7,4 | 2,5 | -- | 1,2 | - | -- | -- | - | 20 |
| 33 | - | 1,7 | -- | -- | 5 | - | 83 | -- | -- | 7,5 | 2,5 | - | -- | 0,3 | -- | -- | 90 | -- |
| 34* | 0,5 | -- | 38,6 | -- | 44,8 | -- | 14,9 | -- | -- | -- | -- | -- | 1,2 | -- | -- | -- | ≥ 70 | |
| 35* | 0,5 | -- | 43,8 | -- | 39,8 | 9,9 | 5 | -- | -- | -- | -- | -- | 1,0 | -- | -- | -- | - | 20 |
| 36* | 2 | -- | 42,9 | -- | 44,1 | -- | 9,8 | -- | -- | -- | -- | -- | 1,2 | -- | -- | -- | ≥ 70 | |
| 37* | 1,4 | -- | -- | -- | -- | -- | 83 | -- | 5 | -- | -- | | -- | 0,6 | -- | 10 | 90 | -- |
| 38* | 4,5 | -- | -- | -- | -- | -- | 85 | -- | -- | 7,5 | 2,5 | | -- | 0,5 | -- | -- | 100 | -- |
| 39* | 1,5 | -- | - | -- | -- | -- | 83 | -- | -- | -- | -- | | -- | 0,5 | -- | 15 | 90 | -- |
| 40* | 1,5 | -- | 30 * | -- | -- | -- | 53 | -- | -- | -- | -- | | -- | 0,5 | -- | 15 | 90 | -- |
| 41* | 2 | -- | 33,1 | -- | 44,1 | -- | 9,8 | -- | -- | 9,8 | -- | -- | 1,2 | -- | -- | -- | ≥ 70 | -- |
| 42* | 1,5 | -- | 50 | -- | -- | -- | 33 | -- | -- | -- | -- | | -- | 0,5 | -- | 15 | 90 | -- |
| 43 * | 0,5 | -- | 59 | -- | -- | -- | 25 | -- | -- | -- | -- | | -- | 0,5 | -- | 15 | 90 | -- |
| 44* | 1 | -- | 37,8 | -- | 45 | -- | 5 | -- | -- | 10 | -- | | 1,2 | -- | -- | -- | -- | 20 |
| 45* | 1 | -- | 25,8 | -- | 45 | -- | 7 | -- | -- | 20 | -- | | 1,2 | -- | -- | -- | -- | 20 |
| 46* | 1 | -- | 12,8 | -- | 45 | -- | 10 | -- | -- | 30 | -- | | 1,2 | -- | -- | -- | -- | 20 |
| 47* | 1 | -- | -- | -- | 45 | 7,8 | 10 | -- | -- | 35 | -- | | 1,2 | -- | -- | -- | -- | 20 |
| 48* | 1 | -- | 37,8 | 10 | 40 | -- | 5 | -- | -- | -- | -- | | 1,2 | -- | - | 5 | 80 | -- |
| 49* | 1 | -- | 32,8 | -- | 45 | -- | 10 | -- | -- | -- | -- | | 1,2 | -- | -- | 10 | 80 | -- |
| 50* | 1 | -- | 27,8 | -- | 45 | -- | 15 | -- | -- | -- | -- | | 1,2 | -- | -- | 10 | 80 | -- |

| | | | | | | | | | | | | | | | | | | |
|---|---|---|---|---|---|---|---|---|---|---|---|---|---|---|---|---|---|---|
| Bsp. Beispiel # Die Mengenangaben sind in Gew.-%, bezogen auf die zur Polymerisation eingesetzten Verbindungen + Si-haltiger Verdicker (EO)-Silion Belsil® 6031, ethoxiliertes Dimethylsiloxan; SI-UPA siliconhaltiges Urethan-Polyallyl VP Vinylpyrrolidon VCap N-Vinylcaprolactam MAS Methacrylsäure AS Acrylsäure VI Vinylimidazol DMAPMAM Dimethylaminopropylmethacrylamid SMA Stearylmethacrylat MMA Methylmethacrylat * = nicht erfindungsgemäß C₁₈-PEG-MA mit einem C₁₈-Fettalkohol terminiertes Polyethylenglykolmethacrylat BMA n-Butylmethacrylat EGDMA Ethylenglykoldimethacrylat PETAE Pentaerythrittriallylether PEG 9000 Polyethylenglykol, M_{w} = 9 000 Lutensol® AT 25 C₁₆-C₁₈-Fettalkoholethoxylat mit 25 EO-Einheiten Quat. Quaternisierung Neut. Neutralisierung TEA Triethylamin NG % Neutralisierungsgrad in % | | | | | | | | | | | | | | | | | | |

### Beispiel 83 (Variante B): Polymerisation von VI/MAS/VP/PETAE in Gegenwart eines ethoxilierten Dimethylsiloxans unter Verwendung von zwei Radikalinitiatoren mit unterschiedlicher Zerfallstemperatur (nicht erfindungsgemäß)

| | | |
|---|---|---|
| Vorlage: | 613 g | Butylacetat |
| | 2 g | Belsil® DMC 6031 |
| | 1 g | Trigonox® 101 (2,5-Dimethyl-2,5-di(tert.-butylperoxy)hexan) |
| | | |
| Zulauf 1: | 94 g | Vinylpyrrolidon |
| | 102 g | Vinylimidazol |
| | 6,95 g | Methacrylsäure |
| | 1,2 g | Pentaerythrittriallylether |
| | | |
| Zulauf 2: | 35 g | n-Butylacetat |
| | 0,2 g | tert.-Butylperoctoat |
| | | |
| Zulauf 3 | 175 g | n-Butylacetat |
| | 1,0 g | tert.-Butylperoctoat |
| | | |
| Zulauf 4: | 175 g | n-Butylacetat |
| | 1,0 g | tert.-Butylperoctoat |

In einer Apparatur, die mit Rührer, Rückflusszähler, Innenthermometer und vier Zulaufvorrichtungen ausgestattet war, erwärmte man die Vorlage unter Stickstoffatmosphäre auf 90 °C. Die Zuläufe 1 und 2 wurden innerhalb von 3 h zugegeben und bei 90 °C weitere 1,5 h gerührt. Zulauf 3 wurde bei 100 °C in 1 h zugeben, der Reaktionsansatz 1 h bei dieser Temperatur gerührt. Anschließend wurde Zulauf 4 bei 100 °C innerhalb von 1 h zugegeben und dann nochmals 2 h bei 100 °C gerührt. Die Temperatur wurde auf 125 °C erhöht und bei dieser Temperatur noch 2 h weitergerührt. Die erhaltene weiße Suspension wurde anschließend mit 50 g Methylchlorid quaternisiert. Das Produkt wurde filtriert, mit Aceton gewaschen und bei 70 °C im Vakuum getrocknet.

In analoger Weise wurden alle Produkte - der Variante gemäß Tabelle 2 hergestellt.

### Beispiel 73 (Variante C): Polymerisation von AS/DMAPMAM/PETAE in Gegenwart eines ethoxilierten Dimethylsiloxans unter Verwendung von zwei Radikalinitiatoren mit unterschiedlicher Zerfallstemperatur, (nicht erfindungsgemäß)

| | | |
|---|---|---|
| Vorlage: | 800 g | Ethylacetat |
| | 1,6 g | Belsil® DMC 6031 |
| | 1 g | tert.-Butylperoctoat |
| | | |
| Zulauf 1: | 125 g | Acrylsäure |
| | | |
| Zulauf 2: | 45 g | DMAPMAM |
| | 1,6 g | Pentaerythrittriallylether |
| | | |
| Zulauf 3 | 80 g | Ethylacetat |
| | 0,4 g | Lauroylperoxid |
| | | |
| Zulauf 4: | 200 g | thylacetat |
| | 0,4 g | Lauroylperoxid |

In einer Apparatur, die mit Rührer, Rückflusszähler, Innenthermometer und vier Zulaufvorrichtungen ausgestattet war, erwärmte man die Vorlage unter Stickstoffatmosphäre und Rühren auf 75 °C. Die Zuläufe 1, 2 und 3 wurden innerhalb von 3 h zugegeben und bei 75 °C weitere 2 h gerührt. Zulauf 4 wurde in 1 h bei 80 °C zugeben und dann noch 1 h weitergerührt. Die Temperatur wurde auf 100 °C erhöht und bei dieser Temperatur noch weitere 3 h gerührt. Die erhaltene weiße Suspension wurde anschließend mit 40 g Methylchlorid quaternisiert. Das Produkt wurde filtriert, mit Aceton gewaschen und bei 70 °C im Vakuum getrocknet.

In analoger Weise wurden alle Produkte der Variante C gemäß Tabelle 2 hergestellt.

### Beispiel 52 (Variante D): Polymerisation von AS/DMAPMAM/SMA/PETAE in Gegenwart eines ethoxilierten Dimethylsiloxans unter Verwendung von zwei Radikalinitiatoren mit unterschiedlicher Zerfallstemperatur (nicht erfindungsgemäß)

| | | |
|---|---|---|
| Vorlage: | 670 g | Ethylacetat/Cyclohexan (65:35) |
| | 2 g | Belsil®DMC 6031 |
| | 50 g | Zulauf 1 |
| | 14 g | Zulauf 2 |
| | 1,5 g | Pentaerythrittriallylether |
| | 1,5 g | tert.-Butylperoctoat |
| | | |
| Zulauf 1: | 142,5 g | Acrylsäure |
| | 3 g | Stearylmethacrylat |
| | 3 g | Dimethylaminopropylmethacrylamid |
| | 100 g | Ethylacetat/Cyclohexan (65:35) |
| | 4,3 g | wasserfreies K₂CO₃ |
| | | |
| Zulauf 2: | 70 g | Ethylacetat/Cyclohexan (65:35) |
| | 0,35 g | Trigonox® EHP-C75 (75%ig) |
| | | |
| Zulauf 3 | 70 g | Ethylacetat/Cyclohexan (65:35) |
| | 1,0 g | Trigonox® EHP-C75 (75%ig) |

In einer Apparatur, die mit Rührer, Rückflusszähler, Innenthermometer und drei Zulaufvorrichtungen ausgestattet war, erwärmte man die Vorlage unter Stickstoffatmosphäre und Rühren auf 50 °C. Der Zulauf 1 wurde innerhalb von 1,5 h und der Zulauf 2 innerhalb von 2 h zugegeben und der Ansatz bei 60 °C noch weitere 2 h gerührt. Zulauf 3 wurde in 1 h bei 60 °C zugegeben und dann noch 2 h bei 70 °C gerührt. Die Temperatur wurde auf 100 °C erhöht und bei der Temperatur noch 3 h weitergerührt. Die erhaltene weiße Suspension wurde filtriert, mit Aceton gewaschen und bei 70 °C im Vakuum getrocknet.

In analoger Weise wurden alle Produkte der Variante D gemäß Tabelle 2 hergestellt.

**Tabelle 2:**

| Bsp. | (EO)-Silicon^{#} | VP^{#} | MAS^{#} | AS^{#} | VI^{#} | DMAPMAM^{#} | SMA^{#} | n-BA^{#} | EMA^{#} | PLEX-O^{#} | EGDMA^{#} | PETAE^{#} | Herstellungsvariante |
|---|---|---|---|---|---|---|---|---|---|---|---|---|---|
| 51* | 2 | -- | 90 | -- | 4 | -- | 5 | -- | -- | -- | 1,0 | -- | C |
| 52* | 2 | -- | -- | 95 | -- | 2 | 2 | -- | -- | -- | -- | 1,0 | D |
| 53* | 2 | -- | 85 | -- | 10 | -- | 3,5 | -- | -- | -- | 1,5 | -- | C |
| 54* | 2 | -- | -- | 92 | -- | 4 | 2,5 | -- | -- | -- | -- | 1,5 | D |
| 55* | 2 | -- | -- | 85 | -- | 12 | 1,8 | -- | -- | -- | -- | 1,2 | C |
| 56* | 2 | -- | 85 | -- | 5 | 5 | 3,8 | -- | -- | -- | 1,2 | -- | C |
| 57* | 2 | -- | 78 | -- | 10 | 7 | 3,8 | -- | -- | -- | 1,2 | -- | B |
| 58* | 2 | -- | -- | 75 | -- | 10 | -- | -- | 13,5 | -- | -- | 1,5 | C |
| 59* | 1 | -- | 75 | -- | -- | 23,5 | -- | -- | -- | -- | 1,5 | -- | C |
| 60* | 1 | -- | 70 | -- | -- | 23,5 | -- | -- | -- | 5 | 1,5 | -- | C |
| 61* | 1 | - | 45 | -- | -- | 48,5 | -- | -- | -- | 5 | 1,5 | -- | C |
| 62* | 1 | -- | 50 | -- | 25 | 23,8 | -- | -- | -- | -- | 1,2 | -- | C |
| 63* | 1 | -- | 50 | -- | 25 | 20 | 3,8 | -- | -- | -- | 1,2 | -- | C |
| 64* | 1 | -- | 45 | -- | - | 38,5 | -- | 15 | -- | -- | 1,5 | -- | C |
| 65* | 1 | -- | 45 | -- | -- | 38,5 | -- | 10 | -- | 5 | 1,5 | -- | C |
| 66* | 1 | -- | 45 | - | -- | 33,5 | -- | 15 | -- | 5 | 1,5 | -- | C |
| 67* | 1 | -- | 45 | -- | -- | 33,5 | -- | 25 | -- | -- | 1,5 | -- | C |
| 68* | 1 | -- | 45 | -- | -- | 28,5 | -- | 25 | -- | -- | 1,5 | -- | C |
| 69* | 1 | -- | 45 | -- | -- | 28 | -- | 25 | -- | -- | 2 | -- | C |
| 70* | 1 | -- | 45 | - | -- | 123,5 | -- | 30 | -- | - | 1,5 | -- | C |
| 71* | 1 | -- | 40 | -- | -- | 23,5 | -- | 35 | -- | -- | 1,5 | -- | C |
| 72* | 1 | -- | 70 | -- | -- | 28,8 | -- | -- | -- | -- | 1,2 | -- | C |
| 73* | 1 | -- | -- | 70 | -- | 28,8 | -- | -- | -- | -- | -- | 1,2 | C |
| 74* | 1 | 25 | 70 | -- | -- | 3,8 | -- | -- | -- | -- | 1,2 | -- | B |
| 75* | 1 | 25 | - | 70 | - | 3,8 | -- | -- | -- | -- | 1,2 | -- | B |
| 76* | 1 | 25 | 50 | -- | -- | 23,8 | -- | -- | -- | -- | 1,2 | -- | C |
| 77* | 1 | 25 | -- | 50 | -- | 23,8 | -- | -- | -- | -- | -- | 1,2 | C |
| 78* | 1 | 45 | 50 | -- | -- | 3,8 | -- | -- | -- | -- | 1,2 | -- | C |
| 79* | 1 | 45 | -- | 50 | -- | 3,8 | -- | -- | -- | -- | - | 1,2 | C |
| 80* | 1 | 45 | 30 | -- | -- | 23,8 | -- | -- | -- | -- | 1,2 | -- | C |
| 81* | 1 | 45 | -- | 30 | -- | 23,8 | -- | -- | -- | -- | 1,2 | -- | C |
| 82* | 1 | 61 | 3,3 | -- | 35 | -- | -- | - | -- | -- | -- | 0,7 | B |
| 83* | 1 | 46 | 3,3 | -- | 50 | -- | -- | -- | -- | -- | -- | 0,7 | B |
| 84* | 1 | 31 | 3,3 | -- | 65 | -- | -- | -- | -- | -- | -- | 0,7 | B |
| 85* | 1 | 16 | 3,3 | -- | 80 | -- | -- | -- | -- | -- | -- | 0,7 | B |
| 86* | 2 | -- | 4,5 | -- | 85 | -- | -- | -- | 10 | -- | -- | 0,5 | B |
| 87* | 2 | -- | 5,5 | -- | 94 | -- | -- | -- | -- | -- | -- | 0,5 | B |
| 88* | 2 | -- | 5,5 | -- | 90 | -- | 4 | -- | 4 | -- | -- | 0,5 | B |
| 89* | 1 | -- | 3,3 | -- | 93 | -- | 3 | -- | 3 | -- | -- | 0,7 | B |
| 90* | 1 | -- | 2,3 | -- | 95 | -- | 2 | -- | 2 | -- | -- | 0,7 | B |

| | | | | | | | | | | | | | |
|---|---|---|---|---|---|---|---|---|---|---|---|---|---|
| Bsp. Beispiel # Die Mengenangaben sind in Gew.-%, bezogen auf die zur Polymerisation eingesetzten ungesättigten Verbindungen, die Gewichtsteile Si-haltiger Vebindung sind separat angegeben (EO)-Silicon Belsil® 6031, ethoxiliertes Dimethylsiloxan VP Vinylpyrrolidon MAS ethacrylsäure AS Acrylsäure VI Vinylimidazol DMAPMAM Dimethylaminopropylmethacrylamid SMA Stearylmethacrylat EGDMA thylenglykoldimethacrylat PETAE Pentaerythrittriallylether EMA Ethylmethacrylat n-BA n-butylacrylat PLEX-O Plex® 6877-0 = ethacrylsäureester eines mit 25 Mol Ethylenoxid alkoxilierten C₁₆-C₁₈-Fettalkohols als 25%ige Lösung in Methylmethacrylat * = nicht erfindungsgemäß | | | | | | | | | | | | | |

## Patentansprüche

1. Silicongruppen-haltiges Copolymer A) erhältlich durch radikalische Copolymerisation von
a) wenigstens einer Verbindung mit einer radikalisch polymerisierbaren, a,β-ethylenisch ungesättigten Doppelbindung und mindestens einer ionogenen und/oder ionischen Gruppe pro Molekül, wobei die Monomere a) ausschließlich ausgewählt sind unter Verbindungen a1) mit anionogenen und/oder anionischen Gruppen oder wobei die Komponente a) wenigstens eine Verbindung mit mindestens einer anionogenen und/oder anionischen Gruppe a1) und wenigstens eine Verbindung mit mindestens einer kationogenen und/oder kationischen Gruppe a2) umfasst, und wobei das Copolymer A) einen molaren Überschuss an anionogenen/anionischen Gruppen gegenüber kationogenen/kationischen Gruppen von größer als 1 : 1 aufweist,
b) wenigstens einer radikalisch polymerisierbaren vernetzenden Verbindung, die wenigstens zwei a,β-ethylenisch ungesättigte Doppelbindungen pro Molekül enthält,
in Gegenwart wenigstens einer eine radikalisch polymerisierbare olefinisch ungesättigte Doppelbindung enthaltenden Siliconverbindung c), wobei es sich bei der wenigstens eine radikalisch polymerisierbare Doppelbindung aufweisenden Siliconverbindung um ein radikalisch polymerisierbares Siloxangruppen-haltiges Urethan(meth)acrylat handelt.

2. Copolymer A) nach Anspruch 1, das durch radikalische Copolymerisation nach der Methode der Fällungspolymerisation erhältlich ist.

3. Copolymer nach einem der Ansprüche 1 oder 2, wobei die Komponente a) wenigstens eine Verbindung a1) mit mindestens einer anionogenen und/oder anionischen Gruppe umfasst, die ausgewählt ist unter Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure, 2-Acrylamido-2-methylpropansulfonsäure, Vinylphosphonsäure und Mischungen davon.

4. Copolymer nach einem der vorhergehenden Ansprüche, wobei die Komponente a) wenigstens eine Verbindung a2) mit mindestens einer kationogenen und/oder kationischen Gruppe umfasst, die ausgewählt ist unter Estern a,β-ethylenisch ungesättigter Mono- und Dicarbonsäuren mit Aminoalkoholen, welche am Aminstickstoff mono- oder dialkyliert sein können, Amiden a,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen und Mischungen davon.

5. Copolymer nach einem der vorhergehenden Ansprüche, das zusätzlich wenigstens ein weiteres Monomer d) einpolymerisiert enthält, das ausgewählt ist unter α,β-ethylenisch ungesättigten amidgruppenhaltigen Verbindung der allgemeinen Formel I 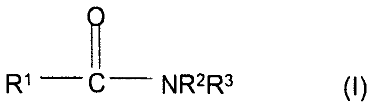 wobei
einer der Reste R¹ bis R³ für eine Gruppe der Formel CH₂=C_{R}⁴- mit R⁴ = H oder C₁-C₄-Alkyl steht und die übrigen Reste R¹ bis R³ unabhängig voneinander für H, Alkyl, Cycloalkyl, Heterocycloalkyl, Aryl oder Hetaryl stehen,
wobei R¹ und R² gemeinsam mit der Amidgruppe, an die sie gebunden sind, auch für ein Lactam mit 5 bis 8 Ringatomen stehen können,
wobei R² und R³ gemeinsam mit dem Stickstoffatom, an das sie gebunden sind, auch für einen fünf- bis siebengliedrigen Heterocyclus stehen können,
mit der Maßgabe, dass die Summe der Kohlenstoffatome der Reste R¹, R² und R³ höchstens 8 beträgt.

6. Copolymer nach einem der vorhergehenden Ansprüche, das zusätzlich wenigstens eine Verbindung e) einpolymerisiert enthält, die ausgewählt ist unter Verbindungen der allgemeinen Formeln III a), III b), III c), III d) und III e) 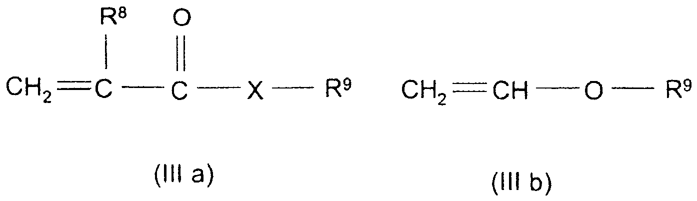 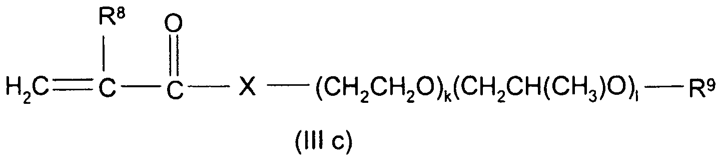 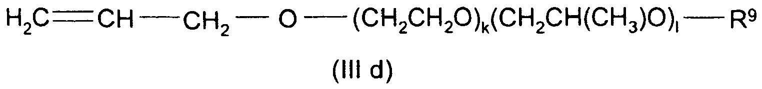 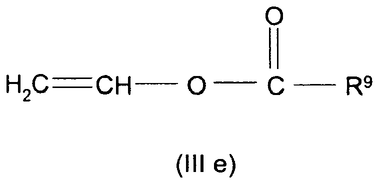 worin
die Reihenfolge der Alkylenoxideinheiten beliebig ist,
k und I unabhängig voneinander für eine ganze Zahl von 0 bis 1000 stehen, wobei die Summe aus k und I mindestens 5 beträgt,
R⁸ für Wasserstoff oder C₁-C₄-Alkyl, vorzugsweise Methyl, steht,
R⁹ für C₈-C₃₀-Alkyl oder C₈-C₃₀-Alkenyl steht, und
X für O oder eine Gruppe der Formel NR¹⁰ steht, worin R¹⁰ für H, Alkyl, Alkenyl, Cycloalkyl, Heterocycloalkyl, Aryl oder Hetaryl steht.

7. Copolymer nach einem der vorhergehenden Ansprüche, das zusätzlich wenigstens eine Verbindung f) einpolymerisiert enthält, die ausgewählt ist unter Verbindungen der allgemeinen Formeln III a*), III b*), III c*), III d*) und III e*) 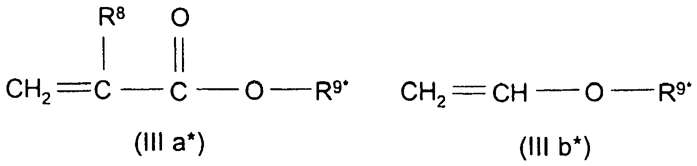 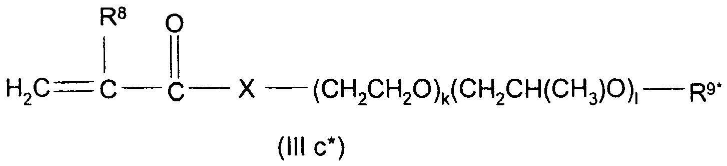
H₂C=CH-CH₂-O-(CH₂CH₂O)ₖ(CH₂CH(CH₃)O)₁-R^{9*} (III d*) 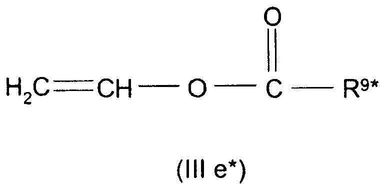
worin
die Reihenfolge der Alkylenoxideinheiten beliebig ist,
k und I unabhängig voneinander für eine ganze Zahl von 0 bis 1000 stehen, wobei die Summe aus k und I mindestens 5 beträgt,
R⁸ für Wasserstoff oder C₁-C₄-Alkyl, vorzugsweise Methyl, steht,
R^{9*} für Wasserstoff, C₁-C₈-Alkyl oder C₃-C₈-Alkenyl steht, und
X für O oder eine Gruppe der Formel NR¹⁰ steht, worin R¹⁰ für H, Alkyl, Alkenyl, Cycloalkyl, Heterocycloalkyl, Aryl oder Hetaryl steht.

8. Copolymer nach einem der vorhergehenden Ansprüche, das zusätzlich wenigstens eine Verbindung g) einpolymerisiert enthält, die ausgewählt ist unter Estern a,β-ethylenisch ungesättigter Mono- und Dicarbonsäuren mit C₂-C₃₀-Diolen, Amiden a,β-ethylenisch ungesättigter Mono- und Dicarbonsäuren mit C₂-C₃₀-Aminoalkoholen, die eine primäre oder sekundäre Aminogruppe aufweisen, Estern von Allylalkohol mit C₁-C₇-Monocarbonsäuren, von III c) und III c*) verschiedenen Polyetheracrylaten, Vinylaromaten, Vinylhalogeniden, Vinylidenhalogeniden, C₁-C₈-Monoolefinen, nicht aromatischen Kohlenwasserstoffen mit mindestens zwei konjugierten Doppelbindungen und Mischungen davon.

9. Copolymer A) nach einem der Ansprüche 1 bis 8, das
- wenigstens 2 Gew.-% mindestens einer Verbindung a1) mit einer radikalisch polymerisierbaren, a,β-ethylenisch ungesättigten Doppelbindung und mindestens einer anionogenen und/oder anionischen Gruppe pro Molekül, vorzugsweise Acrylsäure und/oder Methacrylsäure,
- 0,05 bis 5 Gew.-% wenigstens eines Vernetzers b), vorzugsweise Ethylenglykoldi(meth)acrylat und/oder Pentaerythrittriallylether,
- 0,05 bis 30 Gew.-% wenigstens einer Siliconverbindung c), einpolymerisiert enthält.

10. Copolymer A) nach einem der Ansprüche 1 bis 8, das
- wenigstens 2 Gew.-% mindestens einer Verbindung a1) mit einer radikalisch polymerisierbaren, a,β-ethylenisch ungesättigten Doppelbindung und mindestens einer anionogenen und/oder anionischen Gruppe pro Molekül, vorzugsweise Acrylsäure und/oder Methacrylsäure,
- 0,05 bis 5 Gew.-% wenigstens eines Vernetzers b), vorzugsweise Ethylenglykoldi(meth)acrylat und/oder Pentaerythrittriallylether,
- 0,05 bis 30 Gew.-% wenigstens einer Siliconverbindung c),
- 20 bis 95 Gew.-% Vinylpyrrolidon d),
- 0,1 bis 20 Gew.-% wenigstens einer Verbindung e), die vorzugsweise ausgewählt ist unter C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon,
- 5 bis 40 Gew.-% wenigstens eines Monomers f), das vorzugsweise ausgewählt ist unter C₁-C₆-(Meth)acrylaten, insbesondere Methyl(meth)acrylat, Ethyl(meth)acrylat, n-Butyl(meth)acrylat und Mischungen davon, einpolymerisiert enthält.

11. Copolymer A) nach einem der Ansprüche 1 bis 10, erhältlich durch radikalische Copolymerisation von
- wenigstens 2 Gew.-%, bezogen auf das Gesamtgewicht der zur Polymerisation eingesetzten Monomere, wenigstens eines Monomerenpaars aus wenigstens einer N-Vinylimidazol-Verbindung a2) und Acrylsäure und/oder Methacrylsäure a1),
- wenigstens 1 Gew.-%, bezogen auf das Gesamtgewicht der zur Polymerisation eingesetzten Monomere, wenigstens eines zusätzlichen Monomers a1) mit einer anionogenen oder anionischen Gruppe oder wenigstens eines zusätzlichen Monomers a2) mit einer kationogenen oder kationischen Gruppe,
- 0,05 bis 5 Gew.-%, bezogen auf das Gesamtgewicht der zur Polymerisation eingesetzten Monomere, wenigstens einer radikalisch polymerisierbaren vernetzenden Verbindung b),
- 0,05 bis 30 Gew.-%, besonders bevorzugt 0,1 bis 20 Gew.-% wenigstens einer Siliconverbindung c),
- 0 bis 95 Gew.-% wenigstens eines amidgruppenhaltigen Monomers d),
- 0 bis 40 Gew.-%, bezogen auf das Gesamtgewicht der zur Polymerisation eingesetzten Monomere, wenigstens eines hydrophoben Monomers e),
- 0 bis 40 Gew.-%, bezogen auf das Gesamtgewicht der zur Polymerisation eingesetzten Monomere, wenigstens eines Monomers f).

12. Verfahren zur Herstellung eines Silicongruppen-haltigen Copolymers A), wie in einem der Ansprüche 1 bis 11 definiert, durch radikalische Copolymerisation nach der Methode der Fällungspolymerisation.

13. Verfahren nach Anspruch 12, wobei die Polymerisation in einem weitgehend wasserfreien, aprotischen Lösungsmittel oder Lösungsmittelgemisch, vorzugsweise in Ethylacetat und/oder n-Butylacetat, erfolgt.

14. Verfahren nach einem der Ansprüche 12 oder 13, bei dem zur Copolymerisation wenigstens zwei Initiatoren eingesetzt werden, deren Zerfallstemperaturen um wenigstens 10 °C, vorzugsweise um wenigstens 15 °C, voneinander verschieden sind.

15. Verfahren nach Anspruch 14, wobei die Copolymerisation bis zum Abschluss der Fällung des Copolymers bei einer Temperatur größer oder gleich der niedrigeren Zerfallstemperatur und kleiner der höheren Zerfallstemperatur erfolgt und nach der Fällung eine weitere Umsetzung bei einer Temperatur größer oder gleich der höheren Zerfallstemperatur erfolgt.

16. Verfahren nach einem der Ansprüche 12 bis 14, umfassend eine erste Polymerisationsphase bei einer ersten Polymerisationstemperatur und eine zweite Polymerisationsphase bei einer zweiten Polymerisationstemperatur oberhalb der ersten Polymerisationstemperatur, wobei zur Polymerisation wenigstens zwei Initiatoren eingesetzt werden, deren Halbwertszeiten bei der ersten Polymerisationstemperatur sich so unterscheiden, dass wenigstens einer dieser Initiatoren während der ersten Polymerisationsphase in Radikale zerfällt und wenigstens einer dieser Initiatoren während der ersten Polymerisationsphase im Wesentlichen nicht in Radikale zerfällt und während der zweiten Polymerisationsphase in Radikale zerfällt.

17. Kosmetisches oder pharmazeutisches Mittel, enthaltend
A) wenigstens ein silicongruppen-haltiges Copolymer wie in einem der Ansprüche 1 bis 11 definiert,
B) wenigstens einen kosmetisch oder pharmazeutisch akzeptablen Wirk- oder Effektstoff, und
C) gegebenenfalls wenigstens einen weiteren, von B) verschiedenen kosmetisch oder pharmazeutisch akzeptablen Hilfsstoff.

## Claims

1. A copolymer A) containing silicone groups, obtainable by free-radical copolymerization of
a) at least one compound with a free-radically polymerizable, α,β-ethylenically unsaturated double bond and at least one ionogenic and/or ionic group per molecule, where the monomers a) are exclusively chosen from compounds a1) with anionogenic and/or anionic groups or where the component a) comprises at least one compound with at least one anionogenic and/or anionic group a1) and at least one compound with at least one cationogenic and/or cationic group a2), and where the copolymer A) has a molar excess of anionogenic/anionic groups compared to cationogenic/cationic groups of greater than 1:1.
b) at least one free-radically polymerizable crosslinking compound which comprises at least two α,β-ethylenically unsaturated double bonds per molecule,
in the presence of at least one silicone compound c) which comprises a free-radically polymerizable olefinically unsaturated double bond, where the silicone compound which has at least one free-radically polymerizable double bond is a free-radically polymerizable methane (meth)acrylate which contains siloxane groups.

2. The copolymer A) according to claim 1, which is obtainable by free-radical copolymerization in accordance with the method of precipitation polymerization.

3. The copolymer according to either of claims 1 or 2, where the component a) comprises at least one compound a1) with at least one anionogenic and/or anionic group which is chosen from acrylic acid, methacrylic acid, ethacrylic acid, α-chloroacrylic acid, crotonic acid, maleic acid, maleic anhydride, fumaric acid, itaconic acid, citraconic acid, mesaconic acid, glutaconic acid, aconitic acid, 2-acrylamido-2-methylpropanesulfonic acid, vinylphosphonic acid and mixtures thereof.

4. The copolymer according to any of the preceding claims, where the component a) comprises at least one compound a2) with at least one cationogenic and/or cationic group which is chosen from esters of α,β-ethylenically unsaturated mono- and dicarboxylic acids with amino alcohols, which may be mono- or dialkylated on the amine nitrogen, amides of α,β-ethylenically unsaturated mono- and dicarboxylic acids with diamines which have at least one primary or secondary amino group, N,N-diallylamine, N,N-diallyl-N-alkylamines and derivatives thereof, vinyl- and allyl-substituted nitrogen heterocycles, vinyl- and allyl-substituted heteroaromatic compounds and mixtures thereof.

5. The copolymer according to any of the preceding claims, which additionally comprises at least one further monomer d) in copolymerized form which is chosen from α,β-ethylenically unsaturated compounds containing amide groups and of the general formula I 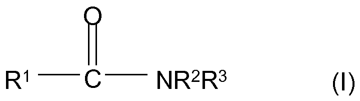 where
one of the radicals R¹ to R³ is a group of the formula CH₂=CR⁴- where R⁴ is H or C₁-C₄-alkyl and the other radicals R¹ to R³, independently of one another, are H, alkyl, cycloalkyl, heterocycloalkyl, aryl or hetaryl,
where R¹ and R², together with the amide group to which they are bonded, may also be a lactam having 5 to 8 ring atoms,
where R² and R³, together with the nitrogen atom to which they are bonded, may also be a five- to seven-membered heterocycle,
with the proviso that the sum of the carbon atoms of the radicals R¹, R² and R³ is at most 8.

6. The copolymer according to any of the preceding claims, which additionally comprises at least one compound e) in copolymerized form which is chosen from compounds of the general formulae III a), III b), III c), III d) and III e) 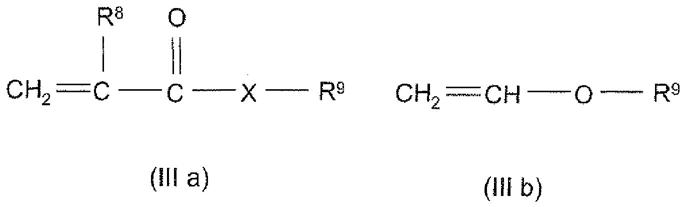 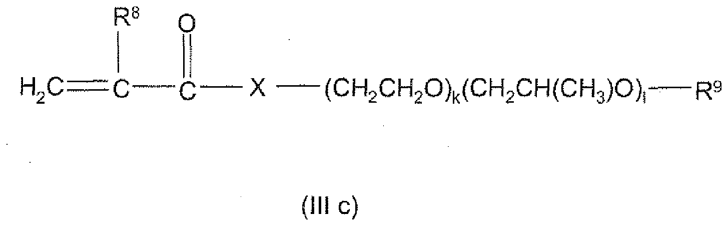
H₂C=CH-CH₂-O-(CH₂CH₂O)ₖ(CH₂CH(CH₃)O)ₗ-R⁹ (III d) 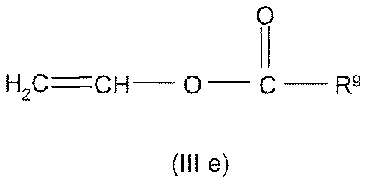
in which
the order of the alkylene oxide units is arbitrary,
k and l, independently of one another, are an integer from 0 to 1000, where the sum of k and l is at least 5,
R⁸ is hydrogen or C₁-C₄-alkyl, preferably methyl,
R⁹ is C₈-C₃₀-alkyl or C₈-C₃₀-alkenyl, and
X is O or a group of the formula NR¹⁰, in which R¹⁰ is H, alkyl, alkenyl, cycloalkyl, heterocycloalkyl, aryl or hetaryl.

7. The copolymer according to any of the preceding claims, which additionally comprises at least one compound f) in copolymerized form which is chosen from compounds of the general formulae III a*), III b*), III c*), III d*) and III e*) 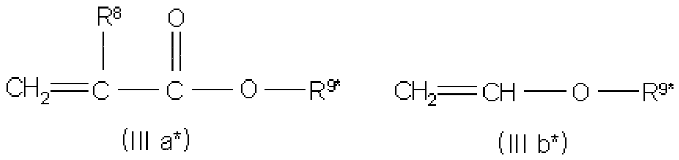 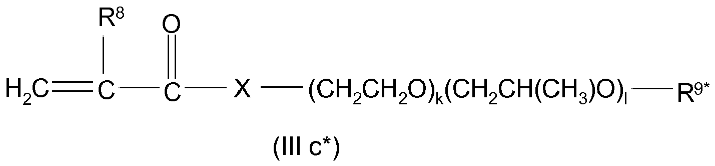
H₂C=CH-CH₂-O-(CH₂CH₂O)ₖ(CH₂CH(CH₃)O)ₗ-R^{9*} (III d*) 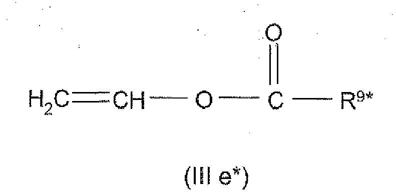
in which
the order of the alkylene oxide units is arbitrary,
k and l, independently of one another, are an integer from 0 to 1000, where the sum of k and 1 is at least 5,
R⁸ is hydrogen or C₁-C₄-alkyl, preferably methyl,
R^{9*} is hydrogen, C₁-C₈-alkyl or C₃-C₈-alkenyl, and
X is O or a group of the formula NR¹⁰, in which R¹⁰ is H, alkyl, alkenyl, cycloalkyl, heterocycloalkyl, aryl or hetaryl.

8. The copolymer according to any of the preceding claims, which additionally comprises at least one compound g) in copolymerized form which is chosen from esters of α,β-ethylenically unsaturated mono- and dicarboxylic acids with C₂-C₃₀-diols, amides of α,β-ethylenically unsaturated mono- and dicarboxylic acids with C₂-C₃₀-amino alcohols having a primary or secondary amino group, esters of allyl alcohol with C₁-C₇-monocarboxylic acids, polyether acrylates different from III c) and III c*), vinylaromatics, vinyl halides, vinylidene halides, C₁-C₈-monoolefins, nonaromatic hydrocarbons with at least two conjugated double bonds and mixtures thereof.

9. The copolymer A) according to any of claims 1 to 8, which comprises, in copolymerized form,
- at least 2% by weight of at least one compound a1) with a free-radically polymerizable, α,β-ethylenically unsaturated double bond and at least one anionogenic and/or anionic group per molecule, preferably acrylic acid and/or methacrylic acid,
- 0.05 to 5% by weight of at least one crosslinker b), preferably ethylene glycol di(meth)acrylate and/or pentaerythritol triallyl ether,
- 0.05 to 30% by weight of at least one silicone compound c).

10. The copolymer A) according to any of claims 1 to 8, which comprises, in copolymerized form,
- at least 2% by weight of at least one compound a1) with a free-radically polymerizable, α,β-ethylenically unsaturated double bond and at least one anionogenic and/or anionic group per molecule, preferably acrylic acid and/or methacrylic acid,
- 0.05 to 5% by weight of at least one crosslinker b), preferably ethylene glycol di(meth)acrylate and/or pentaerythritol triallyl ether,
- 0.05 to 30% by weight of at least one silicone compound c),
- 20 to 95% by weight of vinylpyrrolidone d),
- 0.1 to 20% by weight of at least one compound e) which is preferably chosen from C₈-C₂₂-(meth)acrylates, C₈-C₂₂-alkyl vinyl ethers, polyether (meth)acrylates terminated with C₈-C₂₂-alkyl groups, allyl alcohol alkoxylates terminated with C₈-C₂₂-alkyl groups, C₈-C₂₂-carboxylic acid vinyl esters and mixtures thereof,
- 5 to 40% by weight of at least one monomer f) which is preferably chosen from C₁-C₆-(meth)acrylates, in particular methyl (meth)acrylate, ethyl (meth)acrylate, n-butyl (meth)acrylate and mixtures thereof.

11. The copolymer A) according to any of claims 1 to 10, obtainable by free-radical copolymerization of
- at least 2% by weight, based on the total weight of the monomers used for the polymerization, of at least one monomer pair of at least one N-vinylimidazole compound a2) and acrylic acid and/or methacrylic acid a1),
- at least 1% by weight, based on the total weight of the monomers used for the polymerization, of at least one additional monomer a1) with an anionogenic or anionic group or at least one additional monomer a2) with a cationogenic or cationic group,
- 0.05 to 5% by weight, based on the total weight of the monomers used for the polymerization, of at least one free-radically polymerizable crosslinking compound b),
- 0.05 to 30% by weight, particularly preferably 0.1 to 20% by weight, of at least one silicone compound c),
- 0 to 95% by weight of at least one monomer d) which contains amide groups,
- 0 to 40% by weight, based on the total weight of the monomers used for the polymerization, of at least one hydrophobic monomer e),
- 0 to 40% by weight, based on the total weight of the monomers used for the polymerization, of at least one monomer f).

12. A process for the preparation of a copolymer A) containing silicone groups as defined in any of claims 1 to 11, by free-radical copolymerization in accordance with the method of precipitation polymerization.

13. The process according to claim 12, where the polymerization takes place in a largely anhydrous, aprotic solvent or solvent mixture, preferably in ethyl acetate and/or n-butyl acetate.

14. The process according to either of claims 12 or 13, in which, for the copolymerization, at least two initiators are used whose decomposition temperatures are different from one another by at least 10°C, preferably by at least 15°C.

15. The process according to claim 14, where the copolymerization takes place until completion of the precipitation of the copolymer at a temperature greater than or equal to the lower decomposition temperature and less than the higher decomposition temperature and, after the precipitation, a further reaction takes place at a temperature greater than or equal to the higher decomposition temperature.

16. The process according to any of claims 12 to 14, comprising a first polymerization phase at a first polymerization temperature and a second polymerization phase at a second polymerization temperature above the first polymerization temperature, where, for the polymerization, at least two initiators are used whose half-lives at the first polymerization temperature differ in such a way that at least one of these initiators decomposes into free radicals during the first polymerization phase and at least one of these initiators essentially does not decompose into free radicals during the first polymerization phase and decomposes into free radicals during the second polymerization phase.

17. A cosmetic or pharmaceutical composition comprising
A) at least one copolymer containing silicone groups as defined in any of claims 1 to 11,
B) at least one cosmetically or pharmaceutically acceptable active substance or effect substance, and
C) if appropriate at least one further cosmetically or pharmaceutically acceptable auxiliary different from B).

## Revendications

1. Copolymère A) contenant des groupes silicone, pouvant être obtenu par copolymérisation radicalaire de a) au moins un composé contenant une double liaison α,β-éthyléniquement insaturée polymérisable par voie radicalaire et au moins un groupe ionogène et/ou ionique par molécule, les monomères a) étant exclusivement choisis parmi les composés a1) contenant des groupes anionogènes et/ou anioniques ou le composant
a) comprenant au moins un composé contenant au moins un groupe anionogène et/ou anionique a1) et au moins un composé contenant au moins un groupe cationogène et/ou cationique a2), et le copolymère A) présentant un excès molaire de groupes anionogènes/anioniques par rapport aux groupes cationogènes/cationiques supérieur à 1:1,
b) au moins un composé réticulant polymérisable par voie radicalaire, qui contient au moins deux doubles liaisons α,β-éthyléniquement insaturées par molécule, en présence d'au moins un composé de silicone c) contenant une double liaison oléfiniquement insaturée polymérisable par voie radicalaire, dans lequel le composé de silicone comprenant au moins une double liaison polymérisable par voie radicalaire consiste en un (méth)acrylate d'uréthane contenant des groupes siloxane polymérisable par voie radicalaire.

2. Copolymère A) selon la revendication 1, qui peut être obtenu par copolymérisation radicalaire par la méthode de polymérisation par précipitation.

3. Copolymère selon l'une quelconque des revendications 1 ou 2, dans lequel le composant a) comprend au moins un composé a1) contenant au moins un groupe anionogène et/ou anionique, qui est choisi parmi l'acide acrylique, l'acide méthacrylique, l'acide éthacrylique, l'acide α-chloroacrylique, l'acide crotonique, l'acide maléique, l'anhydride de l'acide maléique, l'acide fumarique, l'acide itaconique, l'acide citraconique, l'acide mésaconique, l'acide glutaconique, l'acide aconitique, l'acide 2-acrylamido-2-méthylpropanesulfonique, l'acide vinylphosphonique et leurs mélanges.

4. Copolymère selon l'une quelconque des revendications précédentes, dans lequel le composant a) comprend au moins un composé a2) contenant au moins un groupe cationogène et/ou cationique, qui est choisi parmi les esters d'acides mono- et dicarboxyliques α,β-éthyléniquement insaturés avec des aminoalcools, qui peuvent être mono- ou dialkylés sur l'azote de l'amine, les amides d'acides mono- et dicarboxyliques α,β-éthyléniquement insaturés avec des diamines, qui comprennent au moins un groupe amino primaire ou secondaire, la N,N-diallylamine, les N,N-diallyl-N-alkylamines et leurs dérivés, les hétérocycles azotés à substitution vinyle et allyle, les composés hétéroaromatiques à substitution vinyle et allyle, et leurs mélanges.

5. Copolymère selon l'une quelconque des revendications précédentes, qui contient également sous forme copolymérisée au moins un autre monomère d), qui est choisi parmi un composé α,β-éthyléniquement insaturé contenant des groupes amide de formule générale I 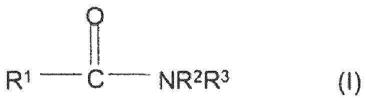 dans laquelle
un des radicaux R¹ à R³ représente un groupe de formule CH₂=CR⁴- avec R⁴= H ou alkyle en C₁-C₄, et les autres radicaux R¹ à R³ représentent indépendamment les uns des autres H, alkyle, cycloalkyle, hétérocycloalkyle, aryle ou hétaryle,
R¹ et R² pouvant également représenter ensemble avec le groupe amide auquel ils sont reliés un lactame contenant 5 à 8 atomes de cycle,
R² et R³ pouvant également représenter ensemble avec l'atome d'azote auquel ils sont reliés un hétérocycle de cinq à sept éléments,
à condition que la somme des atomes de carbone des radicaux R¹, R² et R³ soit d'au plus 8.

6. Copolymère selon l'une quelconque des revendications précédentes, qui contient également sous forme copolymérisée au moins un composé e), qui est choisi parmi les composés des formules générales III a), III b), III c), III d) et III e) 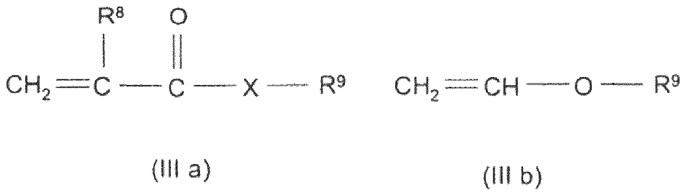 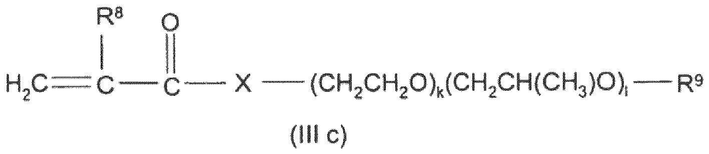 H₂C=CH-CH₂-O-(CH₂CH₂O)ₖ(CH₂CH(CH₃)O)ₗ-R⁹ (III d) 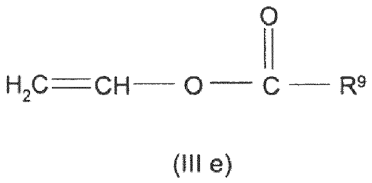
dans lesquelles
l'ordre des unités oxyde d'alkylène est quelconque,
k et 1 représentent indépendamment l'un de l'autre un nombre entier de 0 à 1 000, la somme de k et 1 étant d'au moins 5,
R⁸ représente hydrogène ou alkyle en C₁-C₄, de préférence méthyle,
R⁹ représente alkyle en C₈-C₃₀ ou alcényle en C₈-C₃₀, et
X représente O ou un groupe de formule NR¹⁰, R¹⁰ représentant H, alkyle, alcényle, cycloalkyle, hétérocycloalkyle, aryle ou hétaryle.

7. Copolymère selon l'une quelconque des revendications précédentes, qui contient également sous forme copolymérisée au moins un composé f), qui est choisi parmi les composés des formules générales III a*), III b*), III c*), III d*) et III e*) 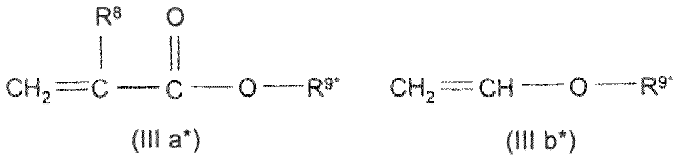 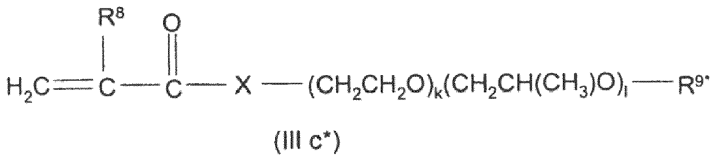
H₂C=CH-CH₂-O-(CH₂CH₂O)ₖ(CH₂CH(CH₃)O)ₗ-R^{9*} (III d*) 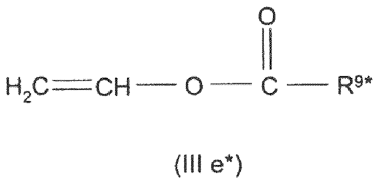
dans lesquelles
l'ordre des unités oxyde d'alkylène est quelconque,
k et 1 représentent indépendamment l'un de l'autre un nombre entier de 0 à 1 000, la somme de k et 1 étant d'au moins 5,
R⁸ représente hydrogène ou alkyle en C₁-C₄, de préférence méthyle,
R^{9*} représente hydrogène, alkyle en C₁-C₈ ou alcényle en C₃-C₈, et
X représente O ou un groupe de formule NR¹⁰, R¹⁰ représentant H, alkyle, alcényle, cycloalkyle, hétérocycloalkyle, aryle ou hétaryle.

8. Copolymère selon l'une quelconque des revendications précédentes, qui contient également sous forme copolymérisée au moins un composé g), qui est choisi parmi les esters d'acides mono- et dicarboxyliques α,β-éthyléniquement insaturés avec des diols en C₂-C₃₀, les amides d'acides mono- et dicarboxyliques α,β-éthyléniquement insaturés avec des aminoalcools en C₂-C₃₀, qui comprennent un groupe amino primaire ou secondaire, les esters d'alcool allylique avec des acides monocarboxyliques en C₁-C₇, les acrylates de polyéther différents de III c) et III c*), les composés aromatiques de vinyle, les halogénures de vinyle, les halogénures de vinylidène, les monooléfines en C₁-C₈, les hydrocarbures non aromatiques contenant au moins deux doubles liaisons conjuguées et leurs mélanges.

9. Copolymère A) selon l'une quelconque des revendications 1 à 8, qui contient sous forme copolymérisée
- au moins 2 % en poids d'au moins un composé a1) contenant une double liaison α,β-éthyléniquement insaturée polymérisable par voie radicalaire et au moins un groupe anionogène et/ou anionique par molécule, de préférence l'acide acrylique et/ou l'acide méthacrylique,
- 0,05 à 5 % en poids d'au moins un agent de réticulation b), de préférence le di(méth)acrylate d'éthylène glycol et/ou l'éther triallylique de pentaérythrite,
- 0,05 à 30 % en poids d'au moins un composé de silicone c).

10. Copolymère A) selon l'une quelconque des revendications 1 à 8, qui contient sous forme copolymérisée
- au moins 2 % en poids d'au moins un composé a1) contenant une double liaison α,β-éthyléniquement insaturée polymérisable par voie radicalaire et au moins un groupe anionogène et/ou anionique par molécule, de préférence l'acide acrylique et/ou l'acide méthacrylique,
- 0,05 à 5 % en poids d'au moins un agent de réticulation b), de préférence le di(méth)acrylate d'éthylène glycol et/ou l'éther triallylique de pentaérythrite,
- 0,05 à 30 % en poids d'au moins un composé de silicone c),
- 20 à 95 % en poids de vinylpyrrolidone d),
- 0,1 à 20 % en poids d'au moins un composé e), qui est de préférence choisi parmi les (méth)acrylates en C₈-C₂₂, les éthers de vinyle d'alkyle en C₈-C₂₂, les (méth)acrylates de polyéther terminés par des groupes alkyle en C₈-C₂₂, les alcoxylates d'alcool allylique terminés par des groupes alkyle en C₈-C₂₂, les esters de vinyle d'acides carboxyliques en C₈-C₂₂, et leurs mélanges,
- 5 à 40 % en poids d'au moins un monomère f), qui est de préférence choisi parmi les (méth)acrylates en C₁-C₆, notamment le (méth)acrylate de méthyle, le (méth)acrylate d'éthyle, le (méth)acrylate de n-butyle et leurs mélanges.

11. Copolymère A) selon quelconque des revendications 1 à 10, pouvant être obtenu par copolymérisation radicalaire de
- au moins 2 % en poids, par rapport au poids total des monomères utilisés pour la polymérisation, d'au moins une paire de monomères constituée d'au moins un composé de N-vinylimidazole a2) et d'acide acrylique et/ou d'acide méthacrylique a1),
- au moins 1 % en poids, par rapport au poids total des monomères utilisés pour la polymérisation, d'au moins un monomère supplémentaire a1) contenant un groupe anionogène ou anionique ou d'au moins un monomère supplémentaire a2) contenant un groupe cationogène ou cationique,
- 0,05 à 5 % en poids, par rapport au poids total des monomères utilisés pour la polymérisation, d'au moins un composé réticulant polymérisable par voie radicalaire b),
- 0,05 à 30 % en poids, de manière particulièrement préférée 0,1 à 20 % en poids, d'au moins un composé de silicone c),
- 0 à 95 % en poids d'au moins un monomère contenant des groupes amide d),
- 0 à 40 % en poids, par rapport au poids total des monomères utilisés pour la polymérisation, d'au moins un monomère hydrophobe e),
- 0 à 40 % en poids, par rapport au poids total des monomères utilisés pour la polymérisation, d'au moins un monomère f).

12. Procédé de fabrication d'un copolymère A) contenant des groupes silicone, tel que défini dans l'une quelconque des revendications 1 à 11, par copolymérisation radicalaire par la méthode de polymérisation par précipitation.

13. Procédé selon la revendication 12, dans lequel la polymérisation a lieu dans un solvant ou mélange de solvants aprotique, essentiellement anhydre, de préférence dans de l'acétate d'éthyle et/ou de l'acétate de n-butyle.

14. Procédé selon l'une quelconque des revendications 12 ou 13, dans lequel au moins deux initiateurs sont utilisés pour la copolymérisation, dont les températures de décomposition diffèrent de l'autre d'au moins 10 °C, de préférence d'au moins 15 °C.

15. Procédé selon la revendication 14, dans lequel la copolymérisation a lieu jusqu'à la fin de la précipitation du copolymère à une température supérieure ou égale à la température de décomposition plus basse et inférieure à la température de décomposition plus élevée, et, après la précipitation, une autre réaction a lieu à une température supérieure ou égale à la température de décomposition plus élevée.

16. Procédé selon l'une quelconque des revendications 12 à 14, comprenant une première phase de polymérisation à une première température de polymérisation et une deuxième phase de polymérisation à une deuxième température de polymérisation supérieure à la première température de polymérisation, au moins deux initiateurs étant utilisés pour la polymérisation, dont les demi-vies à la première température de polymérisation diffèrent de manière à ce qu'au moins un de ces initiateurs se décompose en radicaux pendant la première phase de polymérisation et qu'au moins un de ces initiateurs ne se décompose essentiellement pas en radicaux pendant la première phase de polymérisation et se décompose en radicaux pendant la deuxième phase de polymérisation.

17. Agent cosmétique ou pharmaceutique, contenant :
A) au moins un copolymère contenant des groupes silicone tel que défini dans l'une quelconque des revendications 1 à 11,
B) au moins un agent actif ou à effet cosmétiquement ou pharmaceutiquement acceptable, et
C) éventuellement au moins un autre adjuvant cosmétiquement ou pharmaceutiquement acceptable, différent de B).
